# EUROPEAN PATENT SPECIFICATION

(11) **EP 3 245 225 B1**
(45) Date of publication and mention of the grant of the patent: **22.09.2021**
(21) Application number: 15878250.8
(22) Date of filing: 13.07.2015
(51) Int. Cl.: C07K 16/18, C07K 16/30, C12P 19/14, A61P 35/00

(54) **COMPOSITIONS AND METHODS FOR TREATMENT AND DETECTION OF CANCERS**
ZUSAMMENSETZUNGEN UND VERFAHREN ZUR BEHANDLUNG UND DETEKTION VON KREBS
COMPOSITIONS ET MÉTHODES POUR TRAITER ET DÉTECTER DES CANCERS

(30) Priority: 16.01.2015 US 201514599174; 16.01.2015 WO PCT/US2015/011748; 27.05.2015 US 201514723297; 27.05.2015 WO PCT/US2015/032745
(43) Date of publication of application: 22.11.2017
(73) Proprietor: Academia Sinica, Nankang, Taipei 115 (TW)
(72) Inventor: WONG, Chi-Huey, Rancho Santa Fe, CA 92067 (US); HSU, Tsui-Ling, Taipei 11529 (TW); LOU, Yi-Wei, Taipei City 115 (TW); LIN, Chih-Wei, Keelung City 204 (TW); YEH, Shih-Chi, Nankang Taipei 115 (TW); WU, Chung-Yi, New Taipei City 221 (TW); WU, Han-Chung, Taipei City 105 (TW)
(74) Representative: Lavoix
(86) International application number: PCT/US2015/040199
(87) International publication number: WO 2016/114819

(56) References cited:
- WO-A1-2013/130603
- WO-A2-2015/109180
- US-A1- 2010 068 806
- US-A1- 2011 263 828
- US-A1- 2011 263 828
- US-A1- 2012 328 646
- US-A1- 2016 280 794
- Y.-W. LOU ET AL: "Stage-specific embryonic antigen-4 as a potential therapeutic target in glioblastoma multiforme and other cancers", PROCEEDINGS OF THE NATIONAL ACADEMY OF SCIENCES, vol. 111, no. 7, 3 February 2014 (2014-02-03), pages 2482-2487, XP055218264, ISSN: 0027-8424, DOI: 10.1073/pnas.1400283111

## Description

### FIELD

Antibodies that bind to SSEA-4 are disclosed herein, as well as related compositions and their therapeutic use, which includes, without limitation, cancer therapies and diagnostics.

### BACKGROUND OF THE INVENTION

Glioblastoma multiforme (GBM), accounting for 60 to 70% of malignant gliomas, is the most aggressive form of gliomas and the most common primary brain tumor in adults (Louis DN, et al. (2007) The 2007 WHO classification of tumours of the central nervous system. Acta Neuropathol 114(2):97-109). Despite many treatments, including surgery, and chemo- or radiotherapy available for GBM, the prognosis and survival rate for GBM patients are still poor, with the median survival rate of 14-15 months (Van Meir EG, et al. (2010) Exciting new advances in neuro-oncology: the avenue to a cure for malignant glioma. CA Cancer J Clin 60(Meyer MA (2008) Malignant gliomas in adults. N Engl J Med 359(17):1850; author reply 1850):166-193). GBM is notoriously resistant to most anti-cancer drugs and extremely infiltrative, which hampers complete surgical resection, and therefore most patients develop tumor recurrence or progression even after multiple therapies. Because of the high mortality, new therapeutic approaches, such as immunotherapy and gene therapy, have been proposed for the treatment of GBM (Meyer MA (2008) Malignant gliomas in adults. N Engl J Med 359(17):1850; author reply 1850).

Altered glycosylation is a feature of cancer cells, and several glycan structures are well-known tumor markers (Meezan E, Wu HC, Black PH, & Robbins PW (1969) Comparative studies on the carbohydrate-containing membrane components of normal and virus-transformed mouse fibroblasts. II. Separation of glycoproteins and glycopeptides by sephadex chromatography. Biochemistry 8(6):2518-2524; Hakomori S (2002) Glycosylation defining cancer malignancy: new wine in an old bottle. Proc Natl Acad Sci U S A 99(16):10231-10233). These aberrant changes include the overall increase in the branching of N-linked glycans (Lau KS & Dennis JW (2008) N-Glycans in cancer progression. Glycobiology 18(10):750-760) and sialic acid content (van Beek WP, Smets LA, & Emmelot P (1973) Increased sialic acid density in surface glycoprotein of transformed and malignant cells--a general phenomenon? Cancer Res 33(11):2913-2922), and the overexpression of certain glycan epitopes, such as sialyl Lewis x (sLex), sialyl Tn (sTn), Lewis y (Ley), Globo H, and polysialic acid (Sell S (1990) Cancer-associated carbohydrates identified by monoclonal antibodies. Hum Pathol 21(10):1003-1019; Hakomori S & Zhang Y (1997) Glycosphingolipid antigens and cancer therapy. Chem Biol 4(2):97-104; Taylor-Papadimitriou J & Epenetos AA (1994) Exploiting altered glycosylation patterns in cancer: progress and challenges in diagnosis and therapy. Trends Biotechnol 12(6):227-233). Many tumors also exhibit increased expression of certain glycolipids, especially the gangliosides, glycosphingolipids (GSLs) with sialic acid(s) attached to the glycan chain. Gangliosides are normally observed in neural systems, and are found to be elevated in tumors, particularly the complex gangliosides associated with malignancy (Birkle S, Zeng G, Gao L, Yu RK, & Aubry J (2003) Role of tumor-associated gangliosides in cancer progression. Biochimie 85(3-4):455-463).

It has been reported that human glioma show expression of ganglosides (Fredman P, et al. (1986) Potential ganglioside antigens associated with human gliomas. Neurol Res 8(2):123-126; Yates AJ, Becker LE, & Sachs LA (1979) Brain tumors in childhood. Childs Brain 5(1):31-39; Traylor TD & Hogan EL (1980) Gangliosides of human cerebral astrocytomas. J Neurochem 34(1):126-131; Berra B, Gaini SM, & Riboni L (1985) Correlation between ganglioside distribution and histological grading of human astrocytomas. Int J Cancer 36(3):363-366; Fredman P, von Holst H, Collins VP, Granholm L, & Svennerholm L (1988) Sialyllactotetraosylceramide, a ganglioside marker for human malignant gliomas. J Neurochem 50(3):912-919; Mansson JE, et al. (1986) Characterization of new gangliosides of the lactotetraose series in murine xenografts of a human glioma cell line. FEBS Lett 201(1):109-113; Fredman P, von Holst H, Collins VP, Dellheden B, & Svennerholm L (1993) Expression of gangliosides GD3 and 3'-isoLM1 in autopsy brains from patients with malignant tumors. J Neurochem 60(1):99-105). Since some of glioma-associated gangliosides are rarely expressed or even absent in normal tissues (Svennerholm L, et al. (1989) Human brain gangliosides: developmental changes from early fetal stage to advanced age. Biochim Biophys Acta 1005(2): 109-117), they are suitable for targeted therapy (Kato Y, et al. (2010) GMab-1, a high-affinity anti-3'-isoLM1/3',6'-isoLD1 IgG monoclonal antibody, raised in lacto-series ganglioside-defective knockout mice. Biochem Biophys Res Commun 391(1):750-755). Hence, discovering novel glioma-associated GSLs would provide new targets for development of new therapies against gliomas.

The GSLs of globo-series feature a Galα1-4Gal linkage to lactosylceramides, and this linkage is catalyzed by lactosylceramide 4-alpha-galactosyltransferase (A4GALT). While globotriosylceramide (Gb3Cer) and globoside (Gb4Cer) constitute the basis of P-blood group system (Schenkel-Brunner H (1995) P System. Human Blood Groups, (Springer Vienna), pp 211-234), galactosyl globoside (Gb5Cer) and sialyl galactosyl globoside (sialyl Gb5Cer, SGG, MSGG), also known as stage-specific embryonic antigen-3 (SSEA-3) and SSEA-4 (Kannagi R, et al. (1983) Stage-specific embryonic antigens (SSEA-3 and -4) are epitopes of a unique globo-series ganglioside isolated from human teratocarcinoma cells. EMBO J 2(12):2355-2361), respectively, are widely used as cell-surface markers to define human embryonic stem cells. Globo-series GSLs have also been observed in tumors: Globo H (fucosyl Gb5Cer) is overexpressed in many epithelial cancers, such as ovarian, gastric, prostate, lung, breast, and pancreatic cancers (Zhang S, et al. (1997) Selection of tumor antigens as targets for immune attack using immunohistochemistry: I. Focus on gangliosides. Int J Cancer 73(1):42-49); SSEA-3, SSEA-4 and Globo H are expressed not only on breast cancer cells, but also on breast cancer stem cells (Chang WW, et al. (2008) Expression of Globo H and SSEA3 in breast cancer stem cells and the involvement of fucosyl transferases 1 and 2 in Globo H synthesis. Proc Natl Acad Sci U S A 105(33):11667-11672; Huang YL, et al. (2013) Carbohydrate-based vaccines with a glycolipid adjuvant for breast cancer. Proc Natl Acad Sci U S A 110(7):2517-2522). Moreover, in renal cell carcinoma, high-level expressions of SSEA-4 and disialosyl galactosyl globoside (disialosyl Gb5Cer, DSGG) are observed (Saito S, et al. (1997) Expression of globo-series gangliosides in human renal cell carcinoma. Jpn J Cancer Res 88(7):652-659), but it is still not known whether globo-series GSLs are expressed on GBM. Lou VW et al., PNAS, 2014, 111(7), 2482-2487, relates to SSEA4 as a potential therapeutic target in glioblastoma multiforme and other cancers.

It is of great interest to identify glycan markers associated with and/or predictive of cancers, and develop antibodies against the markers for use in diagnosing and treating a broad spectrum of cancers.

### SUMMARY OF THE INVENTION

The present invention is defined in claims 1 to 14 and refers to an SSEA4-specific isolated humanized monoclonal antibody that specifically binds to Neu5Acα2→ 3Galβ1→ 3GalNAcβ1→ 3Galα1→ 4Galβ1→ 4Glcβ1, wherein the antibody comprises a VH having SEQ ID NO: 147 and a VL having SEQ ID NO:148 and further its use for treating cancer in a subject in need thereof. The present disclosure is also based on the discovery that Globo H, SSEA-3 and SSEA-4 are aberrantly expressed in a broad spectrum of cancers, but not on normal cells. Cancers cells expressing Globo H, SSEA-3 and SSEA-4 include, but are not limited to, brain cancer, lung cancer, breast cancer, oral cancer, esophageal cancer, stomach cancer, liver cancer, bile duct cancer, bone cancer (osteosarcoma), skin cancer, pancreatic cancer, colon cancer, kidney cancer, cervical cancer, ovarian cancer and prostate cancer.

Exemplary antibodies described herein can have one or more characteristics of:

a) is a recombinant antibody, a monoclonal antibody, a chimeric antibody, a humanized antibody, a human antibody, an antibody fragment, a bispecific antibody, a monospecific antibody, a monovalent antibody, an IgG1 antibody, an IgG2 antibody, or derivative of an antibody; b) is a human, murine, humanized, or chimeric antibody, antigen-binding fragment, or derivative of an antibody; c) is a single-chain antibody fragment, a multibody, a Fab fragment, and/or an immunoglobulin of the IgG, IgM, IgA, IgE, IgD isotypes and/or subclasses thereof; d) has one or more of the following characteristics: (i) mediates ADCC and/or CDC of cancer cells; (ii) induces and/or promotes apoptosis of cancer cells; (iii) inhibits proliferation of target cells of cancer cells; (iv) induces and/or promotes phagocytosis of cancer cells; and/or (v) induces and/or promotes the release of cytotoxic agents; e) does not bind an antigen expressed on non-cancer cells, non-tumor cells, and/or benign tumor cells.

In some examples, any of the antibodies described herein can be generated as Fab or single chain by phage display antibody library or single B cells using the B cells from patients or recovered patients or healthy persons.

The subject (e.g., a human patient) in need of the treatment is diagnosed with, suspected of having, or at risk for cancer. Examples of the cancer include, but are not limited to, brain cancer, lung cancer, breast cancer, oral cancer, esophagus cancer, stomach cancer, liver cancer, bile duct cancer, pancreatic cancer, colon cancer, kidney cancer, cervical cancer, ovarian cancer and prostate cancer. In some embodiments, the cancer is brain cancer, lung cancer, breast cancer, ovarian cancer, prostate cancer, colon cancer, or pancreatic cancer. In some preferred embodiments, the cancer is brain cancer or glioblastoma multiforme (GBM) cancer.

The antibody is capable of targeting SSEA-4-expressing cancer cells.

The treatment results in reduction of tumor size, elimination of malignant cells, prevention of metastasis, prevention of relapse, reduction or killing of disseminated cancer, prolongation of survival and/or prolongation of time to tumor cancer progression.

In some embodiments, the treatment further comprises administering an additional therapy to said subject prior to, during or subsequent to said administering of the antibodies. In some embodiments, the additional therapy is treatment with a chemotherapeutic agent. In some embodiments, the additional therapy is radiation therapy.

In yet another aspect, the present disclosure features pharmaceutical compositions for use in treating cancer, such as brain cancer, lung cancer, breast cancer, oral cancer, esophagus cancer, stomach cancer, liver cancer, bile duct cancer, pancreatic cancer, colon cancer, kidney cancer, cervical cancer, ovarian cancer and prostate cancer, etc. The pharmaceutical composition comprises any of the antibodies or nucleic acid encoding such and a pharmaceutically acceptable carrier, and uses of such pharmaceutical compositions in manufacturing a medicament for treating cancer.

The details of one or more embodiments of the invention are set forth in the drawings and detailed description below.

### BRIEF DESCRIPTION OF THE DRAWINGS

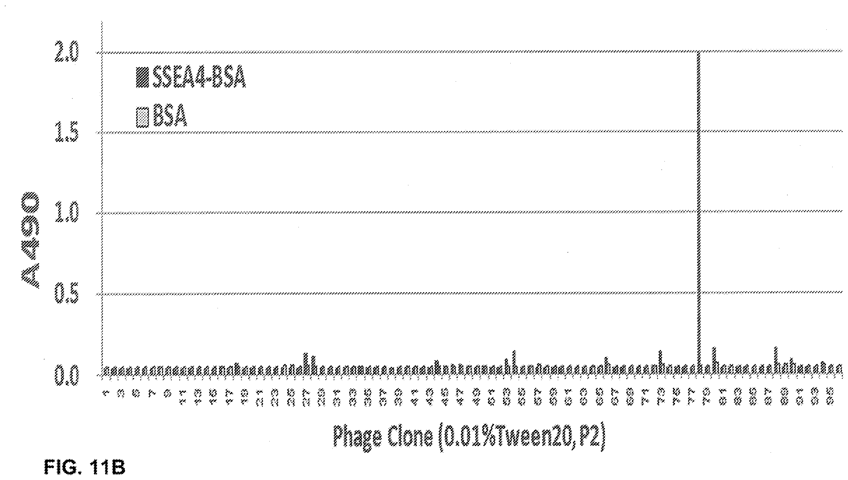

As used herein, symbolic, graphic, and text nomenclature for describing glycans and related structures are well-established and understood in the art, including, for example, "Symbols Nomenclatures for Glycan Representation", Proteomics. 2009 December; 9(24): 5398-5399 by Ajit Varki et al.

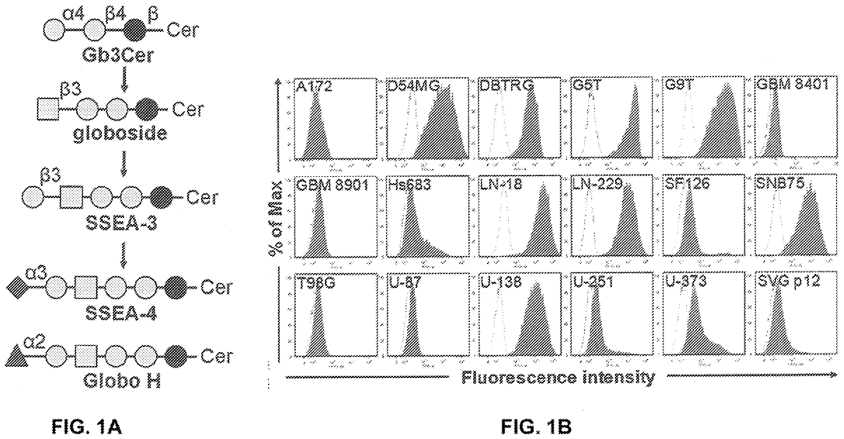

**Figs. 1A-1B****.** The binding characteristics of anti-SSEA-4 mAb to GBM cell lines. (Fig. 1A) Schematic diagram of the biosynthesis of globoseries GSLs. SSEA-3, the precursor of SSEA-4 and Globo H, is synthesized from globoside. Glycosidic linkages and graphic notations are labelled (Glc, glucose; Gal, galactose; GalNAc, N-acetylgalactosamine; Fuc, fucose; NeuAc, N-acetylneuraminic acid). (Fig. 1B) GBM cells were stained with Alexa Fluor 488-conjuagated MC813-70 and the staining intensity was analyzed with flow cytometry. All the cells examined were GBM cell lines except SVG p12, which is a normal human fetal glial cell line transformed with SV40 large T antigen. The histograms of the cells stained with MC813-70 and isotype control are shown in gray and white, respectively.

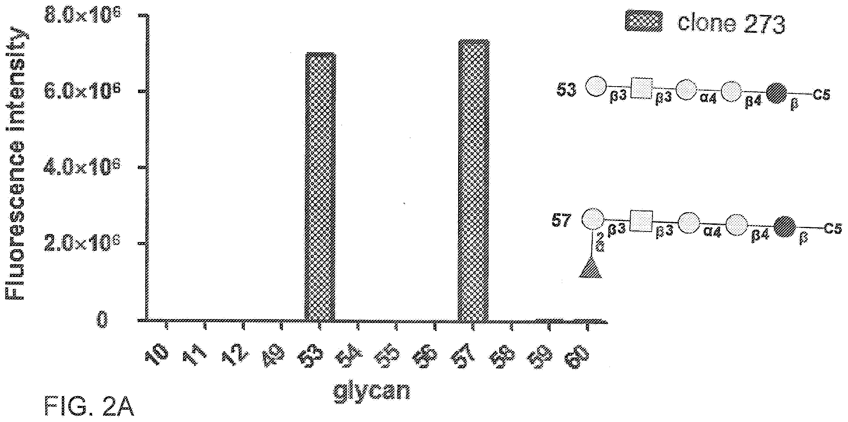

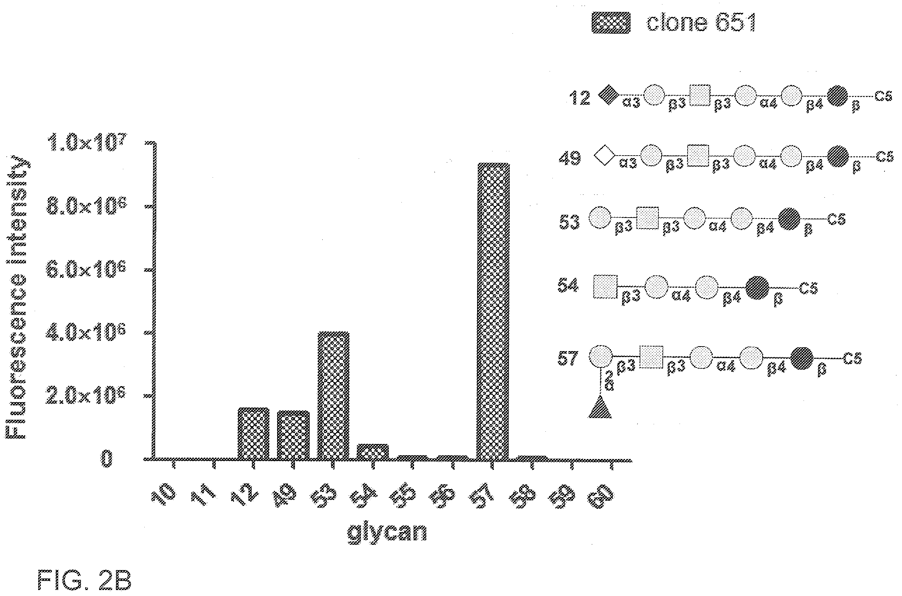

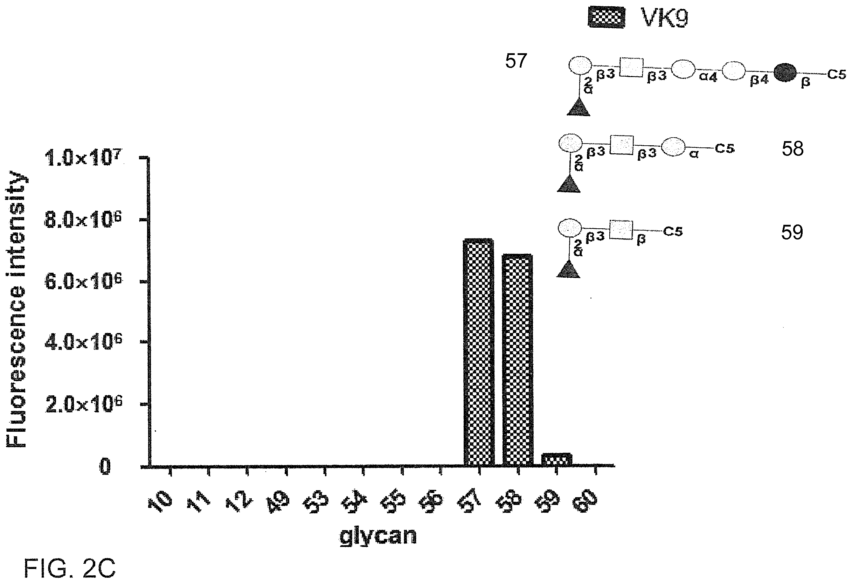

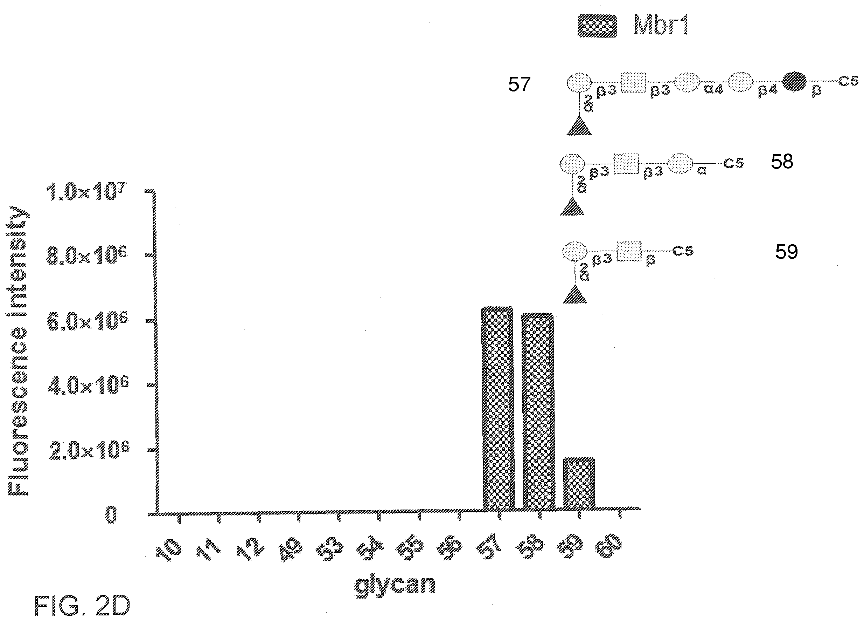

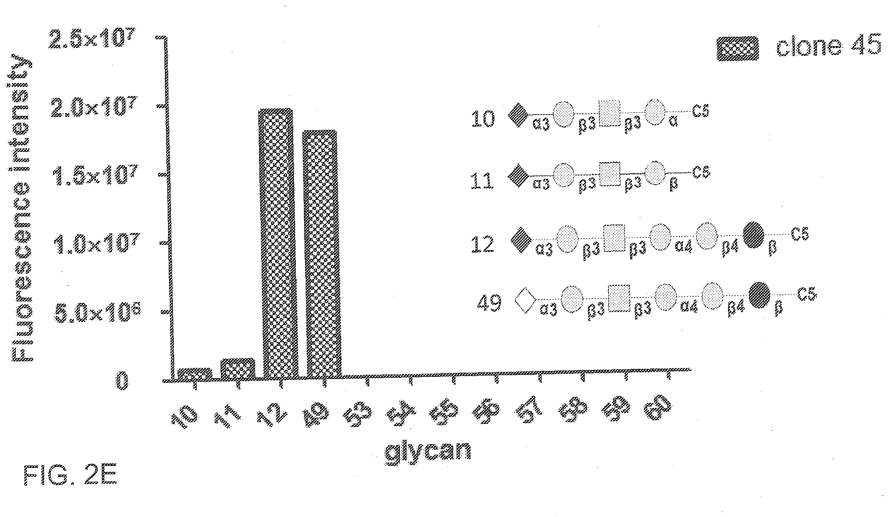

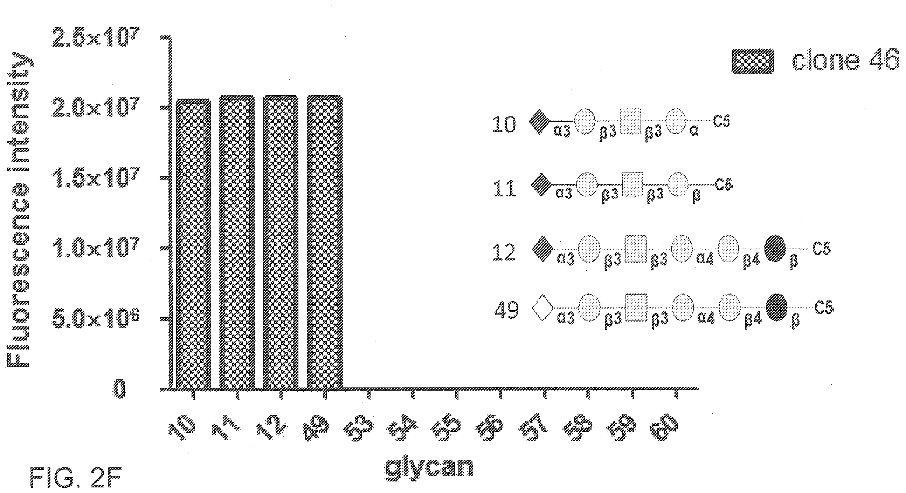

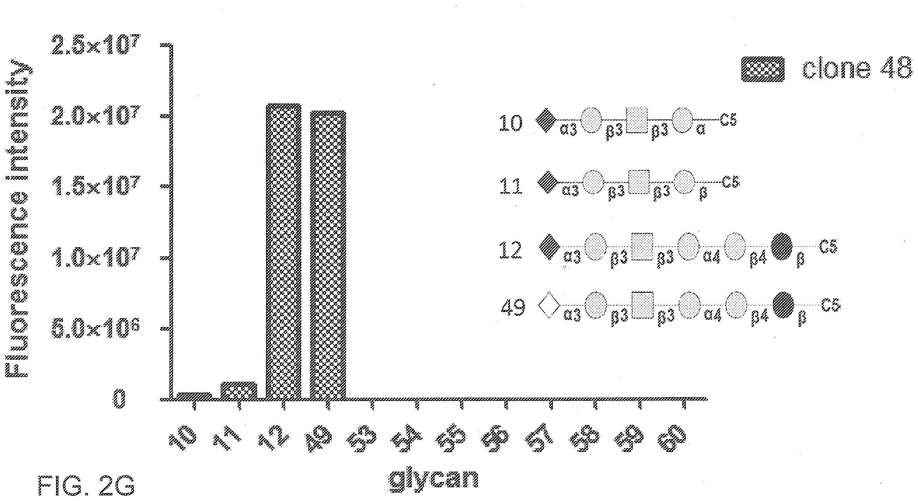

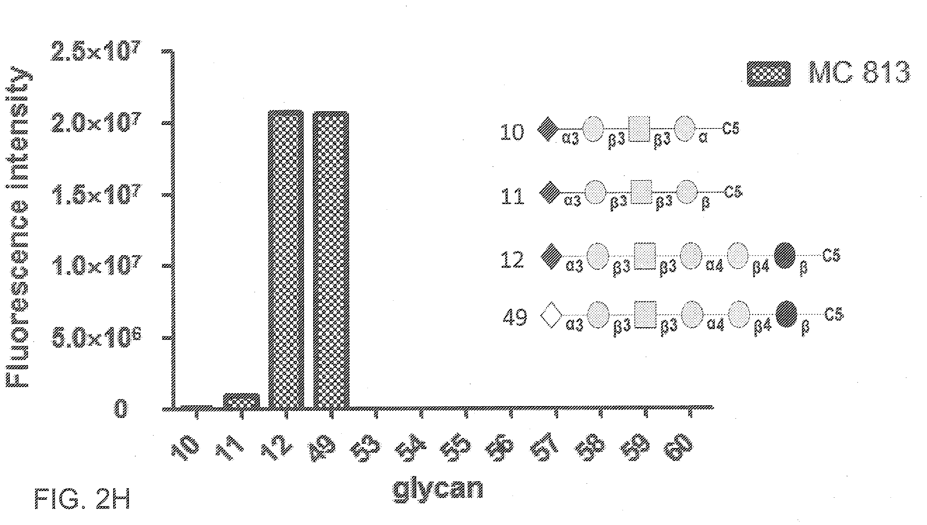

**Figs. 2A-2H****.** Glycan binding profiles of antibodies. The glycan microarrays on glass slides were interacted with Alexa Flour 647-conjugated antibody (10 µg/mL) and read in an array scanner at 635 nm. Data are presented as mean ± SD. (Fig. 2A) binding profile of mAb 273 (Fig. 2B) binding profile of mAb 651 (Fig. 2C) binding profile of mAb VK9 (Fig. 2D) binding profile of mAb Mbr1 (Fig. 2E) binding profile of mAb 45 (Fig. 2F) binding profile of mAb 46(Fig. 2G) binding profile of mAb 48(Fig. 2H) binding profile of MC813-70.

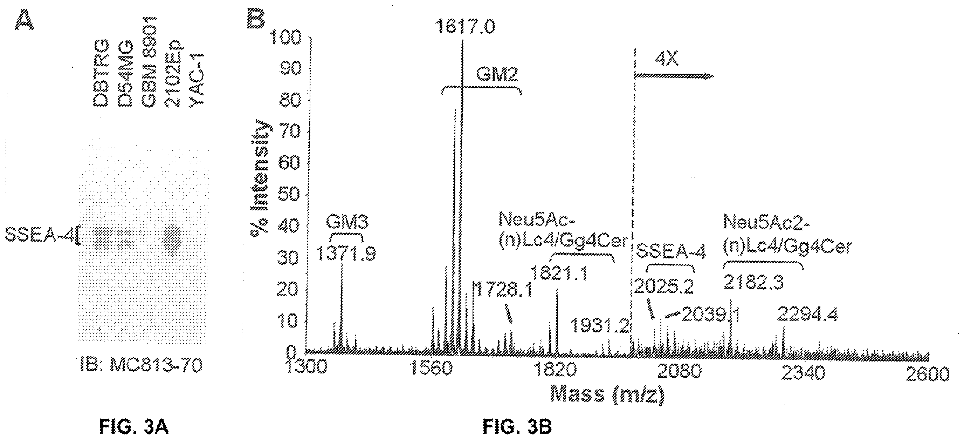

**Figs. 3A-3B****.** HPTLC immunostaining and MALDI-MS profiles of gangliosides from GBM cell lines. (Fig. 3A) Gangliosides were separated on an HPTLC plate and detected with MC813-70 mAb. Gangliosides from 2012Ep (human embryonal carcinoma cell line) and YAC-1 (mouse lymphoma cell line) were applied to serve as the positive controls for SSEA-4 and GM1b, respectively. SSEA-4 with different chain lengths of fatty acids migrated as two close bands. (Fig. 3B) The extracted gangliosides from DBTRG GBM cells were permethylated and analyzed by MALDI-MS. The major gangliosides in DBTRG cells were GM3 (*mlz* = 1371.9), GM2 (*m*/*z* = 1617.0), Neu5Ac-(n)Lc4/Gg4Cer (*m*/*z* = 1821.1), and Neu5Ac2-(n)Lc4/Gg4Cer (*m*/*z* = 2182.3). Although in a relatively weak signal, SSEA-4 (Neu5Ac-Hex4-HexNAc-Cer, *m*/*z* = 2025.2) was also observed. Gangliosides with the same glycan moiety but with different fatty acyl contents are bracketed.

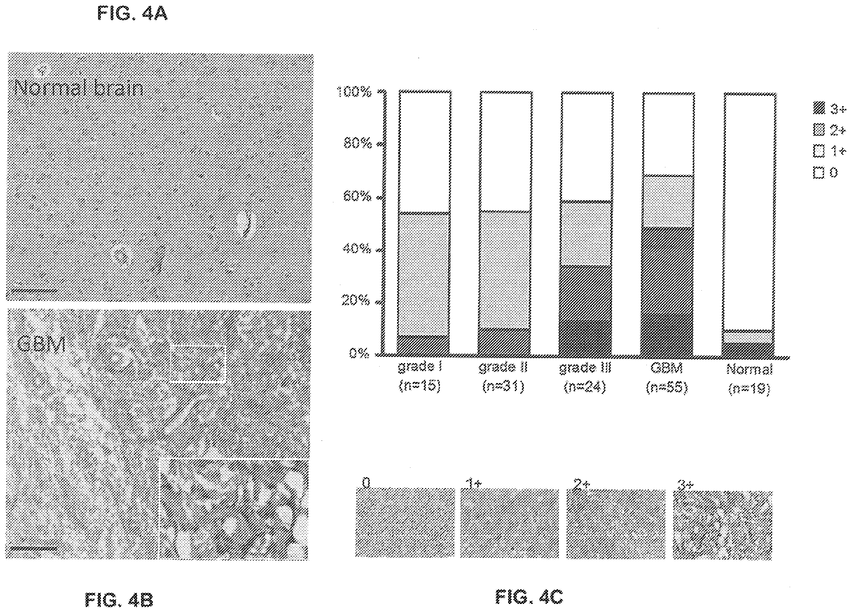

**Figs. 4A-4C****.** Expression profile of SSEA-4 in GBM. Representative images of normal brain tissues (Fig. 4A) and GBM (Fig. 4B) after immunohistochemical staining with MC-813-70. The inset in panel B shows a magnified picture of the small rectangular area. Scale bar, 100 µm. (Fig. 4C) Statistical results of SSEA-4 IHC. The staining intensity of GBM specimens was graded as 0 (negative, i), 1+ (weak, ii), 2+ (moderate, iii), and 3+ (strong, iv). Scale bar, 100 µm. Grade I (n=15), grade II (n=31), grade III (n=24), grade IV (GBM, n=55) specimens and normal brain tissues (n=19) were counterstained with hematoxylin after IHC. The staining intensity of the tissues was graded as 0 (negative), 1+ (weak), 2+ (moderate), and 3+ (strong).

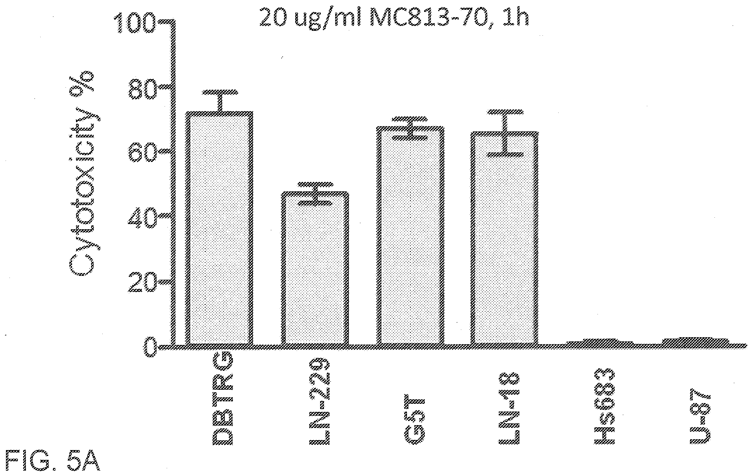

**Figs. 5A-5B****.** Complement dependent cytotoxicity (CDC) effects of anti-SSEA-4.

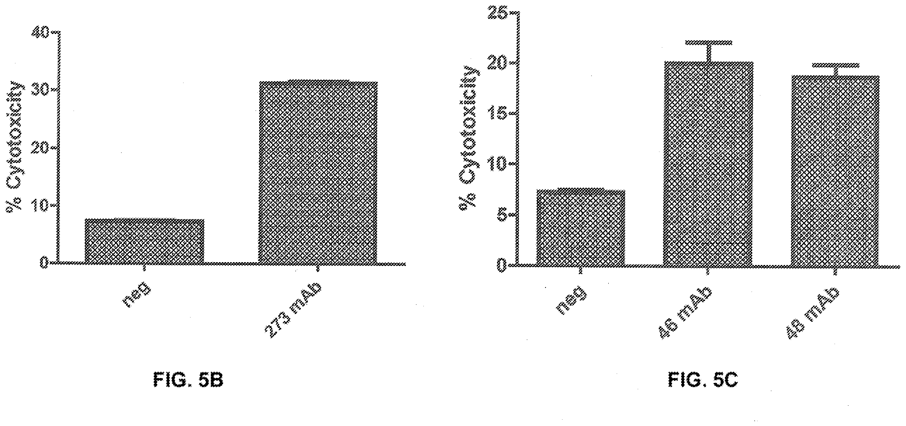

(Fig. 5A) MC813-70 on GBM. GBM cell lines were treated with 20 µg/mL and rabbit complement to observe MC813-70-induced cell lysis. The CDC activity of MC813-70 was measured by lactate dehydrogenase (LDH) release assay as described in "Materials and Methods." The data are shown as mean ± SD. (Fig. 5B) mAb 273 on MCF-7 in vitro. (Fig. 5C) mAbs 46 and 48 on human pancreatic cancer cells BxPC3 in vitro. Aliquots of tumor cells (10⁴ cells) were incubated with 80µL of antibody at various concentrations in the presence of 20µL of human-serum or rabbit serum as complement source for 2 hour at 37°C. Cytotoxicity was determined within the tumor cell population after addition of 7-amino-actinomycin D (7-AAD).

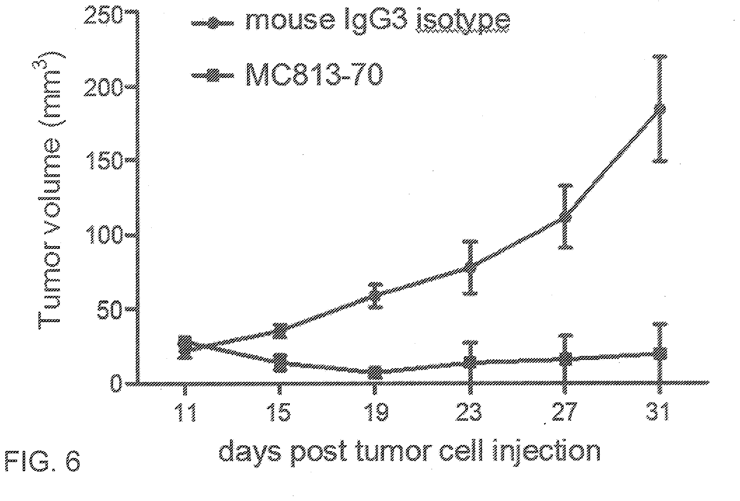

**Fig. 6****.** Inhibition of DBTRG tumor growth by anti-SSEA-4. Male nude mice were inoculated with DBTRG cells on the right flank at day 0, intraperitoneally administered with MC813-70 or mouse IgG3 isotype control (200 µg per dose) at day 11, 15, and 19, and sacrificed at day 31. The tumor volume in each group (n=3) was measured at different time points and shown as mean ± SD. *P*=0.001 was obtained by two-way ANOVA.

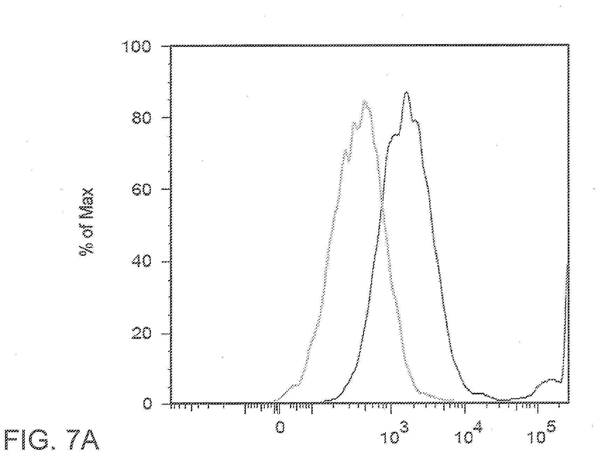

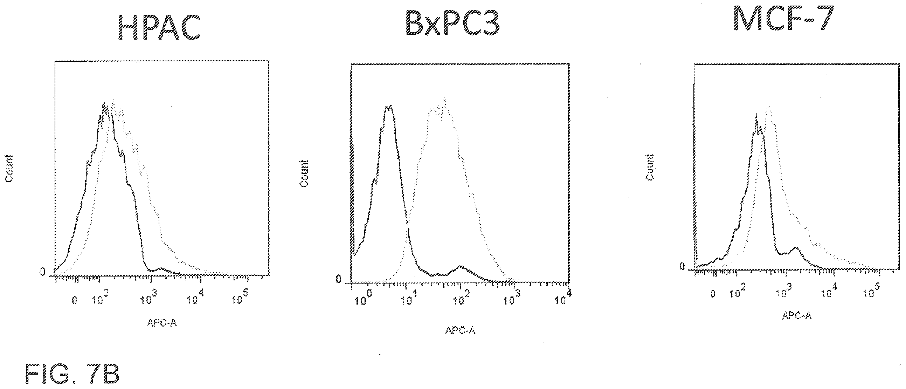

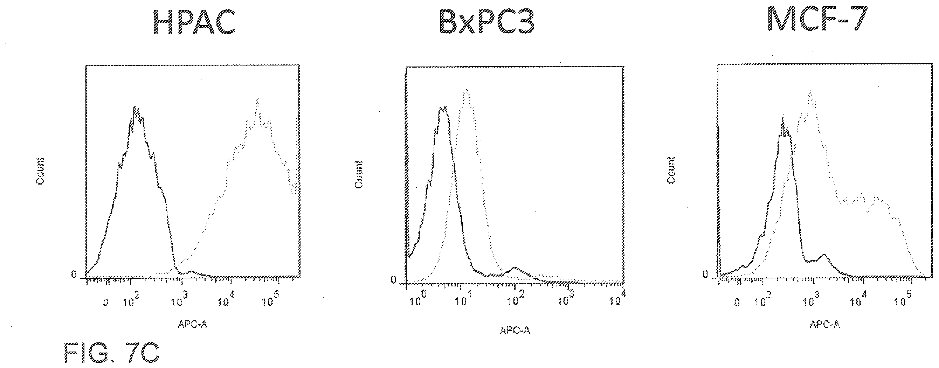

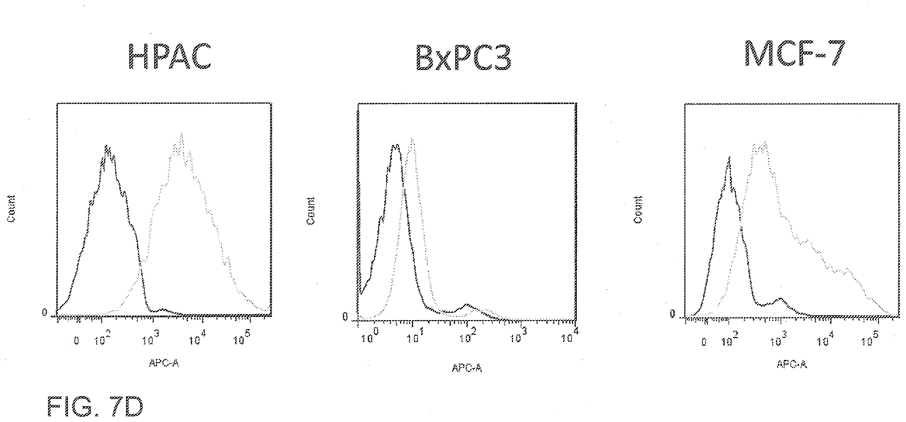

**Figs. 7A-7D****.** Binding of antibodies to cancer cells. (Fig. 7A) Breast cancer cells MCF-7 were stained with mAb 273. (Fig. 7B) Pancreatic cancer cells (HPAC and BxPC3) and breast cancer cells MCF-7 were stained with mAb 45. (Fig. 7C) Pancreatic cancer cells (HPAC and BxPC3) and breast cancer cells MCF-7 were stained with mAb 46. (Fig. 7D) Pancreatic cancer cells (HPAC and BxPC3) and breast cancer cells MCF-7 were stained with mAb 48. These cells were stained with Alexa Fluor 488-conjuagated antibodies and the staining intensity was analyzed with flow cytometry. The cells stained with mAbs and isotype controls are shown in blue and red, respectively.

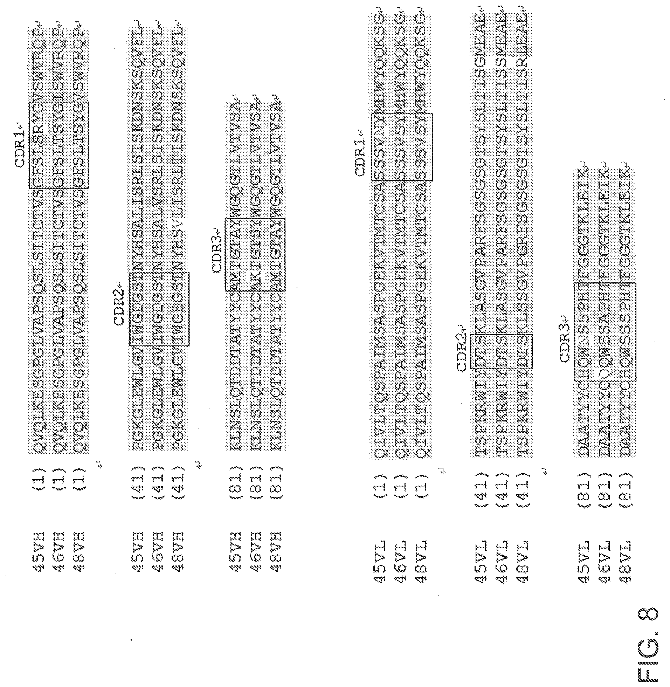

**Fig. 8****.** Comparison of amino acid sequences mAb 45 (SEQ ID NOS 23 and 24, respectively, in order of appearance), mAb 46 (SEQ ID NOS 33 and 34, respecively, in order of appearance) and mAb 48 (SEQ ID NOS 43 and 44, respectively, in order of appearance). For multi-sequence alignments, ClustalW uses progressive alignment methods.

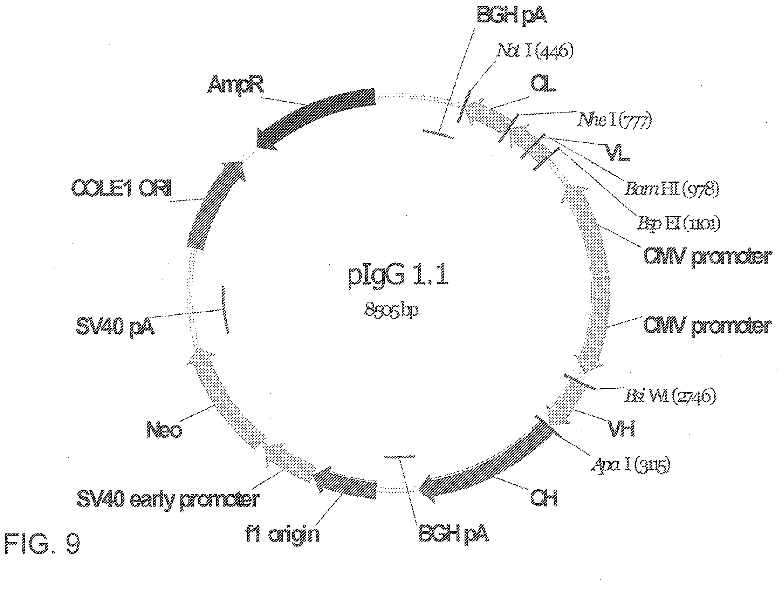

**Fig. 9****.** Human IgG antibody expression vector.

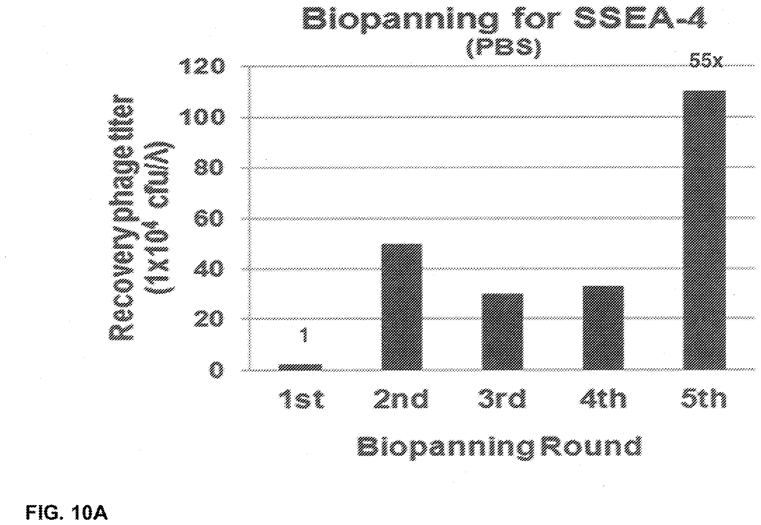

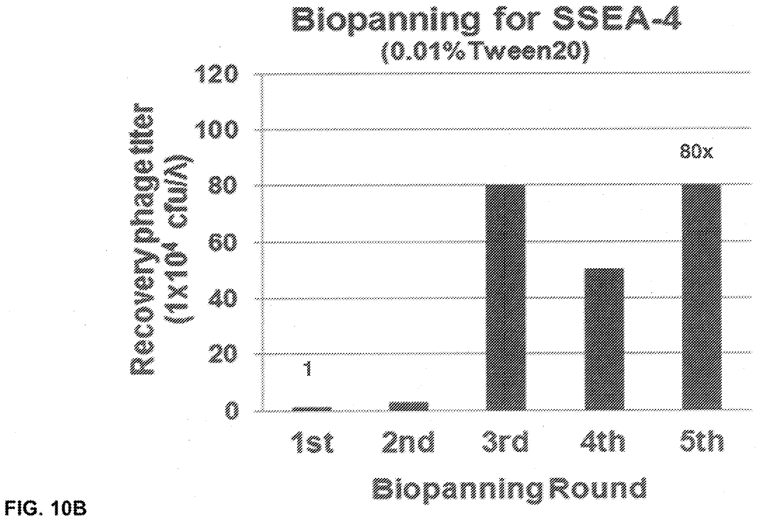

**Figs. 10A-10B****.** Biopanning for SSEA-4 by phage-displayed human naive scFv library. Selection of phage-displayed scFv that bound to SSEA-4. A phage-displayed human naive scFv library was used to select phages that bound to SSEA-4-PEG-conjugated Dynabeads. The recovery rate of the phages was increased after the fifth round of biopanning compared to first round. PBS (Fig. 10A) and PBS containing 0.01% Tween20 (Fig. 10B) was used as wash buffer system during biopanning process.

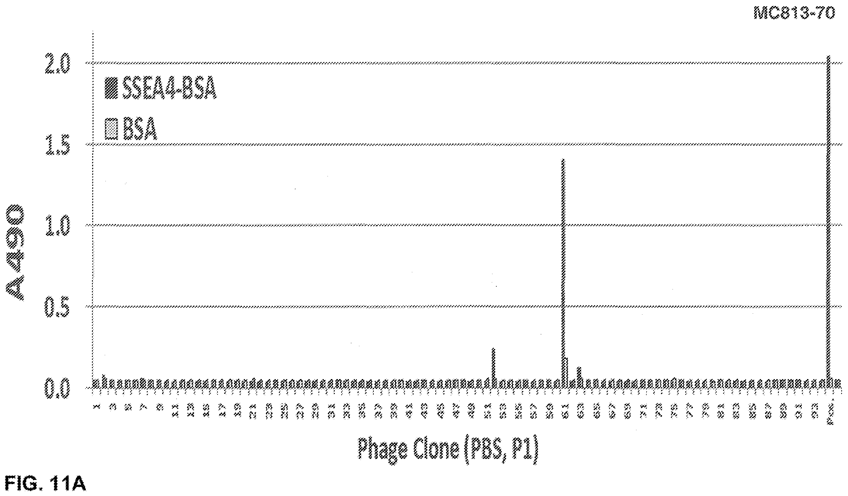

**Figs. 11A-11D****.** Screening of phage-displayed scFv that bound to SSEA-4 by ELISA.
The randomly selected phage clones were screened via ELISA to reveal different binding. (Fig. 11A) The colonies were selected from the PBS wash group. (Figs. 11B-11D) The colonies were selected from the PBST_{0.01} wash group. Eight phage clones were found to have superior binding activity to SSEA-4-BSA (A490 ≧ 0.2). Control phage was used as a negative control (Neg.) Commercial anti-SSEA-4 mAb (MC813-70) was used as a positive control (Pos.). A490: absorbance at 490 nm.

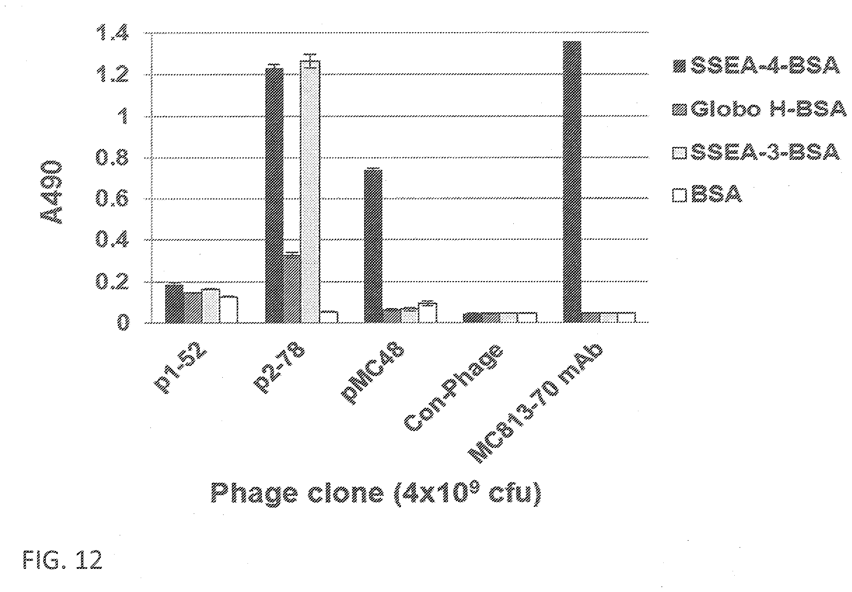

**Fig. 12****.** Comparison of the binding activity of anti-SSEA-4 phage clones to Globo-series glycans. ELISA was performed to examine the binding of anti-SSEA-4 phage clones to SSEA-4-BSA, Globo H-BSA and SSEA-3-BSA. Commercial anti-SSEA-4 mouse monoclonal antibody (MC813-70) and control phage (Con-phage) were used as a positive and negative control, respectively. The pMC48 is the phage-displayed scFv format of anti-SSEA-4 mouse mAb created by Dr. Wong's Lab. A490: absorbance at 490 nm

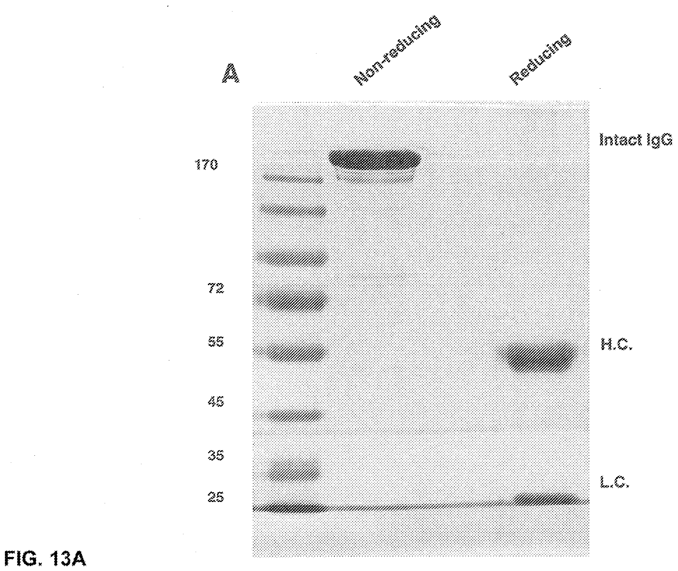

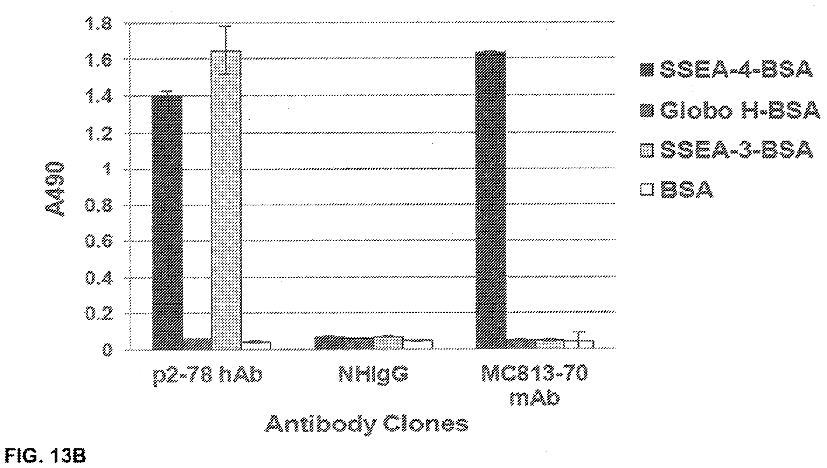

**Figs. 13A-13B****.** Analysis of the binding activity of p2-78 hAb by ELISA. (Fig. 13A) The purity of IgG was analyzed by SDS-PAGE with coomassie blue staining. (Fig. 13B) ELISA was performed to examine the binding of 1 µg/ml anti-SSEA-4 hAb to the glycans as figure indicated. Commercial anti-SSEA-4 mouse monoclonal antibody (MC813-70; 0.5 µg/ml) and normal human IgG (NHIgG) were used as a positive and negative control, respectively. H.C.: heavy chain. L.C.: light chain. A490: absorbance at 490 nm
**Figs.14A-14B****.** Analysis of the binding activity of p2-78 hAb by glycan array.
(Fig. 14A) The commercially available IgM antibody, MC631 (5 µg/ml), was used as a positive control. (Fig. 14B) The p2-78 hAb (7.5 µg/ml) recognized SSEA4, Sialyl-SSEA4, SSEA4Gc, and Gb5 (SSEA3), and GloboH.
**Figs.15A-15B****.** Binding assay of hMC48 scFv phage clones.
The binding activity of humanized MC48 clones was examined by ELISA. The 3^{rd} scFv phage clone of humanized MC48 variant could bind to SSEA-4 (Fig. 15A) in a dose-dependent manner but not to BSA control protein (Fig. 15B).
**Figs. 16A-16B****.** (Fig. 16A) Complementary-determining regions 1-3 (CDR1-3), and framework regions 1-4 (FW1-4) for both the V_{H} and V_{L} domains from exemplary antibody clones are shown. The variable domains were aligned by IMGT database. (Fig. 16B) Complementary-determining regions 1-3 (CDR1-3), and framework regions 1-4 (FW1-4) for both the VH and VL domains are shown. The variable domains were aligned by IMGT database.
**Figure 17** Illustrates exemplary amino acid and nucleotide sequences of exemplary glycoantibody variants based on mouse monoclonal antibody MC48 (MC48 is an exemplary anti-SSEA-4 mouse monoclonal antibody used for humanization). The exemplary humanized glycoantibody variants are generated as set forth in the examples sections. Tables 17-1 to 17-4 describes exemplary humanized glycoantibody sequences obained from each corresponding round of humanization.
**Figure 18** Binding assay of hMC48 scFv phage clones. The binding activity of humanized MC48 clones was examined by ELISA. The 4^{th} scFv phage clone of humanized MC48 variant could bind to SSEA-4 (A) in a dose-dependent manner but not to BSA control protein (B).

### DETAILED DESCRIPTION OF THE INVENTION

The invention is as defined in the claims.

Accordingly, a SSEA4-specific isolated humanized monoclonal antibody that specifically binds to Neu5Acα2→ 3Galβ1→ 3GalNAcβ1→ 3Galα1→ 4Galβ1→ 4Glcβ1, wherein the antibody comprises a VH having SEQ ID NO: 147 and a VL having SEQ ID NO:148 and its use in the treatment of cancer is provided. The antibodies described herein can bind to a broad spectrum of Globo H, SSEA3 and SSEA-4-expressing cancer cells, thereby facilitating cancer diagnosis and treatment. Cells that can be targeted by the antibodies include carcinomas, such as those in brain, lung, breast, mouse, esophagus, stomach, liver, bile duct, pancreas, colon, kidney, cervix, ovary, prostate cancer, etc.

### Definitions

The practice of the present invention will employ, unless otherwise indicated, conventional techniques of molecular biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press, 1989); DNA Cloning, Volumes I and II (D. N. Glover ed., 1985); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Antibodies: A Laboratory Manual, by Harlow and Lane s (Cold Spring Harbor Laboratory Press, 1988); and Handbook Of Experimental Immunology, Volumes I-IV (D. M. Weir and C. C. Blackwell, eds., 1986).

As used herein, the term "glycan" refers to a polysaccharide, or oligosaccharide. Glycan is also used herein to refer to the carbohydrate portion of a glycoconjugate, such as a glycoprotein, glycolipid, glycopeptide, glycoproteome, peptidoglycan, lipopolysaccharide or a proteoglycan. Glycans usually consist solely of O-glycosidic linkages between monosaccharides. For example, cellulose is a glycan (or more specifically a glucan) composed of β-1,4-linked D-glucose, and chitin is a glycan composed of β-1,4-linked N-acetyl-D-glucosamine. Glycans can be homo or heteropolymers of monosaccharide residues, and can be linear or branched. Glycans can be found attached to proteins as in glycoproteins and proteoglycans. They are generally found on the exterior surface of cells. O- and N-linked glycans are very common in eukaryotes but may also be found, although less commonly, in prokaryotes. N-Linked glycans are found attached to the R-group nitrogen (N) of asparagine in the sequon. The sequon is a Asn-X-Ser or Asn-X-Thr sequence, where X is any amino acid except praline.

As used herein, the term "antigen" is defined as any substance capable of eliciting an immune response.

As used herein, the term "immunogenicity" refers to the ability of an immunogen, antigen, or vaccine to stimulate an immune response.

As used herein, the term "CD1d" refers to a member of the CD1 (cluster of differentiation 1) family of glycoproteins expressed on the surface of various human antigen-presenting cells. CDld presented lipid antigens activate natural killer T cells. CDld has a deep antigen-binding groove into which glycolipid antigens bind. CDld molecules expressed on dendritic cells can bind and present glycolipids, including alpha-GalCer analogs such as C34.

As used herein, the term "epitope" is defined as the parts of an antigen molecule which contact the antigen binding site of an antibody or a T cell receptor.

As used herein, the term "vaccine" refers to a preparation that contains an antigen, consisting of whole disease-causing organisms (killed or weakened) or components of such organisms, such as proteins, peptides, or polysaccharides, that is used to confer immunity against the disease that the organisms cause. Vaccine preparations can be natural, synthetic or derived by recombinant DNA technology.

As used herein, the term "antigen specific" refers to a property of a cell population such that supply of a particular antigen, or a fragment of the antigen, results in specific cell proliferation.

As used herein, the term "specifically binding," refers to the interaction between binding pairs (e.g., an antibody and an antigen). In various instances, specifically binding can be embodied by an affinity constant of about 10-6 moles/liter, about 10-7 moles/liter, or about 10-8 moles/liter, or less.

An "isolated" antibody is one which has been identified and separated and/or recovered from a component of its natural environment. Contaminant components of its natural environment are materials which would interfere with research, diagnostic or therapeutic uses for the antibody, and may include enzymes, hormones, and other proteinaceous or nonproteinaceous solutes. Thehe antibody will be purified (1) to greater than 95% by weight of antibody as determined by, for example, the Lowry method, and more than 99% by weight, (2) to a degree sufficient to obtain at least 15 residues of N-terminal or internal amino acid sequence by use of, for example, a spinning cup sequenator, or (3) to homogeneity by SDS-PAGE under reducing or nonreducing conditions using, for example, Coomassie blue or silver stain. Isolated antibody includes the antibody in situ within recombinant cells since at least one component of the antibody's natural environment will not be present. Ordinarily, however, isolated antibody will be prepared by at least one purification step.

The phrase "substantially similar," "substantially the same", "equivalent", or "substantially equivalent", as used herein, denotes a sufficiently high degree of similarity between two numeric values (for example, one associated with a molecule and the other associated with a reference/comparator molecule) such that one of skill in the art would consider the difference between the two values to be of little or no biological and/or statistical significance within the context of the biological characteristic measured by said values (e.g., Kd values, anti-viral effects, etc.). The difference between said two values is, for example, less than about 50%, less than about 40%, less than about 30%, less than about 20%, and/or less than about 10% as a function of the value for the reference/comparator molecule.

The phrase "substantially reduced," or "substantially different", as used herein, denotes a sufficiently high degree of difference between two numeric values (generally one associated with a molecule and the other associated with a reference/comparator molecule) such that one of skill in the art would consider the difference between the two values to be of statistical significance within the context of the biological characteristic measured by said values (e.g., Kd values). The difference between said two values is, for example, greater than about 10%, greater than about 20%, greater than about 30%, greater than about 40%, and/or greater than about 50% as a function of the value for the reference/comparator molecule.

"Binding affinity" generally refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention. Specific illustrative examples are described in the following.

In one example, the "Kd" or "Kd value" according to this invention is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of (125I)-labeled antigen in the presence of a titration series of unlabeled antigen, then capturing bound antigen with an anti-Fab antibody-coated plate (Chen, et al., (1999) J. Mol Biol 293:865-881). To establish conditions for the assay, microtiter plates (Dynex) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23° C.). In a non-adsorbent plate (Nunc #269620), 100 pM or 26 pM [125I]-antigen are mixed with serial dilutions of a Fab of interest (e.g., consistent with assessment of an anti-VEGF antibody, Fab-12, in Presta et al., (1997) Cancer Res. 57:4593-4599). The Fab of interest is then incubated overnight; however, the incubation may continue for a longer period (e.g., 65 hours) to insure that equilibrium is reached. Thereafter, the mixtures are transferred to the capture plate for incubation at room temperature (e.g., for one hour). The solution is then removed and the plate washed eight times with 0.1% Tween-20 in PBS. When the plates have dried, 150 µl/well of scintillant (MicroScint-20; Packard) is added, and the plates are counted on a Topcount gamma counter (Packard) for ten minutes. Concentrations of each Fab that give less than or equal to 20% of maximal binding are chosen for use in competitive binding assays. The Kd or Kd value is measured by using surface plasmon resonance assays using a BIAcore™-2000 or a BIAcore™-3000 (BIAcore, Inc., Piscataway, N.J.) at 25° C. with immobilized antigen CM5 chips at ^{∼}10 response units (RU). Briefly, carboxymethylated dextran biosensor chips (CM5, BIAcore Inc.) are activated with N-ethyl-N'-(3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (^{∼}0.2 µM) before injection at a flow rate of 5 µl/minute to achieve approximately 10 response units (RU) of coupled protein. Following the injection of antigen, 1 M ethanolamine is injected to block unreacted groups. In each experiment, a spot was activated and ethanolamine blocked without immobilizing protein, to be used for reference subtraction. For kinetics measurements, two-fold serial dilutions of Fab (0.78 nM to 500 nM) are injected in PBS with 0.05% Tween 20 (PBST) at 25° C. at a flow rate of approximately 25 µl/min. Association rates (kon) and dissociation rates (koff) are calculated using a simple one-to-one Langmuir binding model (BIAcore Evaluation Software version 3.2) by simultaneously fitting the association and dissociation sensorgrams. The equilibrium dissociation constant (Kd) is calculated as the ratio koff/kon. See, e.g., Chen, Y., et al., (1999) J. Mol Biol 293:865-881. If the on-rate exceeds 106 M-1s-1 by the surface plasmon resonance assay above, then the on-rate can be determined by using a fluorescent quenching technique that measures the increase or decrease in fluorescence emission intensity (excitation=295 nm; emission=340 nm, 16 nm band-pass) at 25° C. of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-Aminco spectrophotometer (ThermoSpectronic) with a stirred cuvette.

An "on-rate" or "rate of association" or "association rate" or "kon" according to this invention can also be determined with the same surface plasmon resonance technique described above using a BIAcore™-2000 or a BIAcore™-3000 (BIAcore, Inc., Piscataway, N.J.) at 25° C. with immobilized antigen CM5 chips at ^{∼}10 response units (RU). Briefly, carboxymethylated dextran biosensor chips (CM5, BIAcore Inc.) are activated with N-ethyl-N'-(3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (^{∼}0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% Tween 20 (PBST) at 25° C. at a flow rate of approximately 25 µl/min. Association rates (kon) and dissociation rates (koff) are calculated using a simple one-to-one Langmuir binding model (BIAcore Evaluation Software version 3.2) by simultaneously fitting the association and dissociation sensorgram. The equilibrium dissociation constant (Kd) was calculated as the ratio koff/kon. See, e.g., Chen, Y., et al., (1999)J. Mol Biol 293:865-881. However, if the on-rate exceeds 106 M-1s-1 by the surface plasmon resonance assay above, then the on-rate can be determined by using a fluorescent quenching technique that measures the increase or decrease in fluorescence emission intensity (excitation=295 nm; emission=340 nm, 16 nm band-pass) at 25° C. of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-Aminco spectrophotometer (ThermoSpectronic) with a stirred cuvette.

The term "vector," as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a phage vector. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "recombinant vectors"). In general, expression vectors of utility in recombinant DNA techniques are often in the form of plasmids. In the present specification, "plasmid" and "vector" may be used interchangeably as the plasmid is the most commonly used form of vector.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps," substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs and basic nucleoside analogs such as methyl riboside. One or more phosphodiester linkages may be replaced by alternative linking groups. These alternative linking groups include, but are not limited to, examples wherein phosphate is replaced by P(O)S ("thioate"), P(S)S ("dithioate"), "(O)NR2 ("amidate"), P(O)R, P(O)OR', CO or CH2 (" formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single-stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules which generally lack antigen specificity. Polypeptides of the latter kind are, for example, produced at low levels by the lymph system and at increased levels by myelomas.

The terms "antibody" and "immunoglobulin" are used interchangeably in the broadest sense and include monoclonal antibodies (e.g., full length or intact monoclonal antibodies), polyclonal antibodies, monovalent, multivalent antibodies, multispecific antibodies (e.g., bispecific antibodies so long as they exhibit the desired biological activity) and may also include certain antibody fragments (as described in greater detail herein). An antibody can be chimeric, human, humanized and/or affinity matured.

The "variable region" or "variable domain" of an antibody refers to the amino-terminal domains of heavy or light chain of the antibody. These domains are generally the most variable parts of an antibody and contain the antigen-binding sites.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework (FR). The variable domains of native heavy and light chains each comprise four FR regions, largely adopting a beta-sheet configuration, connected by three CDRs, which form loops connecting, and in some cases forming part of, the beta-sheet structure. The CDRs in each chain are held together in close proximity by the FR regions and, with the CDRs from the other chain, contribute to the formation of the antigen-binding site of antibodies (see Kabat et al., Sequences of Proteins of Immunological Interest, Fifth Edition, National Institute of Health, Bethesda, Md. (1991)). The constant domains are not involved directly in binding an antibody to an antigen, but exhibit various effector functions, such as participation of the antibody in antibody-dependent cellular toxicity.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-combining sites and is still capable of cross-linking antigen.

"Fv" is the minimum antibody fragment which contains a complete antigen-recognition and -binding site. In a two-chain Fv species, this region consists of a dimer of one heavy- and one light-chain variable domain in tight, non-covalent association. In a single-chain Fv species, one heavy- and one light-chain variable domain can be covalently linked by a flexible peptide linker such that the light and heavy chains can associate in a "dimeric" structure analogous to that in a two-chain Fv species. It is in this configuration that the three CDRs of each variable domain interact to define an antigen-binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequences of the constant domains of their heavy chains, antibodies (immunoglobulins) can be assigned to different classes. There are five major classes of immunoglobulins: IgA, IgD, IgE, IgG and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, δ, ε, γ, and µ, respectively. The subunit structures and three-dimensional configurations of different classes of immunoglobulins are well known and described generally in, for example, Abbas et al. Cellular and Mol. Immunology, 4th ed. (2000). An antibody may be part of a larger fusion molecule, formed by covalent or non-covalent association of the antibody with one or more other proteins or peptides.

The terms "full length antibody," "intact antibody" and "whole antibody" are used herein interchangeably, to refer to an antibody in its substantially intact form, not antibody fragments as defined below. The terms particularly refer to an antibody with heavy chains that contain the Fc region.

"Antibody fragments" comprise only a portion of an intact antibody, wherein the portion retains at least one, and as many as most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an in vivo half life substantially similar to an intact antibody. For example, such an antibody fragment may comprise an antigen binding arm linked to an Fc sequence capable of conferring in vivo stability to the fragment.

The term "monoclonal antibody" as used herein refers to an antibody obtained from a population of substantially homogeneous antibodies, e.g., the individual antibodies comprising the population are identical except for possible naturally occurring mutations that may be present in minor amounts or comprising only homogeneous glycoform profile (having only a single glycan or single glycan profile on a glycoantibody in a population). Examples of homogeneous antibody composition to enhance the effector functions by using the 2,3- and 2,6-sialyl and defucosylated complex bi-antennary glycans at the Fc-297 position are described in US 12/959, 351. Thus, the modifier "monoclonal" indicates the character of the antibody as not being a mixture of discrete antibodies. Such monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones or recombinant DNA clones. It should be understood that the selected target binding sequence can be further altered, for example, to improve affinity for the target, to humanize the target binding sequence, to improve its production in cell culture, to reduce its immunogenicity in vivo, to create a multispecific antibody, etc., and that an antibody comprising the altered target binding sequence is also a monoclonal antibody of this invention. In contrast to polyclonal antibody preparations which typically include different antibodies directed against different determinants (epitopes), each monoclonal antibody of a monoclonal antibody preparation is directed against a single determinant on an antigen. In addition to their specificity, the monoclonal antibody preparations are advantageous in that they are typically uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (e.g., Kohler et al., Nature, 256: 495 (1975); Harlow et al., Antibodies: A Laboratory Manual, (Cold Spring Harbor Laboratory Press, 2nd ed. 1988); Hammerling et al., in: Monoclonal Antibodies and T-Cell hybridomas 563-681 (Elsevier, N.Y., 1981)), recombinant DNA methods (see, e.g., U.S. Pat. No. 4,816,567), phage display technologies (See, e.g., Clackson et al., Nature, 352: 624-628 (1991); Marks et al., J. Mol. Biol. 222: 581-597 (1992); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132 (2004), and technologies for producing human or human-like antibodies in animals that have parts or all of the human immunoglobulin loci or genes encoding human immunoglobulin sequences (see, e.g., WO98/24893; WO96/34096; WO96/33735; WO91/10741; Jakobovits et al., Proc. Natl. Acad. Sci. USA 90: 2551 (1993); Jakobovits et al., Nature 362: 255-258 (1993); Bruggemann et al., Year in Immunol. 7:33 (1993); U.S. Pat. Nos. 5,545,807; 5,545,806; 5,569,825; 5,625,126; 5,633,425; 5,661,016; Marks et al., Bio. Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996) and Lonberg and Huszar, Intern. Rev. Immunol. 13: 65-93 (1995).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al., Proc. Natl. Acad. Sci. USA 81:6851-6855 (1984)).

Humanized" forms of non-human (e.g., murine) antibodies are chimeric antibodies that contain minimal sequence derived from non-human immunoglobulin. In one embodiment, a humanized antibody is a human immunoglobulin (recipient antibody) in which residues from a hypervariable region of the recipient are replaced by residues from a hypervariable region of a non-human species (donor antibody) such as mouse, rat, rabbit or nonhuman primate having the desired specificity, affinity, and/or capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994).

The term "hypervariable region", "HVR", or "HV", when used herein refers to the regions of an antibody variable domain which are hypervariable in sequence and/or form structurally defined loops. Generally, antibodies comprise six hypervariable regions; three in the VH (H1, H2, H3), and three in the VL (L1, L2, L3). A number of hypervariable region delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, Md. (1991)). Chothia refers instead to the location of the structural loops (Chothia and Lesk J. Mol. Biol. 196:901-917 (1987)). The AbM hypervariable regions represent a compromise between the Kabat CDRs and Chothia structural loops, and are used by Oxford Molecular's AbM antibody modeling software. The "contact" hypervariable regions are based on an analysis of the available complex crystal structures. The residues from each of these hypervariable regions are noted below.
Loop Kabat AbM Chothia Contact
   L1 L24-L34 L24-L34 L26-L32 L30-L36
   L2 L50-L56 L50-L56 L50-L52 L46-L55
   L3 L89-L97 L89-L97 L91-L96 L89-L96
   H1 H31-H35B H26-H35B H26-H32 H30-H35B
(Kabat Numbering)
   H1 H31-H35 H26-H35 H26-H32 H30-H35
(Chothia Numbering)
   H2 H50-H65 H50-H58 H53-H55 H47-H58
   H3 H95-H102 H95-H102 H96-H101 H93-H101

Hypervariable regions may comprise "extended hypervariable regions" as follows: 24-36 or 24-34 (L1), 46-56 or 50-56 or 49-56 (L2) and 89-97 or 89-96 (L3) in the VL and 26-35 (H1), 50-65 or 49-65 (H2) and 93-102, 94-102, or 95-102 (H3) in the VH. The variable domain residues are numbered according to Kabat et al., supra, for each of these definitions.

"Framework" or "FR" residues are those variable domain residues other than the hypervariable region residues as herein defined.

The term "variable domain residue numbering as in Kabat" or "amino acid position numbering as in Kabat," and variations thereof, refers to the numbering system used for heavy chain variable domains or light chain variable domains of the compilation of antibodies in Kabat et al., Sequences of Proteins of Immunological Interest, 5th Ed. Public Health Service, National Institutes of Health, Bethesda, Md. (1991). Using this numbering system, the actual linear amino acid sequence may contain fewer or additional amino acids corresponding to a shortening of, or insertion into, a FR or HVR of the variable domain. For example, a heavy chain variable domain may include a single amino acid insert (residue 52a according to Kabat) after residue 52 of H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains which enables the scFv to form the desired structure for antigen binding. For a review of scFv see Pluckthun, in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies are described more fully in, for example, EP 404,097; WO93/1161; and Hollinger et al., Proc. Natl. Acad. Sci. USA 90: 6444-6448 (1993).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues.

An "affinity matured" antibody is one with one or more alterations in one or more HVRs thereof which result in an improvement in the affinity of the antibody for antigen, compared to a parent antibody which does not possess those alteration(s). In one embodiment, an affinity matured antibody has nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

A "blocking" antibody or an "antagonist" antibody is one which inhibits or reduces biological activity of the antigen it binds. Certain blocking antibodies or antagonist antibodies substantially or completely inhibit the biological activity of the antigen.

An "agonist antibody", as used herein, is an antibody which mimics at least one of the functional activities of a polypeptide of interest.

A "disorder" is any condition that would benefit from treatment with an antibody of the invention. This includes chronic and acute disorders or diseases including those pathological conditions which predispose the mammal to the disorder in question. Non-limiting examples of disorders to be treated herein include cancer.

The terms "cell proliferative disorder" and "proliferative disorder" refer to disorders that are associated with some degree of abnormal cell proliferation. In one embodiment, the cell proliferative disorder is cancer.

"Tumor," as used herein, refers to all neoplastic cell growth and proliferation, whether malignant or benign, and all pre-cancerous and cancerous cells and tissues. The terms "cancer," "cancerous," "cell proliferative disorder," "proliferative disorder" and "tumor" are not mutually exclusive as referred to herein.

The terms "cancer" and "cancerous" refer to or describe the physiological condition in mammals that is typically characterized by unregulated cell growth/proliferation. Examples of cancer include, but are not limited to, carcinoma, lymphoma (e.g., Hodgkin's and non-Hodgkin's lymphoma), blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing or decreasing inflammation and/or tissue/organ damage, decreasing the rate of disease progression, amelioration or palliation of the disease state, and remission or improved prognosis. In some embodiments, antibodies of the invention are used to delay development of a disease or disorder.

An "individual" or a "subject" is a vertebrate. In certain embodiments, the vertebrate is a mammal. Mammals include, but are not limited to, farm animals (such as cows), sport animals, pets (such as cats, dogs, and horses), primates, mice and rats. In certain embodiments, the vertebrate is a human.

"Mammal" for purposes of treatment refers to any animal classified as a mammal, including humans, domestic and farm animals, and zoo, sports, or pet animals, such as dogs, horses, cats, cows, etc. In certain embodiments, the mammal is human.

An "effective amount" refers to an amount effective, at dosages and for periods of time necessary, to achieve the desired therapeutic or prophylactic result.

A "therapeutically effective amount" of a substance/molecule of the invention may vary according to factors such as the disease state, age, sex, and weight of the individual, and the ability of the substance/molecule, to elicit a desired response in the individual. A therapeutically effective amount is also one in which any toxic or detrimental effects of the substance/molecule are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount would be less than the therapeutically effective amount.

The term "cytotoxic agent" as used herein refers to a substance that inhibits or prevents the function of cells and/or causes destruction of cells. The term is intended to include radioactive isotopes (e.g., At211, 1131, 1125, Y90, Re186, Re188, Sm153, Bi212, P32, Pb212 and radioactive isotopes of Lu), chemotherapeutic agents (e.g., methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolyticenzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gamma1I and calicheamicin omegaI1 (see, e.g., Agnew, Chem. Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., TAXOL® paclitaxel (Bristol-Myers Squibb Oncology, Princeton, N.J.), ABRAXANE™ Cremophor-free, albumin-engineered nanoparticle formulation of paclitaxel (American Pharmaceutical Partners, Schaumberg, I11.), and TAXOTERE® doxetaxel (Rhône-Poulenc Rorer, Antony, France); chloranbucil; gemcitabine (GEMZAR®); 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluoromethylornithine (DMFO); retinoids such as retinoic acid; capecitabine (XELODA®); pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN™) combined with 5-FU and leucovovin.

### Antibodies Triple-Targeting Globo H, SSEA3 and SSEA-4 (not part of the present invention

One aspect of the present disclosure features the new antibody triple-targeting Globo H, SSEA3 and SSEA-4. The triple-targeting antibody specifically binds to Fucα→ 2Galβ1→ 3GalNAcβ1→ 3Galα1→ 4Galβ1→ 4Glcβ1 (Globo H hexasaccharide) and Galβ1→ 3GalNAcβ1→ 3Galα1→ 4Galβ1→ 4Glcβ1 (SSEA-3 pentasaccharide) and Neu5Acα2→ 3Galβ1→ 3GalNAcβ1→ 3Galα1→ 4Galβ1→ 4Glcβ1 (SSEA-4 hexasaccharide).In one example, the triple-targeting antibody is mAb 651, which is not part of the present invention.

The mAb 651 is a mouse monoclonal antibody, produced by the hybridoma cell line. The triple-targeting antibody described herein can contain the same V_{H} and V_{L} chains as antibody MC651.

**Table 1. Amino Acid and Nucleotide Sequences of Antibody MC651 (not part of the present invention)**

| SEQ ID NO | DESCRIPTION | SEQUENCE |
|---|---|---|
| 11 | MC651 VH nucleotide sequence | GAGGTCCAGCTGCAACAATCTGGGTCTGTGCTGGTGAGGCCTGGAG CTTCAGTGAAGCTGTCCTGCAAGGCTTCTGGCTACACCTTCACCAAC TCCTGGATGCACTGGGCGAAGCAGAGGCCTGGACAAGGCCTTGTGT GGATTGGAGAGATTGATCCTAATACTGGTAATACTAACTACAATGA GAACTTCAAGGGCAAGGCCACACTGACTGTAGACACATCCTCCACC ACAGCCTACGTGGATCTCAGCAGCCTGACATCTGAAGACTCTGCGG TCTATTACTGTGCAAGAGGACTCGGGCTACTTGTTTACTGGGGCCAA GGGACTCTGGTCACTGTCTCTGCA |
| 12 | MC651 VL nucleotide sequence | CAAATTGTTCTCACCCAGTCTCCAGCAATCCTGTCTGCATCTCCAGG GGAGAAGGTCACAATGACTTGCAGGGCCAGCTCAAGTGTAAGTTAC ATGCACTGGTACCAGCAGAAGCCAGGATCCTCCCCCAAACCCTGGA TTTATGTCACATCCAACCTGACTTCTGGAGTCCCTGTTCGCTTCAGT GGCAGTGGGTCTGGGACCTCTTACTCTCTCACAATCAGCAGAGTGG AGGCTGAAGATGCTGCCACTTATTACTGCCAGCAGTGGAGTAATAA CCCGTGGACGTTCGGTGGAGGCACCAAGCTGGAAATCAAA |
| 13 | MC651 VH amino acid sequence | EVQLQQSGSVLVRPGASVKLSCKASGYTFTNSWMHWAKQRPGQGLV WIGEIDPNTGNTNYNENFKGKATLTVDTSSTTAYVDLSSLTSEDSAVY YCARGLGLLVYWGQGTLVTVSA |
| 14 | MC651 VL amino acid sequence | QIVLTQSPAILSASPGEKVTMTCRASSSVSYMHWYQQKPGSSPKPWIY VTSNLTSGVPVRFSGSGSGTSYSLTISRVEAEDAATYYCQQWSNNPWT FGGGTKLEIK |
| 15 | MC651 VL CDR1 | SSVSY |
| 16 | MC651 VL CDR2 | VTS |
| 17 | MC651 VL CDR3 | QQWSNNPWT |
| 18 | MC651 VH CDR1 | GYTFTNSW |
| 19 | MC651 VH CDR2 | IDPNTGNT |
| 20 | MC651 VH CDR3 | ARGLGLLVY |

### Antibodies Dual-Targeting Globo H and SSEA3 (not part of the present invention)

One aspect of the present disclosure features the new antibodies dual-targeting Globo H and SSEA3. The dual-targeting antibody specifically binds to Fucα1→ 2Galβ1→ 3GalNAcβ1→ 3Galα1→ 4Galβ1→ 4Glcβ1 (Globo H hexasaccharide) and Galβ1→ 3GalNAcβ1→ 3Galα1→ 4Galβ1→ 4Glcβ1 (SSEA-3 pentasaccharide).In one example, the dual-targeting antibody is mAb 273, which is not part of the present invention.

The mAb 273 is a mouse monoclonal antibody, produced by the hybridoma cell line. The dual-targeting antibodies described herein can contain the same V_{H} and V_{L} chains as antibody MC273.

**Table 2. Amino Acid and Nucleotide Sequences of Antibody MC273 (not part of the present invention)**

| SEQ ID NO | DESCRIPTION | SEQUENCE |
|---|---|---|
| 1 | MC273 VH nucleotide sequence | CAGGTGCAGCTGAAGCAGTCTGGACCTGAGCTAGTGAAGACTGGGG CTTCAGTGAAGATATCCTGCAAGGCTTCTGGTTACTCATTCACTGGT TACTACATGCACTGGGTCAAGCAGAGCCATGGAAAGAGCCTTGAGT GGATTGGATATATTAGTTGTTACAATGGTGGTACTAGGTACAACCT GAAGTTCAAGGGCAAGGCCACATTTACTGTAGACACATCCTCCACC ACAGCCTACATGCAGTTCAACAACCTGACATCTGAAGACTCTGCGG TCTATTACTGTGCAAGAGGGGGGTACGACGAGGGTGACTACTGGGG CCAAGGCACCACTCTCACAGTCTCCTCA |
| 2 | MC273 VL nucleotide sequence | GATATTGTAATGACACAGTCTCCCAAATCCATATTCATGTCAGTTGG AGAGAGGGTCACCTTGAGCTGCAAGGCCAGTGAGAATGTGGGTACT TATGTATCCTGGTATCAACAGAAACCAGAGCAGTCTCCTAAACTGA TGATATACGGGGCATCCAACCGGAACACTGGGGTCCCCGATCGCTT CACAGGCAGTGGATCTGCAACAGATTTCACTCTGACCATCAGCAGT GTGCAGGCTGAAGACCTTGCAGATTATCACTGTGGACAGAGTTACA CCTATCCGTACACGTTCGGAGGGGGGACCAAGCTGGAAATCAAA |
| 3 | MC273 VH amino acid sequence | QVQLKQSGPELVKTGASVKISCKASGYSFTGYYMHWVKQSHGKSLEW IGYISCYNGGTRYNLKFKGKATFTVDTSSTTAYMQFNNLTSEDSAVYY CARGGYDEGDYWGQGTTLTVSS |
| 4 | MC273 VL amino acid sequence | DIVMTQSPKSIFMSVGERVTLSCKASENVGTYVSWYQQKPEQSPKLMI YGASNRNTGVPDRFTGSGSATDFTLTISSVQAEDLADYHCGQSYTYPY TFGGGTKLEIK |
| 5 | MC273 VL CDR1 | ENVGTY |
| 6 | MC273 VL CDR2 | GAS |
| 7 | MC273 VL CDR3 | GQSYTYPYT |
| 8 | MC273 VH CDR1 | GYSFTGYY |
| 9 | MC273 VH CDR2 | ISCYNGGT |
| 10 | MC273 VH CDR3 | ARGGYDEGDY |

### Antibodies Specific To SSEA4

One aspect of the present disclosure features the new antibodies specific to SSEA-4. The anti-SSEA-4 antibody binds to Neu5Acα2→ 3Galβ1→ 3GalNAcβ1→ 3Galα1→ 4Galβ1→ 4Glcβ1→ (SSEA-4 hexasaccharide). In some examples, the antibody is capable of binding Neu5Gcα2→ 3Galβ1→ 3GalNAcβ1→ 3Galα1→ 4Galβ1→ 4Glcβ1→ (an analogue of SSEA-4 hexasaccharide). Preferrably, the antibody is not a mouse IgG3 (e.g., mAb MC-831-70), and the antibody is not amouse IgM(e.g., anti-RM1). Examples of the antibodies include, but are not limited to, mAbs 45, which is not part of the present invention, and 48.

Monoclonal antibody MC45, which is not part of the present invention, is an anti-SSEA-4 mouse monoclonal antibody, produced by the hybridoma cell line. The anti-SSEA-4 antibody described herein can contain the same V_{H} and V_{L} chains as antibody MC45.

**Table 3. Amino Acid and Nucleotide Sequences of Antibody MC45 (not part of the present invention)**

| SEQ ID NO | DESCRIPTION | SEQUENCE |
|---|---|---|
| 21 | MC45 VH nucleotide sequence | CAGGTGCAGCTGAAGGAGTCAGGACCTGGCCTGGTGGCGCCCTCAC AGAGCCTGTCCATCACATGCACTGTCTCAGGGTTCTCATTAAGCAG ATATGGTGTAAGCTGGGTTCGCCAGCCTCCAGGAAAGGGTCTGGAG TGGCTGGGAGTAATATGGGGTGACGGGAGCACAAATTATCATTCAG CTCTCATATCCAGACTGAGCATCAGCAAGGATAACTCCAAGAGCCA AGTTTTCTTAAAACTGAACAGTCTGCAAACTGATGACACAGCCACG TACTACTGTGCCATGACTGGGACAGCTTACTGGGGCCAAGGGACTC TGGTCACTGTCTCTGCA |
| 22 | MC45 VL nucleotide sequence | CAAATTGTTCTCACCCAGTCTCCAGCAATCATGTCTGCATCTCCAGG GGAGAAGGTCACCATGACCTGCAGTGCCAGCTCAAGTGTAAATTAC ATGCACTGGTACCAGCAGAAGTCAGGCACCTCCCCCAAAAGATGGA TTTATGACACATCCAAACTGGCTTCTGGAGTCCCTGCTCGCTTCAGT GGCAGTGGGTCTGGGACCTCTTACTCTCTCACAATCAGCGGCATGG AGGCTGAAGATGCTGCCACTTATTACTGCCACCAGTGGAATAGTAG CCCACACACGTTCGGAGGGGGGACCAAGCTGGAAATAAAA |
| 23 | MC45 VH amino acid sequence | QVQLKESGPGLVAPSQSLSITCTVSGFSLSRYGVSWVRQPPGKGLEWL GVIWGDGSTNYHSALISRLSISKDNSKSQVFLKLNSLQTDDTATYYCA MTGTAYWGQGTLVTVSA |
| 24 | MC45 VL amino acid sequence | QIVLTQSPAIMSASPGEKVTMTCSASSSVNYMHWYQQKSGTSPKRWIY DTSKLASGVPARFSGSGSGTSYSLTISGMEAEDAATYYCHQWNSSPHT FGGGTKLEIK |
| 25 | MC45 VL CDR1 | SSVNY |
| 26 | MC45 VL CDR2 | DTS |
| 27 | MC45 VL CDR3 | HQWNSSPHT |
| 28 | MC45 VH CDR1 | GFSLSRYG |
| 29 | MC45 VH CDR2 | IWGDGST |
| 30 | MC45 VH CDR3 | AMTGTAY |

Monoclonal antibody MC48 is produced by the hybridoma cell line. The anti-SSEA-4 antibody described herein can contain the same V_{H} and V_{L} chains as antibody MC48 or variants thereof. Antibodies binding to the same epitope as MC48 are also within the scope of this disclosure.

**Table 4. Amino Acid and Nucleotide Sequences of Antibody MC48 (not part of the invention).**

| SEQ ID NO | DESCRIPTION | SEQUENCE |
|---|---|---|
| 41 | MC48 VH nucleotide sequence | CAGGTGCAGCTGAAGGAGTCAGGACCTGGCCTGGTGGCGCCCTCAC AGAGCCTGTCCATCACATGCACTGTCTCAGGGTTCTCATTAACCAGC TATGGTGTAAGCTGGGTTCGCCAGCCTCCAGGAAAGGGTCTGGAGT GGCTGGGAGTAATATGGGGTGAGGGAGCACAAATTATCATTCAGT TCTCATATCCAGACTGACCATTAGTAAGGATAACTCCAAGAGCCAA GTTTTCTTAAAACTGAACAGTCTGCAAACTGATGACACAGCCACGT ACTACTGTGCCATGACTGGGACAGCTTACTGGGGCCAAGGGACTCT GGTCACTGTCTCTGCA |
| 42 | MC48 VL nucleotide sequence | CAAATTGTTCTCACCCAGTCTCCAGCAATCATGTCTGCATCTCCAGG GGAGAAGGTCACCATGACCTGCAGTGCCAGCTCAAGTGTAAGTTAC ATGCACTGGTACCAGCAGAAGTCAGGCACCTCCCCCAAAAGATGGA TTTATGACACATCCAAACTGTCTTCTGGAGTCCCTGGTCGCTTCAGT GGCAGTGGGTCTGGGACCTCTTACTCTCTCACAATCAGCAGGTTGG AGGCTGAAGATGCTGCCACTTATTACTGCCATCAGTGGAGTAGTAG TCCACACACGTTCGGAGGGGGGACCAAGTTGGAGATAAAA |
| 43 | MC48 VH amino acid sequence | QVQLKESGPGLVAPSQSLSITCTVSGFSLTSYGVSWVRQPPGKGLEWL GVIWGEGSTNYHSVLISRLTISKDNSKSQVFLKLNSLQTDDTATYYCA MTGTAYWGQGTLVTVSA |
| 44 | MC48 VL amino acid sequence | QIVLTQSPAIMSASPGEKVTMTCSASSSVSYMHWYQQKSGTSPKRWIY DTSKLSSGVPGRFSGSGSGTSYSLTISRLEAEDAATYYCHQWSSSPHTF GGGTKLEIK |
| 45 | MC48 VL CDR1 | SSVSY |
| 46 | MC48 VL CDR2 | DTS |
| 47 | MC48 VL CDR3 | HQWSSSPHT |
| 48 | MC48 VH CDR1 | GFSLTSYG |
| 49 | MC48 VH CDR2 | IWGEGST |
| 50 | MC48 VH CDR3 | AMTGTAY |

One aspect of the present disclosure provides humanized glycoantibodies based on the modification of the MC48. Exemplars and their amino acid and nucleic acid structures/sequences are provided below:

**Table 17-0. Amino Acid and Nucleotide Sequences of Mouse Monoclonal Antibody MC48 (not part of the invention).**

| SEQ ID NO | DESCRIPTION | SEQUENCE |
|---|---|---|
| 41 | MC48 VH nucleotide sequence | CAGGTGCAGCTGAAGGAGTCAGGACCTGGCCTGGTGGCGCCCTCAC AGAGCCTGTCCATCACATGCACTGTCTCAGGGTTCTCATTAACCAGC TATGGTGTAAGCTGGGTTCGCCAGCCTCCAGGAAAGGGTCTGGAGT GGCTGGGAGTAATATGGGGTGAGGGGAGCACAAATTATCATTCAGT TCTCATATCCAGACTGACCATTAGTAAGGATAACTCCAAGAGCCAA GTTTTCTTAAAACTGAACAGTCTGCAAACTGATGACACAGCCACGT ACTACTGTGCCATGACTGGGACAGCTTACTGGGGCCAAGGGACTCT GGTCACTGTCTCTGCA |
| 42 | MC48 VL nucleotide sequence | CAAATTGTTCTCACCCAGTCTCCAGCAATCATGTCTGCATCTCCAGG GGAGAAGGTCACCATGACCTGCAGTGCCAGCTCAAGTGTAAGTTAC ATGCACTGGTACCAGCAGAAGTCAGGCACCTCCCCCAAAAGATGGA TTTATGACACATCCAAACTGTCTTCTGGAGTCCCTGGTCGCTTCAGT |
| | | GGCAGTGGGTCTGGGACCTCTTACTCTCTCACAATCAGCAGGTTGG AGGCTGAAGATGCTGCCACTTATTACTGCCATCAGTGGAGTAGTAG TCCACACACGTTCGGAGGGGGGACCAAGTTGGAGATAAAA |
| 43 | MC48 VH amino acid sequence | QVQLKESGPGLVAPSQSLSITCTVSGFSLTSYGVSWVRQPPGKGLEWL GVIWGEGSTNYHSVLISRLTISKDNSKSQVFLKLNSLQTDDTATYYCA MTGTAYWGQGTLVTVSA |
| 44 | MC48 VL amino acid sequence | QIVLTQSPAIMSASPGEKVTMTCSASSSVSYMHWYQQKSGTSPKRWIY DTSKLSSGVPGRFSGSGSGTSYSLTISRLEAEDAATYYCHQWSSSPHTF GGGTKLEIK |
| 45 | MC48 VL CDR1 | SSVSY |
| 46 | MC48 VL CDR2 | DTS |
| 47 | MC48 VL CDR3 | HQWSSSPHT |
| 48 | MC48 VH CDR1 | GFSLTSYG |
| 49 | MC48 VH CDR2 | IWGEGST |
| 50 | MC48 VH CDR3 | AMTGTAY |

**Table 17-1 Amino Acid and Nucleotide Sequences of Humanized Monoclonal Antibody MC48 (1^{st}) (not part of the invention).**

| SEQ ID NO | DESCRIPTION | SEQUENCE |
|---|---|---|
| 115 | hMC48 VH nucleotide sequence | CAGGTGCAGCTGCAAGAGTCAGGACCTGGCCTGGTGAAACCCTCAG AAACTCTGTCCCTTACATGCACTGTCTCAGGGTTCTCATTAACCAGC TATGGTGTAAGCTGGATTCGCCAGCCTCCAGGAAAGGGTCTGGAGT GGATTGGAGTAATATGGGGTGAGGGGAGCACAAATTATCATTCAGT TCTCATATCCAGACTGACCATTAGTGTGGATACCTCCAAGAATCAAT TTAGCTTAAAACTGAGCAGTGTTACCGCTGCTGACACAGCCGTTTAC TACTGTGCCATGACTGGGACAGCTTACTGGGGCCAAGGGACTCTGG TCACTGTCTCTAGC |
| 116 | hMC48 VL nucleotide sequence | GAGATTGTGCTGACCCAGAGCCCTGCCACACTGTCACTGAGCCCAG GCGAGCGAGCCACACTGTCCTGTTCTGCTAGCTCCTCTGTCTCCTAC ATGCATTGGTATCAGCAGAAGCCAGGACTGGCACCACGACTGCTGA TCTATGACACTTCTAAACTGAGTTCAGGCATTCCCGCCAGATTCAGT GGCTCAGGGAGCGGAACCGACTTTACTCTGACCATTAGCTCCCTGG AGCCTGAAGATTTCGCCGTGTACTATTGCCATCAGTGGTCATCAAGC CCTCATACCTTCGGGGGGGGGACTAAGGTGGAAATCAAACGC |
| 117 | hMC48 VH amino acid sequence | QVQLQESGPGLVKPSETLSLTCTVSGFSLTSYGVSWIRQPPGKGLEWI GVIWGEGSTNYHSVLISRLTISVDTSKNQFSLKLSSVTAADTAVYYCA MTGTAYWGQGTLVTVSS |
| 118 | hMC48 VL amino acid sequence | EIVLTQSPATLSLSPGERATLSCSASSSVSYMHWYQQKPGLAPRLLIY DTSKLSSGIPARFSGSGSGTDFTLTISSLEPEDFAVYYCHQWSSSPHTFG GGTKVEIKR |
| 119 | hMC48 VL CDR1 | SSVSY |
| 120 | hMC48 VL CDR2 | DTS |
| 121 | hMC48 VL CDR3 | HQWSSSPHT |
| 122 | hMC48 VH CDR1 | GFSLTSYG |
| 123 | hMC48 VH CDR2 | IWGEGST |
| 124 | hMC48 VH CDR3 | AMTGTAY |

**Table 17-2. Amino Acid and Nucleotide Sequences of Humanized Monoclonal Antibody MC48 (2^{nd}) (not part of the invention).**

| SEQ ID NO | DESCRIPTION | SEQUENCE |
|---|---|---|
| 125 | hMC48 VH nucleotide sequence | CAGGTGCAGCTGAAGCAGAGCGGACCTGGCCTGGTGCAGCCCTCAC AGAGCCTGAGCATCACTTGTACCGTCAGTGGATTCTCCCTGACATCT TACGGCGTGTCTTGGGTCAGGCAGAGCCCTGGCAAGGGGCTGGAGT GGCTGGGCGTGATCTGGGGAGAAGGCTCAACTAACTATCACAGCGT CCTGATCAGTCGCCTGTCAATTAACAAGGACAATTCTAAAAGTCAG GTGTTCTTTAAAATGAACAGCCTGCAGTCCAATGATACCGCCATCTA CTATTGCGCTATGACCGGCACAGCATACTGGGGGCAGGGAACACTG GTGACTGTCTCCGCT |
| 126 | hMC48 VL nucleotide sequence | GAGATTGTGCTGACCCAGAGCCCTGCCACACTGTCACTGAGCCCAG GCGAGCGAGCCACACTGTCCTGTTCTGCTAGCTCCTCTGTCTCCTAC ATGCATTGGTATCAGCAGAAGCCAGGACTGGCACCACGACTGCTGA TCTATGACACTTCTAAACTGAGTTCAGGCATTCCCGCCAGATTCAGT GGCTCAGGGAGCGGAACCGACTTTACTCTGACCATTAGCTCCCTGG AGCCTGAAGATTTCGCCGTGTACTATTGCCATCAGTGGTCATCAAGC CCTCATACCTTCGGGGGGGGGACTAAGCTGGAAATCAAACGC |
| 127 | hMC48 VH amino acid sequence | QVQLKQSGPGLVQPSQSLSITCTVSGFSLTSYGVSWVRQSPGKGLEWL GVIWGEGSTNYHSVLISRLSINKDNSKSQVFFKMNSLQSNDTAIYYCA MTGTAYWGQGTLVTVSA |
| 128 | hMC48 VL amino acid sequence | EIVLTQSPATLSLSPGERATLSCSASSSVSYMHWYQQKPGLAPRLLIY DTSKLSSGIPARFSGSGSGTDFTLTISSLEPEDFAVYYCHQWSSSPHTFG GGTKVLEIKR |
| 129 | hMC48 VL CDR1 | SSVSY |
| 130 | hMC48 VL CDR2 | DTS |
| 131 | hMC48 VL CDR3 | HQWSSSPHT |
| 132 | hMC48 VH CDR1 | GFSLTSYG |
| 133 | hMC48 VH CDR2 | IWGEGST |
| 134 | hMC48 VH CDR3 | AMTGTAY |

**Table 17-3. Amino Acid and Nucleotide Sequences of Humanized Monoclonal Antibody MC48 (3^{rd}) (not part of the invention).**

| SEQ ID NO | DESCRIPTION | SEQUENCE |
|---|---|---|
| 135 | hMC48 VH nucleotide sequence | CAGGTGCAGCTGCAGGAAAGCGGACCCGGACTGGTGAAACCTAGC GAAACACTGAGCCTGACTTGTACCGTGAGCGGATTTTCCCTGACCTC TTATGGAGTGAGCTGGATCAGACAGCCCCCTGGCAAGGGACTGGAG TGGATCGGCGTGATTTGGGGAGAAGGCTCCACAAACTATCACAGTG TCCTGATCTCACGACTGACTATTTCTAAGGACAACTCTAAAAGTCAG GTCTTCCTGAAACTGAATAGTCTGCAGACTGACGATACCGCTACAT ACTATTGCGCAATGACAGGGACAGCATACTGGGGACAGGGAACCCT GGTGACAGTCAGCTCC |
| 136 | hMC48 VL nucleotide sequence | CAGATCGTGCTGACACAGTCCCCTGCAATTATGTCAGCCAGCCCAG GGGAAAAGGTGACAATGACTTGTAGTGCTTCTAGTTCAGTCTCATA CATGCATTGGTATCAGCAGAAGCCAGGCCTGGCCCCCAGACTGCTG ATCTACGACACCTCCAAACTGAGCTCCGGCGTGCCCGGGAGATTTT CCGGCTCTGGGAGTGGAACTTCATATAGCCTGACCATTTCTAGGCTG GAGGCCGAAGATGCCGCTACATACTATTGCCACCAGTGGAGCAGTA GCCCCCATACATTCGGAGGCGGGACCAAAGTGGAAATCAAACGC |
| 137 | hMC48 VH amino acid sequence | QVQLQESGPGLVKPSETLSLTCTVSGFSLTSYGVSWIRQPPGKGLEWI GVIWGEGSTNYHSVLISRLTISKDNSKSQVFLKLNSLQTDDTATYYCA MTGTAYWGQGTLVTVSS |
| 138 | hMC48 VL amino acid sequence | QIVLTQSPAIMSASPGEKVTMTCSASSSVSYMHWYQQKPGLAPRLLIY DTSKLSSGVPGRFSGSGSGTSYSLTISRLEAEDAATYYCHQWSSSPHTF GGGTKVEIKR |
| 139 | hMC48 VL CDR1 | SSVSY |
| 140 | hMC48 VL CDR2 | DTS |
| 141 | hMC48 VL CDR3 | HQWSSSPHT |
| 142 | hMC48 VH CDR1 | GFSLTSYG |
| 143 | hMC48 VH CDR2 | IWGEGST |
| 144 | hMC48 VH CDR3 | AMTGTAY |

**Table 17-4. Amino Acid and Nucleotide Sequences of Humanized Monoclonal Antibody MC48 (4^{th})**

| SEQ ID NO | DESCRIPTION | SEQUENCE |
|---|---|---|
| 145 | hMC48 VH nucleotide sequence | CAGGTCCAGCTGAAAGAGAGCGGCCCGGACTGGTCGCCCCTTCAC AGAGCCTGAGCATTACTTGCACCGTGAGCGGATTTTCACTGACCAG CTACGGAGTGAGCTGGATTAGACAGCCTCCTGGCAAGGGACTGGAG TGGATCGGCGTGATTTGGGGAGAAGGCAGCACCAACTATCACAGTG TCCTGATCTCACGCCTGACAATTTCCAAGGACAACAGCAAATCCCA GGTCTTCCTGAAACTGAATTCTCTGCAGACTGACGATACCGCTACAT ACTATTGCGCAATGACAGGGACAGCATACTGGGGACAGGGAACCCT GGTGACAGTCAGTAGT |
| 146 | hMC48 VL nucleotide sequence | CAGATCGTGCTGACACAGTCCCCAGCAATTATGTCTGCCAGTCCCG GGGAGAAGGTGACAATGACTTGTAGTGCCAGCTCCTCTGTCTCATA CATGCATTGGTATCAGCAGAAGTCCGGCACATCTCCTAAACGGTGG ATCTACGACACTTCTAAACTGAGTTCAGGCGTGCCCGGGAGATTTTC AGGCAGCGGGTCCGGAACTTCTTATAGTCTGACCATTTCCCGACTG GAGGCCGAAGATGCCGCTACCTACTATTGCCATCAGTGGTCTTCAA GCCCTCATACTTTTGGGGGGGGAACTAAGGTGGAAATCAAGCGA |
| 147 | hMC48 VH amino acid sequence | QVQLKESGPGLVAPSQSLSITCTVSGFSLTSYGVSWIRQPPGKGLEWIG VIWGEGSTNYHSVLISRLTISKDNSKSQVFLKLNSLQTDDTATYYCAMT GTAYWGQGTLVTVSS |
| 148 | hMC48 VL amino acid sequence | QIVLTQSPAIMSASPGEKVTMTCSASSSVSYMHWYQQKSGTSPKRWIY DTSKLSSGVPGRFSGSGSGTSYSLTISRLEAEDAATYYCHQWSSSPHTF GGGTKVEIKR |
| 149 | hMC48 VL CDR1 | SSVSY |
| 150 | hMC48 VL CDR2 | DTS |
| 151 | hMC48 VL CDR3 | HQWSSSPHT |
| 152 | hMC48 VH CDR1 | GFSLTSYG |
| 153 | hMC48 VH CDR2 | IWGEGST |
| 154 | hMC48 VH CDR3 | AMTGTAY |
| 155 | hMC48 VL FR1 | QIVLTQSPAIMSASPGEKVTMTCSAS |
| 156 | hMC48 VL FR2 | MHWYQQKSGTSPKRWIY |
| 157 | hMC48 VL FR3 | KLSSGVPGRFSGSGSGTSYSLTISRLEAEDAATYYC |
| 158 | hMC48 VL FR4 | FGGGTKVEIKR |
| 159 | hMC48 VH FR1 | QVQLKESGPGLVAPSQSLSITCTVS |
| 160 | hMC48 VH FR2 | VSWIRQPPGKGLEWIGV |
| 161 | hMC48 VH FR3 | NYHSVLISRLTISKDNSKSQVFLKLNSLQTDDTATYYC |
| 162 | hMC48 VH FR4 | WGQGTLVTVSS |

### Antibodies Specific To SSEA4 and Fragment Thereof (not part of the present invention)

One aspect of the present disclosure features the new antibodies that bind to SSEA-4 and fragments thereof. The anti-SSEA-4 antibody binds to Neu5Acα2→ 3Galβ1→ 3GalNAcβ1→ 3Galα1→ 4Galβ1→ 4Glcβ1→ (SSEA-4 hexasaccharide) and Neu5Acα2→ 3Galβ1→ 3GalNAcβ1→ 3Galα1(fragment of SSEA-4 hexasaccharide). In some examples, the antibody is capable of Neu5Acα2→ 3Galβ1→ 3GalNAcβ1→ 3Galβ1. In some examples, the antibody is capable of Neu5Gcα2→ 3Galβ1→ 3GalNAcβ1→ 3Galα1→ 4Galβ1→ 4Glcβ1(an analogue of SSEA-4 hexasaccharide). In one example, the antibody is mAb 46, which is not part of the present invention).

Monoclonal antibodyMC46 is produced by the hybridoma cell line. The anti-SSEA-4 antibody described herein can contain the same V_{H} and V_{L} chains as antibody MC46.

**Table 5. Amino Acid and Nucleotide Sequences of Antibody MC46 (not part of the present invention)**

| SEQ ID NO | DESCRIPTION | SEQUENCE |
|---|---|---|
| 31 | MC46 VH nucleotide sequence | CAGGTGCAGCTGAAGGAGTCAGGACCTGGCCTGGTGGCGCCCTCAC AGAGCCTGTCCATCACATGCACTGTCTCAGGATTCTCATTAACCAGC TATGGTATAAGCTGGGTTCGCCAGCCTCCAGGAAAGGGTCTGGAGT GGCTGGGAGTAATATGGGGTGACGGGAGCACAAATTATCATTCAGC TCTCGTATCCAGACTGAGCATCAGCAAGGATAACTCCAAGAGCCAA GTTTTCTTAAAACTGAACAGTCTGCAAACTGATGACACAGCCACGT ACTACTGTGCCAAAACTGGGACATCTTACTGGGGCCAAGGGACTCT GGTCACTGTCTCTGCA |
| 32 | MC46 VL nucleotide sequence | CAAATTGTTCTCACCCAGTCTCCAGCAATCATGTCTGCATCTCCAGG GGAGAAGGTCACCATGACCTGCAGTGCCAGCTCAAGTGTAAGTTAC ATGCACTGGTACCAGCAGAAGTCAGGCACCTCCCCCAAAAGATGGA TTTATGACACATCCAAACTGGCTTCTGGAGTCCCTGCTCGCTTCAGT GGCAGTGGGTCTGGGACCTCTTACTCTCTCACAATCAGCAGCATGG AGGCTGAAGATGCTGCCACTTATTACTGCCAGCAGTGGAGTAGTGC |
| | | CCCACACACGTTCGGAGGGGGACCAAGCTGGAAATAAAA |
| 33 | MC46 VH amino acid sequence | QVQLKESGPGLVAPSQSLSITCTVSGFSLTSYGISWVRQPPGKGLEWLG VIWGDGSTNYHSALVSRLSISKDNSKSQVFLKLNSLQTDDTATYYCAK TGTSYWGQGTLVTVSA |
| 34 | MC46 VL amino acid sequence | QIVLTQSPAIMSASPGEKVTMTCSASSSVSYMHWYQQKSGTSPKRWIY DTSKLASGVPARFSGSGSGTSYSLTISSMEAEDAATYYCQQWSSAPHTF GGGTKLEIK |
| 35 | MC46 VL CDR1 | SSVSY |
| 36 | MC46 VL CDR2 | DTS |
| 37 | MC46 VL CDR3 | QQWSSAPHT |
| 38 | MC46 VH CDR1 | GFSLTSYG |
| 39 | MC46 VH CDR2 | IWGDGST |
| 40 | MC46 VH CDR3 | AKTGTSY |

A "MC45 antibody" or "mAb 45" or "antibody from clone MC45" refers to an antibody expressed by clone 45 or to an antibody synthesized in other manners, but having the same CDRs and optionally, the same framework regions as the antibody expressed by clone MC45. Similarly, antibodies MC46 (mAb 46 or clone 46), MC48 (mAb 48 or clone 48), MC273 (mAb 273 or clone 273), MC651 (mAb 651 or clone 651) and the like refer to antibodies expressed by the corresponding clone(s) and/or to antibodies synthesized in other manners, but having the same CDRs and optionally, the same framework regions as the referenced antibodies.

**Table 6. Comparison of Binding Epitope and Isotype of Antibodies**

| Antibody | Isotype | Binding Epitope |
|---|---|---|
| Mbrl | Mouse IgM | Fucα1→ 2Gaβ1→ 3GalNAcβ1→ 3Galα1→ 4Galβ1→ 4Glcβ1 (**Globo H**) |
| | | Fucα1→ 2Galβ1→ 3GalNAcβ1→ 3Galα1 |
| VK9 | Mouse IgG3 | Fucα1→ 2Galβ1→ 3GalNAcβ1→ 3Galα1→ 4Galβ1→ 4Glcβ1 (**Globo H**) |
| | | Fucα1→ 2Galβ1→ 3GalNAcβ1→ 3Galα1 |
| MC-813-70 | Mouse IgG3 | Neu5Acα2→ 3Galβ1→ 3GalNAcβ1→ 3Galα→ 4Galβ1→ 4Glcβ1 (**SSEA-4**) |
| | | Neu5Gcα2→3Galβ1→3GalNAcβ1→3Galβ1→4Galβ1→ 4Glcβ1 |
| MC-651 | Mouse IgG1 | Fucα1→2Galβ1→3GalNAcβ1→3Galβ1→4Galβ1→4Glcβ1 **(Globo H**) |
| | | Galβ1→3GalNAcβ1→3Galβ1→4Galβ1→4Glcβ1 (**SSEA-3**) |
| | | Neu5Acα2→3Galβ1→3GalNAcβ1→3Galα→ 4Galβ1→4Glcβ1 (**SSEA-4**) |
| MC-273 | Mouse IgG1 | Fucα1→2Galβ1→3GalNAcβ1→3Galα1→4Galβ1→4Glcβ1 (**Globo H**) |
| | | Galβ1→3GalNAcβ1→3Galα1→4Galβ1→4Glcβ1 (**SSEA-3**) |
| MC-45 | Mouse IgG1 | Neu5Acαu2→ 3Galβ1→ 3GalNAcβ1→ 3Galα→ 4Galβ1→ 4Glcβ1 (**SSEA-4**) |
| | | Neu5Gcα2→ 3Galβ1→ 3GalNAcβ1→ 3Galα→ 4Galβ1→ 4Glcβ1 |
| MC-46 | Mouse IgG1 | Neu5Acα2→ 3Galβ1→ 3GalNAcβ1→ 3Galα→ 4Galβ1→ 4Glcβ1 (**SSEA-4**) |
| | | Neu5Gcα2→ 3Galβ1→ 3GalNAcβ1→ 3Galα→ 4Galβ1→ 4Glcβ1 |
| | | Neu5Acα2→ 3Galβ1→ 3GalNAcβ1→ 3Galα1 |
| | | Neu5Acα2→ 3Galβ1→ 3GalNAcβ1→ 3Galβ1 |
| MC-48 | Mouse IgG1 | Neu5Acα2→ 3Galβ1→ 3GalNAcβ1→ 3Galα→ 4Galβ1→ 4Glcβ1 (**SSEA-4**) |
| | | Neu5Gcα2→ 3Galβ1→ 3GalNAcβ1→ 3Galα→ 4Galβ1→ 4Glcβ1 |

Any of the antibodies described herein can be a full length antibody or an antigen-binding fragment thereof. In some examples, the antigen binding fragment is a Fab fragment, a F(ab')2 fragment, or a single-chain Fv fragment. In some examples, the antigen binding fragment is a Fab fragment, a F(ab')2 fragment, or a single-chain Fv fragment. In some examples, the isolated antibody is a human antibody, a humanized antibody, a chimeric antibody, or a single-chain antibody.

Any of the antibodies described herein has one or more characteristics of:
a) is a recombinant antibody, a monoclonal antibody, a chimeric antibody, a humanized antibody, a human antibody, an antibody fragment, a bispecific antibody, a monospecific antibody, a monovalent antibody, an IgG1 antibody, an IgG2 antibody, or derivative of an antibody; b) is a human, murine, humanized, or chimeric antibody, antigen-binding fragment, or derivative of an antibody; c) is a single-chain antibody fragment, a multibody, a Fab fragment, and/or an immunoglobulin of the IgG, IgM, IgA, IgE, IgD isotypes and/or subclasses thereof; d) has one or more of the following characteristics: (i) mediates ADCC and/or CDC of cancer cells; (ii) induces and/or promotes apoptosis of cancer cells; (iii) inhibits proliferation of target cells of cancer cells; (iv) induces and/or promotes phagocytosis of cancer cells; and/or (v) induces and/or promotes the release of cytotoxic agents; e) specifically binds the tumor-associated carbohydrate antigen, which is a tumor-specific carbohydrate antigen; f) does not bind an antigen expressed on non-cancer cells, non-tumor cells, benign cancer cells and/or benign tumor cells; and/or g) specifically binds a tumor-associated carbohydrate antigen expressed on cancer stem cells and on normal cancer cells.

Preferably the binding of the antibodies to their respective antigens is specific. The term "specific" is generally used to refer to the situation in which one member of a binding pair will not show any significant binding to molecules other than its specific binding partner (s) and e.g. has less than about 30%, preferably 20%, 10%, or 1 % cross-reactivity with any other molecule other than those specified herein.

The antibodies are suitable bind to its target epitopes with a high affinity (low KD value), and preferably KD is in the nanomolar range or lower. Affinity can be measured by methods known in the art, such as, for example; surface plasmon resonance.

### Exemplary Antibody Preparation

Exemplary Antibodies capable of binding to the SSEA-4 epitopes described herein can be made by any method known in the art. See, for example, Harlow and Lane, (1988) Antibodies: A Laboratory Manual, Cold Spring Harbor Laboratory, New York.

### Immunization of Host Animals and Hybridoma Technology

Exemplary Polyclonal antibodies against the anti-SSEA-4 antibodies may be prepared by collecting blood from the immunized mammal examined for the increase of desired antibodies in the serum, and by separating serum from the blood by any conventional method. Polyclonal antibodies include serum containing the polyclonal antibodies, as well as the fraction containing the polyclonal antibodies may be isolated from the serum.

Polyclonal antibodies are generally raised in host animals (e.g., rabbit, mouse, horse, or goat) by multiple subcutaneous (sc) or intraperitoneal (ip) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, etc.

Any mammalian animal may be immunized with the antigen for producing the desired antibodies. In general, animals of Rodentia, Lagomorpha, or Primates can be used. Animals of Rodentia include, for example, mouse, rat, and hamster. Animals of Lagomorpha include, for example, rabbit. Animals of Primates include, for example, a monkey of Catarrhini (old world monkey) such as Macaca fascicularis, rhesus monkey, baboon, and chimpanzees.

Methods for immunizing animals with antigens are known in the art. Intraperitoneal injection or subcutaneous injection of antigens is a standard method for immunization of mammals. More specifically, antigens may be diluted and suspended in an appropriate amount of phosphate buffered saline (PBS), physiological saline, etc. If desired, the antigen suspension may be mixed with an appropriate amount of a standard adjuvant, such as Freund's complete adjuvant, made into emulsion, and then administered to mammalian animals. Animals are immunized against the antigen, immunogenic conjugates, or derivatives by combining 1 mg or 1 µg of the peptide or conjugate (for rabbits or mice, respectively) with 3 volumes of Freund's incomplete adjuvant.

Animals can be boosted until the titer plateaus by several administrations of antigen mixed with an appropriately amount of Freund's incomplete adjuvant every 4 to 21 days. Animals are boosted with 1/5 to 1/10 the original amount of peptide or conjugate in Freund's complete adjuvant by subcutaneous injection at multiple sites. Seven to 14 days later the animals are bled and the serum is assayed for antibody titer. An appropriate carrier may also be used for immunization. After immunization as above, serum is examined by a standard method for an increase in the amount of desired antibodies. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

Over the past two to three decades, a number of methodologies have been developed to prepare chimeric, humanized or human antibodies for human in-vivo therapeutic applications. The most used and proven methodology is to prepare mouse mAbs using hybridoma methodology and then to humanize the mAbs by converting the framework regions of the V_{H} and V_{L} domains and constant domains of the mAbs into most homologous human framework regions of human V_{H} and V_{L} domains and constant regions of a desirable human γ immunoglobulin isotype and subclass. Many mAbs, such as Xolair, used clinically are humanized mAbs of human γ1, κ isotype and subclass and prepared using this methodology.

In some embodiments, antibodies can be made by the conventional hybridoma technology. Kohler et al., Nature, 256:495 (1975). In the hybridoma method, a mouse or other appropriate host animal, such as a hamster or rabbit, is immunized as hereinabove described to elicit lymphocytes that produce or are capable of producing antibodies that will specifically bind to the protein used for immunization. Alternatively, lymphocytes may be immunized in vitro.

To prepare monoclonal antibodies, immune cells are collected from the mammal immunized with the antigen and checked for the increased level of desired antibodies in the serum as described above, and are subjected to cell fusion. The immune cells used for cell fusion are preferably obtained from spleen. Other preferred parental cells to be fused with the above immunocyte include, for example, myeloma cells of mammalians, and more preferably myeloma cells having an acquired property for the selection of fused cells by drugs.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, Calif. USA, and SP-2 cells available from the American Type Culture Collection, Rockville, Md. USA. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, pp. 51-63 (Marcel Dekker, Inc., New York, 1987)).

The above immunocyte and myeloma cells can be fused according to known methods, for example, the method of Milstein et al. (Galfre et al., Methods Enzymol. 73:3-46, 1981). Lymphocytes are fused with myeloma cells using a suitable fusing agent, such as polyethylene glycol, to form a hybridoma cell (Goding, Monoclonal Antibodies: Principles and Practice, pp.59-103 (Academic Press, 1986)). Resulting hybridomas obtained by the cell fusion may be selected by cultivating them in a standard selection medium, such as HAT medium (hypoxanthine, aminopterin, and thymidine containing medium). The cell culture is typically continued in the HAT medium for several days to several weeks, the time being sufficient to allow all the other cells, with the exception of the desired hybridoma (non-fused cells), to die. Then, the standard limiting dilution is performed to screen and clone a hybridoma cell producing the desired antibody.

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay. Measurement of absorbance in enzyme-linked immunosorbent assay (ELISA), enzyme immunoassay (EIA), radioimmunoassay (RIA), and/or immunofluorescence may be used to measure the antigen binding activity of the antibody of the invention. In ELISA, the antibody of the present invention is immobilized on a plate, protein of the invention is applied to the plate, and then a sample containing a desired antibody, such as culture supernatant of antibody producing cells or purified antibodies, is applied. Then, a secondary antibody that recognizes the primary antibody and is labeled with an enzyme, such as alkaline phosphatase, is applied, and the plate is incubated. Next, after washing, an enzyme substrate, such as p-nitrophenyl phosphate, is added to the plate, and the absorbance is measured to evaluate the antigen binding activity of the sample. A fragment of the protein, such as a C-terminal or N-terminal fragment may be used in this method. BIAcore (Pharmacia) may be used to evaluate the activity of the antibody according to the present invention. The binding affinity of the monoclonal antibody can, for example, be determined by the Scatchard analysis of Munson et al., Anal. Biochem., 107:220 (1980).

Applying any of the conventional methods, including those described above, hybridoma cells producing antibodies that bind to epitopes described herein can be identified and selected for further characterization.

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

In addition, the hybridoma cells may be grown in vivo as ascites tumors in an animal. For example, the obtained hybridomas can be subsequently transplanted into the abdominal cavity of a mouse and the ascites are harvested.

The obtained monoclonal antibodies can be purified by, for example, ammonium sulfate precipitation, a protein A or protein G column, DEAE ion exchange chromatography, or an affinity column to which the protein of the present invention is coupled. The antibody of the present invention can be used not only for purification and detection of the protein of the present invention, but also as a candidate for agonists and antagonists of the protein of the present invention. In addition, this antibody can be applied to the antibody treatment for diseases related to the protein of the present invention.

### Recombinant Technology

The monoclonal antibodies thus obtained can be also recombinantly prepared using genetic engineering techniques (see, for example, Borrebaeck C. A. K. and Larrick J. W. Therapeutic Monoclonal Antibodies, published in the United Kingdom by MacMillan Publishers LTD, 1990). A DNA encoding an antibody may be cloned from an immune cell, such as a hybridoma or an immunized lymphocyte producing the antibody, inserted into an appropriate vector, and introduced into host cells to prepare a recombinant antibody. The present invention also provides recombinant antibodies prepared as described above.

When the obtained antibody is to be administered to the human body (antibody treatment), a human antibody or a humanized antibody is preferable for reducing immunogenicity. For example, transgenic animals having a repertory of human antibody genes may be immunized with an antigen selected from a protein, protein expressing cells, or their lysates. Antibody producing cells are then collected from the animals and fused with myeloma cells to obtain hybridoma, from which human antibodies against the protein can be prepared. Alternatively, an immune cell, such as an immunized lymphocyte, producing antibodies may be immortalized by an oncogene and used for preparing monoclonal antibodies.

DNA encoding the monoclonal antibodies can be readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Pluckthun, Immunol. Rev., 130:151-188 (1992).

DNAs encoding the antibodies produced by the hybridoma cells described above can be genetically modified, via routine technology, to produce genetically engineered antibodies. Genetically engineered antibodies, such as humanized antibodies, chimeric antibodies, single-chain antibodies, and bi-specific antibodies, can be produced via, e.g., conventional recombinant technology. The DNA can then be modified, for example, by substituting the coding sequence for human heavy and light chain constant domains in place of the homologous murine sequences, Morrison et al., (1984) Proc. Nat. Acad. Sci. 81:6851, or by covalently joining to the immunoglobulin coding sequence all or part of the coding sequence for a non-immunoglobulin polypeptide. In that manner, genetically engineered antibodies, such as "chimeric" or "hybrid" antibodies; can be prepared that have the binding specificity of a target antigen.

Techniques developed for the production of "chimeric antibodies" are well known in the art. See, e.g., Morrison et al. (1984) Proc. Natl. Acad. Sci. USA 81, 6851; Neuberger et al. (1984) Nature 312, 604; and Takeda et al. (1984) Nature 314:452.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

Chimeric or hybrid antibodies also may be prepared in vitro using known methods in synthetic protein chemistry, including those involving crosslinking agents. For example, immunotoxins may be constructed using a disulfide-exchange reaction or by forming a thioether bond. Examples of suitable reagents for this purpose include iminothiolate and methyl-4-mercaptobutyrimidate.

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. Accordingly, such "humanized" antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987)). Another method uses a particular framework derived from the consensus sequence of all human antibodies of a particular subgroup of light or heavy chains. The same framework may be used for several different humanized antibodies (Carter et al., Proc. Natl. Acad Sci. USA, 89:4285 (1992); Prestaetal., J. Immnol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i. e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the CDR residues are directly and most substantially involved in influencing antigen binding.

Alternatively, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci. USA, 90:2551 (1993); Jakobovits et al., Nature, 362:255-258 (1993); Bruggermann et al., Year in Immuno., 7:33 (1993). Human antibodies can also be derived from phage-display libraries (Hoogenboom et al., J. Mol. Biol., 227:381 (1991); Marks et al., J. Mol. Biol., 222:581-597 (1991)).

In some examples, a vector comprising a nucleic acid comprising a nucleotide sequence encoding either the heavy chain variable region or the light chain variable region of an anti-SSEA-4 antibody as described herein. In other examples, the vector comprises nucleotide sequences encoding both the heavy chain variable region and the light chain variable region, the expression of which can be controlled by a single promoter or two separate promoters. Also described here are methods for producing anti-SSEA-4 antibodies as described herein, e.g., via the recombinant technology described in this section.

### Other Technology for Preparing Antibodies

Fully human antibodies can be obtained by using commercially available mice that have been engineered to express specific human immunoglobulin proteins. Transgenic animals that are designed to produce a more desirable (e.g., fully human antibodies) or more robust immune response may also be used for generation of humanized or human antibodies. Examples of such technology are Xenomouse^{RTM} from Amgen, Inc. (Fremont, Calif.) and HuMAb-Mouse^{RTM} and TC Mouse™ from Medarex, Inc. (Princeton, N.J.). In another alternative, antibodies may be made recombinantly by phage display technology. See, for example, U.S. Pat. Nos. 5,565,332; 5,580,717; 5,733,743; and 6,265,150; and Winter et al., (1994) Annu. Rev. Immunol. 12:433-455. Alternatively, the phage display technology (McCafferty et al., (1990) Nature 348:552-553) can be used to produce human antibodies and antibody fragments in vitro, from immunoglobulin variable (V) domain gene repertoires from unimmunized donors.

Antigen-binding fragments of an intact antibody (full-length antibody) can be prepared via routine methods. For example, F(ab')2 fragments can be produced by pepsin digestion of an antibody molecule, and Fab fragments that can be generated by reducing the disulfide bridges of F(ab')2 fragments.

Alternatively, the anti-SSEA-4 antibodies described herein can be isolated from antibody phage libraries (e.g., single-chain antibody phage libraries) generated using the techniques described in McCafferty et al., Nature, 348:552-554 (1990). Clackson et al., Nature, 352:624-628 (1991) and Marks et al., J. Mol Biol., 222:581-597 (1991). Subsequent publications describe the production of high affinity (nM range) human antibodies by chain shuffling (Marks et al., Bio/Technology, 10:779-783 (1992)), as well as combinatorial infection and in vivo recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

Antibodies obtained as described herein may be purified to homogeneity. For example, the separation and purification of the antibody can be performed according to separation and purification methods used for general proteins. For example, the antibody may be separated and isolated by the appropriately selected and combined use of column chromatographies, such as affinity chromatography, filter, ultrafiltration, salting-out, dialysis, SDS polyacrylamide gel electrophoresis, isoelectric focusing, and others (Antibodies: A Laboratory Manual. Ed Harlow and David Lane, Cold Spring Harbor Laboratory, 1988), but are not limited thereto. The concentration of the antibodies obtained as above may be determined by the measurement of absorbance, Enzyme-linked immunosorbent assay (ELISA), or so on. Exemplary chromatography, with the exception of affinity includes, for example, ion-exchange chromatography, hydrophobic chromatography, gel filtration, reverse-phase chromatography, adsorption chromatography, and the like (Strategies for Protein Purification and Characterization: A Laboratory Course Manual. Ed Daniel R. Marshak et al., Cold Spring Harbor Laboratory Press, 1996). The chromatographic procedures can be carried out by liquid-phase chromatography, such as HPLC, FPLC.

The antibodies can be characterized using methods well known in the art. For example, one method is to identify the epitope to which the antigen binds, or "epitope mapping." There are many methods known in the art for mapping and characterizing the location of epitopes on proteins, including solving the crystal structure of an antibody-antigen complex, competition assays, gene fragment expression assays, and synthetic peptide-based assays, as described, for example, in Chapter 11 of Harlow and Lane, Using Antibodies, a Laboratory Manual, Cold Spring Harbor Laboratory Press, Cold Spring Harbor, N.Y., 1999. In an additional example, epitope mapping can be used to determine the sequence to which an antibody binds. The epitope can be a linear epitope, i.e., contained in a single stretch of amino acids, or a conformational epitope formed by a three-dimensional interaction of amino acids that may not necessarily be contained in a single stretch (primary structure linear sequence). Peptides of varying lengths (e.g., at least 4-6 amino acids long) can be isolated or synthesized (e.g., recombinantly) and used for binding assays with an antibody. In another example, the epitope to which the antibody binds can be determined in a systematic screening by using overlapping peptides derived from the target antigen sequence and determining binding by the antibody. According to the gene fragment expression assays, the open reading frame encoding the target antigen is fragmented either randomly or by specific genetic constructions and the reactivity of the expressed fragments of the antigen with the antibody to be tested is determined. The gene fragments may, for example, be produced by PCR and then transcribed and translated into protein in vitro, in the presence of radioactive amino acids. The binding of the antibody to the radioactively labeled antigen fragments is then determined by immunoprecipitation and gel electrophoresis. Certain epitopes can also be identified by using large libraries of random peptide sequences displayed on the surface of phage particles (phage libraries). Alternatively, a defined library of overlapping peptide fragments can be tested for binding to the test antibody in simple binding assays.

In an additional example, mutagenesis of an antigen binding domain, domain swapping experiments and alanine scanning mutagenesis can be performed to identify residues required, sufficient, and/or necessary for epitope binding. For example, domain swapping experiments can be performed using a mutant of a target antigen in which various residues in the binding epitope for the candidate antibody have been replaced (swapped) with sequences from a closely related, but antigenically distinct protein (such as another member of the neurotrophin protein family). By assessing binding of the antibody to the mutant target protein, the importance of the particular antigen fragment to antibody binding can be assessed.

Alternatively, competition assays can be performed using other antibodies known to bind to the same antigen to determine whether an antibody binds to the same epitope (e.g., the MC45 antibody described herein) as the other antibodies. Competition assays are well known to those of skill in the art.

### Additional Aspects of Exemplary suitable General Antibody Production Methods

Methods of making monoclonal and polyclonal antibodies and fragments thereof in animals (e.g., mouse, rabbit, goat, sheep, or horse) are well known in the art. See, for example, Harlow and Lane, (1988) Antibodies: A Laboratory Manual, Cold Spring Harbor Laboratory, New York. The term "antibody" includes intact immunoglobulin molecules as well as fragments thereof, such as Fab, F(ab')2, Fv, scFv (single chain antibody), and dAb (domain antibody; Ward, et. al. (1989) Nature, 341, 544).

The compositions disclosed herein can be included in a pharmaceutical composition together with additional active agents, carriers, vehicles, excipients, or auxiliary agents identifiable by a person skilled in the art upon reading of the present disclosure.

The pharmaceutical compositions preferably comprise at least one pharmaceutically acceptable carrier. In such pharmaceutical compositions, the compositions disclosed herein form the "active compound," also referred to as the "active agent." As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions. A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol, or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates, or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes, or multiple dose vials made of glass or plastic.

Compositions comprising an anti-SSEA-4 antibody or at least one polynucleotide comprising sequences encoding an anti-SSEA-4 antibody are provided. In certain embodiments, a composition may be a pharmaceutical composition. These compositions may further comprise suitable carriers, such as pharmaceutically acceptable excipients including buffers, which are well known in the art.

Isolated antibodies and polynucleotides are also provided. In certain embodiments, the isolated antibodies and polynucleotides are substantially pure.

The anti-SSEA-4/ antibodies are monoclonal. In another embodiment, fragments of the anti-SSEA-4 antibodies (e.g., Fab, Fab'-SH and F(ab')2 fragments) are provided. These antibody fragments can be created by traditional means, such as enzymatic digestion, or may be generated by recombinant techniques. Such antibody fragments may be chimeric, humanized, or human. These fragments are useful for the diagnostic and therapeutic purposes set forth below.

A variety of methods are known in the art for generating phage display libraries from which an antibody of interest can be obtained. One method of generating antibodies of interest is through the use of a phage antibody library as described in Lee et al., J. Mol. Biol. (2004), 340(5): 1073-93.

The anti-SSEA-4 antibodies of the invention can be made by using combinatorial libraries to screen for synthetic antibody clones with the desired activity or activities. In principle, synthetic antibody clones are selected by screening phage libraries containing phage that display various fragments of antibody variable region (Fv) fused to phage coat protein. Such phage libraries are panned by affinity chromatography against the desired antigen. Clones expressing Fv fragments capable of binding to the desired antigen are adsorbed to the antigen and thus separated from the non-binding clones in the library. The binding clones are then eluted from the antigen, and can be further enriched by additional cycles of antigen adsorption/elution. Any of the anti-SSEA-4 antibodies of the invention can be obtained by designing a suitable antigen screening procedure to select for the phage clone of interest followed by construction of a full length anti-SSEA-4 antibody clone using the Fv sequences from the phage clone of interest and suitable constant region (Fc) sequences described in Kabat et al., Sequences of Proteins of Immunological Interest, Fifth Edition, NIH Publication 91-3242, Bethesda Md. (1991), vols. 1-3.

The antigen-binding domain of an antibody is formed from two variable (V) regions of about 110 amino acids, one each from the light (VL) and heavy (VH) chains, that both present three hypervariable loops or complementarity-determining regions (CDRs). Variable domains can be displayed functionally on phage, either as single-chain Fv (scFv) fragments, in which VH and VL are covalently linked through a short, flexible peptide, or as Fab fragments, in which they are each fused to a constant domain and interact non-covalently, as described in Winter et al., Ann. Rev. Immunol., 12: 433-455 (1994). As used herein, scFv encoding phage clones and Fab encoding phage clones are collectively referred to as "Fv phage clones" or "Fv clones".

Repertoires of VH and VL genes can be separately cloned by polymerase chain reaction (PCR) and recombined randomly in phage libraries, which can then be searched for antigen-binding clones as described in Winter et al., Ann. Rev. Immunol., 12: 433-455 (1994). Libraries from immunized sources provide high-affinity antibodies to the immunogen without the requirement of constructing hybridomas. Alternatively, the naive repertoire can be cloned to provide a single source of human antibodies to a wide range of non-self and also self antigens without any immunization as described by Griffiths et al., EMBO J, 12: 725-734 (1993). Finally, naive libraries can also be made synthetically by cloning the unrearranged V-gene segments from stem cells, and using PCR primers containing random sequence to encode the highly variable CDR3 regions and to accomplish rearrangement in vitro as described by Hoogenboom and Winter, J. Mol. Biol., 227: 381-388 (1992).

Filamentous phage is used to display antibody fragments by fusion to the minor coat protein pIII. The antibody fragments can be displayed as single chain Fv fragments, in which VH and VL domains are connected on the same polypeptide chain by a flexible polypeptide spacer, e.g. as described by Marks et al., J. Mol. Biol., 222: 581-597 (1991), or as Fab fragments, in which one chain is fused to pIII and the other is secreted into the bacterial host cell periplasm where assembly of a Fab-coat protein structure which becomes displayed on the phage surface by displacing some of the wild type coat proteins, e.g. as described in Hoogenboom et al., Nucl. Acids Res., 19: 4133-4137 (1991).

In general, nucleic acids encoding antibody gene fragments are obtained from immune cells harvested from humans or animals. If a library biased in favor of anti-SSEA-4 clones is desired, the subject is immunized with SSEA-4 to generate an antibody response, and spleen cells and/or circulating B cells or other peripheral blood lymphocytes (PBLs) are recovered for library construction. In one example, a human antibody gene fragment library biased in favor of anti-human SSEA-4 clones is obtained by generating an anti-human SSEA-4 antibody response in transgenic mice carrying a functional human immunoglobulin gene array (and lacking a functional endogenous antibody production system) such that SSEA-4 immunization gives rise to B cells producing human antibodies against SSEA-4. The generation of human antibody-producing transgenic mice is described below.

Additional enrichment for anti-SSEA-4 reactive cell populations can be obtained by using a suitable screening procedure to isolate B cells expressing SSEA-4-specific antibody, e.g., by cell separation with SSEA-4 affinity chromatography or adsorption of cells to fluorochrome-labeled SSEA-4 followed by flow-activated cell sorting (FACS).

Alternatively, the use of spleen cells and/or B cells or other PBLs from an unimmunized donor provides a better representation of the possible antibody repertoire, and also permits the construction of an antibody library using any animal (human or non-human) species in which SSEA-4 is not antigenic. For libraries incorporating in vitro antibody gene construction, stem cells are harvested from the subject to provide nucleic acids encoding unrearranged antibody gene segments. The immune cells of interest can be obtained from a variety of animal species, such as human, mouse, rat, lagomorpha, luprine, canine, feline, porcine, bovine, equine, and avian species, etc.

Nucleic acid encoding antibody variable gene segments (including VH and VL segments) are recovered from the cells of interest and amplified. In the case of rearranged VH and VL gene libraries, the desired DNA can be obtained by isolating genomic DNA or mRNA from lymphocytes followed by polymerase chain reaction (PCR) with primers matching the 5' and 3' ends of rearranged VH and VL genes as described in Orlandi et al., Proc. Natl. Acad. Sci. (USA), 86: 3833-3837 (1989), thereby making diverse V gene repertoires for expression. The V genes can be amplified from cDNA and genomic DNA, with back primers at the 5' end of the exon encoding the mature V-domain and forward primers based within the J-segment as described in Orlandi et al. (1989) and in Ward et al., Nature, 341: 544-546 (1989). However, for amplifying from cDNA, back primers can also be based in the leader exon as described in Jones et al., Biotechnol., 9: 88-89 (1991), and forward primers within the constant region as described in Sastry et al., Proc. Natl. Acad. Sci. (USA), 86: 5728-5732 (1989). To maximize complementarity, degeneracy can be incorporated in the primers as described in Orlandi et al. (1989) or Sastry et al. (1989). In certain examples, the library diversity is maximized by using PCR primers targeted to each V-gene family in order to amplify all available VH and VL arrangements present in the immune cell nucleic acid sample, e.g. as described in the method of Marks et al., J. Mol. Biol., 222: 581-597 (1991) or as described in the method of Orum et al., Nucleic Acids Res., 21: 4491-4498 (1993). For cloning of the amplified DNA into expression vectors, rare restriction sites can be introduced within the PCR primer as a tag at one end as described in Orlandi et al. (1989), or by further PCR amplification with a tagged primer as described in Clackson et al., Nature, 352: 624-628 (1991).

Repertoires of synthetically rearranged V genes can be derived in vitro from V gene segments. Most of the human VH-gene segments have been cloned and sequenced (reported in Tomlinson et al., J. Mol. Biol., 227: 776-798 (1992)), and mapped (reported in Matsuda et al., Nature Genet., 3: 88-94 (1993); these cloned segments (including all the major conformations of the H1 and H2 loop) can be used to generate diverse VH gene repertoires with PCR primers encoding H3 loops of diverse sequence and length as described in Hoogenboom and Winter, J. Mol. Biol., 227: 381-388 (1992). VH repertoires can also be made with all the sequence diversity focused in a long H3 loop of a single length as described in Barbas et al., Proc. Natl. Acad. Sci. USA, 89: 4457-4461 (1992). Human V κ and Vλ segments have been cloned and sequenced (reported in Williams and Winter, Eur. J. Immunol., 23: 1456-1461 (1993)) and can be used to make synthetic light chain repertoires. Synthetic V gene repertoires, based on a range of VH and VL folds, and L3 and H3 lengths, will encode antibodies of considerable structural diversity. Following amplification of V-gene encoding DNAs, germline V-gene segments can be rearranged in vitro according to the methods of Hoogenboom and Winter, J. Mol. Biol., 227: 381-388 (1992).

Repertoires of antibody fragments can be constructed by combining VH and VL gene repertoires together in several ways. Each repertoire can be created in different vectors, and the vectors recombined in vitro, e.g., as described in Hogrefe et al., Gene, 128: 119-126 (1993), or in vivo by combinatorial infection, e.g., the loxP system described in Waterhouse et al., Nucl. Acids Res., 21: 2265-2266 (1993). The in vivo recombination approach exploits the two-chain nature of Fab fragments to overcome the limit on library size imposed by E. coli transformation efficiency. Naive VH and VL repertoires are cloned separately, one into a phagemid and the other into a phage vector. The two libraries are then combined by phage infection of phagemid-containing bacteria so that each cell contains a different combination and the library size is limited only by the number of cells present (about 1012 clones). Both vectors contain in vivo recombination signals so that the VH and VL genes are recombined onto a single replicon and are co-packaged into phage virions. These huge libraries provide large numbers of diverse antibodies of good affinity (Kd-1 of about 10-8 M).

Alternatively, the repertoires may be cloned sequentially into the same vector, e.g. as described in Barbas et al., Proc. Natl. Acad. Sci. USA, 88: 7978-7982 (1991), or assembled together by PCR and then cloned, e.g. as described in Clackson et al., Nature, 352: 624-628 (1991). PCR assembly can also be used to join VH and VL DNAs with DNA encoding a flexible peptide spacer to form single chain Fv (scFv) repertoires. In yet another technique, "in cell PCR assembly" is used to combine VH and VL genes within lymphocytes by PCR and then clone repertoires of linked genes as described in Embleton et al., Nucl. Acids Res., 20: 3831-3837 (1992).

Screening of the libraries can be accomplished by any art-known technique. For example, SSEA-4 targets can be used to coat the wells of adsorption plates, expressed on host cells affixed to adsorption plates or used in cell sorting, or conjugated to biotin for capture with streptavidin-coated beads, or used in any other art-known method for panning phage display libraries.

The phage library samples are contacted with immobilized SSEA-4 under conditions suitable for binding of at least a portion of the phage particles with the adsorbent. Normally, the conditions, including pH, ionic strength, temperature and the like are selected to mimic physiological conditions. The phages bound to the solid phase are washed and then eluted by acid, e.g. as described in Barbas et al., Proc. Natl. Acad. Sci. USA, 88: 7978-7982 (1991), or by alkali, e.g. as described in Marks et al., J. Mol. Biol., 222: 581-597 (1991), or by SSEA-4 antigen competition, e.g. in a procedure similar to the antigen competition method of Clackson et al., Nature, 352: 624-628 (1991). Phages can be enriched from about 20x to about 1,000-fold in a single round of selection. Moreover, the enriched phages can be grown in bacterial culture and subjected to further rounds of selection.

The efficiency of selection depends on many factors, including the kinetics of dissociation during washing, and whether multiple antibody fragments on a single phage can simultaneously engage with antigen. Antibodies with fast dissociation kinetics (and weak binding affinities) can be retained by use of short washes, multivalent phage display and high coating density of antigen in solid phase. The high density not only stabilizes the phage through multivalent interactions, but favors rebinding of phage that has dissociated. The selection of antibodies with slow dissociation kinetics (and good binding affinities) can be promoted by use of long washes and monovalent phage display as described in Bass et al., Proteins, 8: 309-314 (1990) and in WO 92/09690, and a low coating density of antigen as described in Marks et al., Biotechnol., 10: 779-783 (1992).

It is possible to select between phage antibodies of different affinities, even with affinities that differ slightly, for SSEA-4. However, random mutation of a selected antibody (e.g. as performed in some of the affinity maturation techniques described above) is likely to give rise to many mutants, most binding to antigen, and a few with higher affinity. With limiting SSEA-4, rare high affinity phage could be competed out. To retain all the higher affinity mutants, phages can be incubated with excess biotinylated SSEA-4, but with the biotinylated SSEA-4 at a concentration of lower molarity than the target molar affinity constant for SSEA-4. The high affinity-binding phages can then be captured by streptavidin-coated paramagnetic beads. Such "equilibrium capture" allows the antibodies to be selected according to their affinities of binding, with sensitivity that permits isolation of mutant clones with as little as two-fold higher affinity from a great excess of phages with lower affinity. Conditions used in washing phages bound to a solid phase can also be manipulated to discriminate on the basis of dissociation kinetics.

DNA encoding the Fv clones of the invention is readily isolated and sequenced using conventional procedures (e.g. by using oligonucleotide primers designed to specifically amplify the heavy and light chain coding regions of interest from hybridoma or phage DNA template). Once isolated, the DNA can be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of the desired monoclonal antibodies in the recombinant host cells. Review articles on recombinant expression in bacteria of antibody-encoding DNA include Skerra et al., Curr. Opinion in Immunol., 5: 256 (1993) and Pluckthun, Immunol. Revs, 130: 151 (1992).

DNA encoding the Fv clones of the invention can be combined with known DNA sequences encoding heavy chain and/or light chain constant regions (e.g. the appropriate DNA sequences can be obtained from Kabat et al., supra) to form clones encoding full or partial length heavy and/or light chains. It will be appreciated that constant regions of any isotype can be used for this purpose, including IgG, IgM, IgA, IgD, and IgE constant regions, and that such constant regions can be obtained from any human or animal species. A Fv clone derived from the variable domain DNA of one animal (such as human) species and then fused to constant region DNA of another animal species to form coding sequence(s) for "hybrid", full length heavy chain and/or light chain is included in the definition of "chimeric" and "hybrid" antibody as used herein. In one embodiment, a Fv clone derived from human variable DNA is fused to human constant region DNA to form coding sequence(s) for all human, full or partial length heavy and/or light chains.

The antibodies produced by naive libraries (either natural or synthetic) can be of moderate affinity (Kd -1 of about 106 to 107 M-1), but affinity maturation can also be mimicked in vitro by constructing and reselecting from secondary libraries as described in Winter et al. (1994), supra. For example, mutation can be introduced at random in vitro by using error-prone polymerase (reported in Leung et al., Technique, 1: 11-15 (1989)) in the method of Hawkins et al., J. Mol. Biol., 226: 889-896 (1992) or in the method of Gram et al., Proc. Natl. Acad. Sci. USA, 89: 3576-3580 (1992). Additionally, affinity maturation can be performed by randomly mutating one or more CDRs, e.g. using PCR with primers carrying random sequence spanning the CDR of interest, in selected individual Fv clones and screening for higher affinity clones. WO 9607754 (published 14 Mar. 1996) described a method for inducing mutagenesis in a complementarity determining region of an immunoglobulin light chain to create a library of light chain genes. Another effective approach is to recombine the VH or VL domains selected by phage display with repertoires of naturally occurring V domain variants obtained from unimmunized donors and screen for higher affinity in several rounds of chain reshuffling as described in Marks et al., Biotechnol., 10: 779-783 (1992). This technique allows the production of antibodies and antibody fragments with affinities in the 10-9 M range.

### Other Methods of Generating Anti-SSEA-4 Antibodies

Other methods of generating and assessing the affinity of antibodies are well known in the art and are described, e.g., in Kohler et al., Nature 256: 495 (1975); U.S. Pat. No. 4,816,567; Goding, Monoclonal Antibodies: Principles and Practice, pp. 59-103 (Academic Press, 1986; Kozbor, J. Immunol., 133:3001 (1984); Brodeur et al., Monoclonal Antibody Production Techniques and Applications, pp. 51-63 (Marcel Dekker, Inc., New York, 1987; Munson et al., Anal. Biochem., 107:220 (1980); Engels et al., Agnew. Chem. Int. Ed. Engl., 28: 716-734 (1989); Abrahmsen et al., EMBO J., 4: 3901 (1985); Methods in Enzymology, vol. 44 (1976); Morrison et al., Proc. Natl. Acad. Sci. USA, 81: 6851-6855 (1984).

### General Methods

Generation of antibodies can be achieved using routine skills in the art, including those described herein, such as the hybridoma technique and screening of phage displayed libraries of binder molecules. These methods are well-established in the art.

Briefly, the anti-SSEA-4 antibodies of the invention can be made by using combinatorial libraries to screen for synthetic antibody clones with the desired activity or activities. In principle, synthetic antibody clones are selected by screening phage libraries containing phage that display various fragments of antibody variable region (Fv) fused to phage coat protein. Such phage libraries are panned by affinity chromatography against the desired antigen. Clones expressing Fv fragments capable of binding to the desired antigen are adsorbed to the antigen and thus separated from the non-binding clones in the library. The binding clones are then eluted from the antigen, and can be further enriched by additional cycles of antigen adsorption/elution. Any of the anti-SSEA-4 antibodies of the invention can be obtained by designing a suitable antigen screening procedure to select for the phage clone of interest followed by construction of a full length anti-SSEA-4 antibody clone using the Fv sequences from the phage clone of interest and suitable constant region (Fc) sequences described in Kabat et al., Sequences of Proteins of Immunological Interest, Fifth Edition, NIH Publication 91-3242, Bethesda Md. (1991), vols. 1-3.

Anti-SSEA-4 antibodies of the invention are monoclonal. Also encompassed within the scope of the invention are antibody fragments such as Fab, Fab', Fab'-SH and F(ab')2 fragments, and variations thereof, of the anti- SSEA-4 antibodies provided herein. These antibody fragments can be created by traditional means, such as enzymatic digestion, or may be generated by recombinant techniques. Such antibody fragments may be chimeric, human or humanized. These fragments are useful for the experimental, diagnostic, and therapeutic purposes set forth herein.

Monoclonal antibodies can be obtained from a population of substantially homogeneous antibodies, i.e., the individual antibodies comprising the population are identical except for possible naturally occurring mutations that may be present in minor amounts. Thus, the modifier "monoclonal" indicates the character of the antibody as not being a mixture of discrete antibodies.

The anti-SSEA-4 monoclonal antibodies of the invention can be made using a variety of methods known in the art, including the hybridoma method first described by Kohler et al., Nature, 256:495 (1975), or alternatively they may be made by recombinant DNA methods (e.g., U.S. Pat. No. 4,816,567).

### Vectors, Host Cells and Recombinant Methods

For recombinant production of an antibody of the invention, the nucleic acid encoding it is isolated and inserted into a replicable vector for further cloning (amplification of the DNA) or for expression. DNA encoding the antibody is readily isolated and sequenced using conventional procedures (e.g., by using oligonucleotide probes that are capable of binding specifically to genes encoding the heavy and light chains of the antibody). Many vectors are available. The choice of vector depends in part on the host cell to be used. Host cells include, but are not limited to, cells of either prokaryotic or eukaryotic (generally mammalian) origin. It will be appreciated that constant regions of any isotype can be used for this purpose, including IgG, IgM, IgA, IgD, and IgE constant regions, and that such constant regions can be obtained from any human or animal species.

### Generating Antibodies Using Prokaryotic Host Cells

### Vector Construction

Polynucleotide sequences encoding polypeptide components of the antibody of the invention can be obtained using standard recombinant techniques. Desired polynucleotide sequences may be isolated and sequenced from antibody producing cells such as hybridoma cells. Alternatively, polynucleotides can be synthesized using nucleotide synthesizer or PCR techniques. Once obtained, sequences encoding the polypeptides are inserted into a recombinant vector capable of replicating and expressing heterologous polynucleotides in prokaryotic hosts. Many vectors that are available and known in the art can be used for the purpose of the present invention. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides. The vector components generally include, but are not limited to: an origin of replication, a selection marker gene, a promoter, a ribosome binding site (RBS), a signal sequence, the heterologous nucleic acid insert and a transcription termination sequence.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, E. coli is typically transformed using pBR322, a plasmid derived from an E. coli species. pBR322 contains genes encoding ampicillin (Amp) and tetracycline (Tet) resistance and thus provides easy means for identifying transformed cells. pBR322, its derivatives, or other microbial plasmids or bacteriophage may also contain, or be modified to contain, promoters which can be used by the microbial organism for expression of endogenous proteins. Examples of pBR322 derivatives used for expression of particular antibodies are described in detail in Carter et al., U.S. Pat. No. 5,648,237.

In addition, phage vectors containing replicon and control sequences that are compatible with the host microorganism can be used as transforming vectors in connection with these hosts. For example, bacteriophage such as λGEM™-11 may be utilized in making a recombinant vector which can be used to transform susceptible host cells such as E. coli LE392.

The expression vector may comprise two or more promoter-cistron pairs, encoding each of the polypeptide components. A promoter is an untranslated regulatory sequence located upstream (5') to a cistron that modulates its expression. Prokaryotic promoters typically fall into two classes, inducible and constitutive. Inducible promoter is a promoter that initiates increased levels of transcription of the cistron under its control in response to changes in the culture condition, e.g. the presence or absence of a nutrient or a change in temperature.

A large number of promoters recognized by a variety of potential host cells are well known. The selected promoter can be operably linked to cistron DNA encoding the light or heavy chain by removing the promoter from the source DNA via restriction enzyme digestion and inserting the isolated promoter sequence into the vector. Both the native promoter sequence and many heterologous promoters may be used to direct amplification and/or expression of the target genes. Heterologous promoters may be utilized, as they generally permit greater transcription and higher yields of expressed target gene as compared to the native target polypeptide promoter.

Promoters suitable for use with prokaryotic hosts include the PhoA promoter, the β-galactamase and lactose promoter systems, a tryptophan (trp) promoter system and hybrid promoters such as the tac or the trc promoter. However, other promoters that are functional in bacteria (such as other known bacterial or phage promoters) are suitable as well. Their nucleotide sequences have been published, thereby enabling a skilled worker operably to ligate them to cistrons encoding the target light and heavy chains (Siebenlist et al. (1980) Cell 20: 269) using linkers or adaptors to supply any required restriction sites.

Each cistron within the recombinant vector comprises a secretion signal sequence component that directs translocation of the expressed polypeptides across a membrane. In general, the signal sequence may be a component of the vector, or it may be a part of the target polypeptide DNA that is inserted into the vector. The signal sequence selected should be one that is recognized and processed (i.e. cleaved by a signal peptidase) by the host cell. For prokaryotic host cells that do not recognize and process the signal sequences native to the heterologous polypeptides, the signal sequence is substituted by a prokaryotic signal sequence selected, for example, from the group consisting of the alkaline phosphatase, penicillinase, Ipp, or heat-stable enterotoxin II (STII) leaders, LamB, PhoE, PelB, OmpA and MBP. The signal sequences used in both cistrons of the expression system may be STII signal sequences or variants thereof.

The production of the immunoglobulins can occur in the cytoplasm of the host cell, and therefore does not require the presence of secretion signal sequences within each cistron. In that regard, immunoglobulin light and heavy chains are expressed, folded and assembled to form functional immunoglobulins within the cytoplasm. Certain host strains (e.g., the E. coli trxB- strains) provide cytoplasm conditions that are favorable for disulfide bond formation, thereby permitting proper folding and assembly of expressed protein subunits. Proba and Pluckthun Gene, 159:203 (1995).

Antibodies of the invention can also be produced by using an expression system in which the quantitative ratio of expressed polypeptide components can be modulated in order to maximize the yield of secreted and properly assembled antibodies of the invention. Such modulation is accomplished at least in part by simultaneously modulating translational strengths for the polypeptide components.

One technique for modulating translational strength is disclosed in Simmons et al., U.S. Pat. No. 5,840,523. It utilizes variants of the translational initiation region (TIR) within a cistron. For a given TIR, a series of amino acid or nucleic acid sequence variants can be created with a range of translational strengths, thereby providing a convenient means by which to adjust this factor for the desired expression level of the specific chain. TIR variants can be generated by conventional mutagenesis techniques that result in codon changes which can alter the amino acid sequence.Changes in the nucleotide sequence may be silent. Alterations in the TIR can include, for example, alterations in the number or spacing of Shine-Dalgarno sequences, along with alterations in the signal sequence. One method for generating mutant signal sequences is the generation of a "codon bank" at the beginning of a coding sequence that does not change the amino acid sequence of the signal sequence (i.e., the changes are silent). This can be accomplished by changing the third nucleotide position of each codon; additionally, some amino acids, such as leucine, serine, and arginine, have multiple first and second positions that can add complexity in making the bank. This method of mutagenesis is described in detail in Yansura et al. (1992) METHODS: A Companion to Methods in Enzymol. 4:151-158.

A set of vectors may be generated with a range of TIR strengths for each cistron therein. This limited set provides a comparison of expression levels of each chain as well as the yield of the desired antibody products under various TIR strength combinations. TIR strengths can be determined by quantifying the expression level of a reporter gene as described in detail in Simmons et al. U.S. Pat. No. 5,840,523. Based on the translational strength comparison, the desired individual TIRs are selected to be combined in the expression vector constructs.

Prokaryotic host cells suitable for expressing antibodies of the invention include Archaebacteria and Eubacteria, such as Gram-negative or Gram-positive organisms. Examples of useful bacteria include Escherichia (e.g., E. coli), Bacilli (e.g., B. subtilis), Enterobacteria, Pseudomonas species (e.g., P. aeruginosa), Salmonella typhimurium, Serratia marcescans, Klebsiella, Proteus, Shigella, Rhizobia, Vitreoscilla, or Paracoccus. Gram-negative cells may be used. E. coli cells may be used as hosts. Examples of E. coli strains include strain W3110 (Bachmann, Cellular and Molecular Biology, vol. 2 (Washington, D.C.: American Society for Microbiology, 1987), pp. 1190-1219; ATCC Deposit No. 27,325) and derivatives thereof, including strain 33D3 having genotype W3110 ΔfhuA (Δ tonA) ptr3 lac Iq lacL8 ΔompTΔ(nmpc-fepE) degP41 kanR (U.S. Pat. No. 5,639,635). Other strains and derivatives thereof, such as E. coli 294 (ATCC 31,446), E. coli B, E. coli λ1776 (ATCC 31,537) and E. coli RV308 (ATCC 31,608) are also suitable. These examples are illustrative rather than limiting. Methods for constructing derivatives of any of the above-mentioned bacteria having defined genotypes are known in the art and described in, for example, Bass et al., Proteins, 8:309-314 (1990). It is generally necessary to select the appropriate bacteria taking into consideration replicability of the replicon in the cells of a bacterium. For example, E. coli, Serratia, or Salmonella species can be suitably used as the host when well known plasmids such as pBR322, pBR325, pACYC177, or pKN410 are used to supply the replicon. Typically the host cell should secrete minimal amounts of proteolytic enzymes, and additional protease inhibitors may desirably be incorporated in the cell culture.Antibody Production

Host cells are transformed with the above-described expression vectors and cultured in conventional nutrient media modified as appropriate for inducing promoters, selecting transformants, or amplifying the genes encoding the desired sequences.

Transformation means introducing DNA into the prokaryotic host so that the DNA is replicable, either as an extrachromosomal element or by chromosomal integrant. Depending on the host cell used, transformation is done using standard techniques appropriate to such cells. The calcium treatment employing calcium chloride is generally used for bacterial cells that contain substantial cellwall barriers. Another method for transformation employs polyethylene glycol/DMSO. Yet another technique used is electroporation.

Prokaryotic cells used to produce the polypeptides of the invention are grown in media known in the art and suitable for culture of the selected host cells. Examples of suitable media include luria broth (LB) plus necessary nutrient supplementsThe media may also contain a selection agent, chosen based on the construction of the expression vector, to selectively permit growth of prokaryotic cells containing the expression vector. For example, ampicillin is added to media for growth of cells expressing ampicillin resistant gene.

Any necessary supplements besides carbon, nitrogen, and inorganic phosphate sources may also be included at appropriate concentrations introduced alone or as a mixture with another supplement or medium such as a complex nitrogen source. Optionally the culture medium may contain one or more reducing agents selected from the group consisting of glutathione, cysteine, cystamine, thioglycollate, dithioerythritol and dithiothreitol.

The prokaryotic host cells are cultured at suitable temperatures. For E. coli growth, for example, growth occurs at a temperature range including, but not limited to, about 20° C. to about 39° C., about 25° C. to about 37° C., and at about 30° C. The pH of the medium may be any pH ranging from about 5 to about 9, depending mainly on the host organism. For E. coli, the pH can be from about 6.8 to about 7.4, or about 7.0.

If an inducible promoter is used in the expression vector, protein expression is induced under conditions suitable for the activation of the promoter. PhoA promoters are used for controlling transcription of the polypeptides. Accordingly, the transformed host cells are cultured in a phosphate-limiting medium for induction. The the phosphate-limiting medium may be the C.R.A.P medium (see, e.g., Simmons et al., J. Immunol. Methods (2002), 263:133-147). A variety of other inducers may be used, according to the vector construct employed, as is known in the art.

The expressed polypeptides of the present invention are secreted into and recovered from the periplasm of the host cells. Protein recovery typically involves disrupting the microorganism, generally by such means as osmotic shock, sonication or lysis. Once cells are disrupted, cell debris or whole cells may be removed by centrifugation or filtration. The proteins may be further purified, for example, by affinity resin chromatography. Alternatively, proteins can be transported into the culture media and isolated therein. Cells may be removed from the culture and the culture supernatant being filtered and concentrated for further purification of the proteins produced. The expressed polypeptides can be further isolated and identified using commonly known methods such as polyacrylamide gel electrophoresis (PAGE) and Western blot assay.

Antibody production is conducted in large quantity by a fermentation process. Various large-scale fed-batch fermentation procedures are available for production of recombinant proteins. Large-scale fermentations have at least 1000 liters of capacity, for example about 1,000 to 100,000 liters of capacity. These fermentors use agitator impellers to distribute oxygen and nutrients, especially glucose (a common carbon/energy source). Small scale fermentation refers generally to fermentation in a fermentor that is no more than approximately 100 liters in volumetric capacity, and can range from about 1 liter to about 100 liters.

In a fermentation process, induction of protein expression is typically initiated after the cells have been grown under suitable conditions to a desired density, e.g., an OD550 of about 180-220, at which stage the cells are in the early stationary phase. A variety of inducers may be used, according to the vector construct employed, as is known in the art and described above. Cells may be grown for shorter periods prior to induction. Cells are usually induced for about 12-50 hours, although longer or shorter induction time may be used.

To improve the production yield and quality of the polypeptides of the invention, various fermentation conditions can be modified. For example, to improve the proper assembly and folding of the secreted antibody polypeptides, additional vectors overexpressing chaperone proteins, such as Dsb proteins (DsbA, DsbB, DsbC, DsbD and or DsbG) or FkpA (a peptidylprolyl cis,trans-isomerase with chaperone activity) can be used to co-transform the host prokaryotic cells. The chaperone proteins have been demonstrated to facilitate the proper folding and solubility of heterologous proteins produced in bacterial host cells. Chen et al. (1999) J Bio Chem 274:19601-19605; Georgiou et al., U.S. Pat. No. 6,083,715; Georgiou et al., U.S. Pat. No. 6,027,888; Bothmann and Pluckthun (2000) J. Biol. Chem. 275:17100-17105; Ramm and Pluckthun (2000) J. Biol. Chem. 275:17106-17113; Arie et al. (2001) Mol. Microbiol. 39:199-210.

To minimize proteolysis of expressed heterologous proteins (especially those that are proteolytically sensitive), certain host strains deficient for proteolytic enzymes can be used. For example, host cell strains may be modified to effect genetic mutation(s) in the genes encoding known bacterial proteases such as Protease III, OmpT, DegP, Tsp, Protease I, Protease Mi, Protease V, Protease VI and combinations thereof. Some E. coli protease-deficient strains are available and described in, for example, Joly et al. (1998), supra; Georgiou et al., U.S. Pat. No. 5,264,365; Georgiou et al., U.S. Pat. No. 5,508,192; Hara et al., Microbial Drug Resistance, 2:63-72 (1996).

E. coli strains deficient for proteolytic enzymes and transformed with plasmids overexpressing one or more chaperone proteins may be used as host cells in the expression system.

### Antibody Purification

The antibody protein produced herein may be further purified to obtain preparations that are substantially homogeneous for further assays and uses. Standard protein purification methods known in the art can be employed. The following procedures are exemplary of suitable purification procedures: fractionation on immunoaffinity or ion-exchange columns, ethanol precipitation, reverse phase HPLC, chromatography on silica or on a cation-exchange resin such as DEAE, chromatofocusing, SDS-PAGE, ammonium sulfate precipitation, and gel filtration using, for example, Sephadex G-75.

Protein A immobilized on a solid phase is used for immunoaffinity purification of the antibody products of the invention. Protein A is a 41 kD cell wall protein from Staphylococcus aureas which binds with a high affinity to the Fc region of antibodies. Lindmark et al (1983) J. Immunol. Meth. 62:1-13. The solid phase to which Protein A is immobilized can be a column comprising a glass or silica surface, or a controlled pore glass column or a silicic acid column. In some applications, the column is coated with a reagent, such as glycerol, to possibly prevent nonspecific adherence of contaminants.

As the first step of purification, the preparation derived from the cell culture as described above can be applied onto a Protein A immobilized solid phase to allow specific binding of the antibody of interest to Protein A. The solid phase would then be washed to remove contaminants non-specifically bound to the solid phase. Finally the antibody of interest is recovered from the solid phase by elution.

### Generating Antibodies Using Eukaryotic Host Cells

The vector components generally include, but are not limited to, one or more of the following: a signal sequence, an origin of replication, one or more marker genes, an enhancer element, a promoter, and a transcription termination sequence.

### (i) Signal Sequence Component

A vector for use in a eukaryotic host cell may also contain a signal sequence or other polypeptide having a specific cleavage site at the N-terminus of the mature protein or polypeptide of interest. The heterologous signal sequence selected generally is one that is recognized and processed (i.e., cleaved by a signal peptidase) by the host cell. In mammalian cell expression, mammalian signal sequences as well as viral secretory leaders, for example, the herpes simplex gD signal, are available.

The DNA for such precursor region is ligated in reading frame to DNA encoding the antibody.

### (ii) Origin of Replication

Generally, an origin of replication component is not needed for mammalian expression vectors. For example, the SV40 origin may typically be used only because it contains the early promoter.

### (iii) Selection Gene Component

Expression and cloning vectors may contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, where relevant, or (c) supply critical nutrients not available from complex media.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the antibody nucleic acid, such as DHFR, thymidine kinase, metallothionein-I and -II (e.g., primate metallothionein genes), adenosine deaminase, ornithine decarboxylase, etc.

For example, cells transformed with the DHFR selection gene may first be identified by culturing all of the transformants in a culture medium that contains methotrexate (Mtx), a competitive antagonist of DHFR. Appropriate host cells when wild-type DHFR is employed include, for example, the Chinese hamster ovary (CHO) cell line deficient in DHFR activity (e.g., ATCC CRL-9096).

Alternatively, host cells (particularly wild-type hosts that contain endogenous DHFR) transformed or co-transformed with DNA sequences encoding an antibody, wild-type DHFR protein, and another selectable marker such as aminoglycoside 3'-phosphotransferase (APH) can be selected by cell growth in medium containing a selection agent for the selectable marker such as an aminoglycosidic antibiotic, e.g., kanamycin, neomycin, or G418. See U.S. Pat. No. 4,965,199.

### (iv) Promoter Component

Expression and cloning vectors usually contain a promoter that is recognized by the host organism and is operably linked to nucleic acid encoding a polypeptide of interest (e.g., an antibody). Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Antibody polypeptide transcription from vectors in mammalian host cells can be controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, or from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Pat. No. 4,419,446. A modification of this system is described in U.S. Pat. No. 4,601,978. See also Reyes et al., Nature 297:598-601 (1982) on expression of human β-interferon cDNA in mouse cells under the control of a thymidine kinase promoter from herpes simplex virus. Alternatively, the Rous Sarcoma Virus long terminal repeat can be used as the promoter.

### (v) Enhancer Element Component

Transcription of DNA encoding an antibody polypeptide of the invention by higher eukaryotes can often be increased by inserting an enhancer sequence into the vector. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18 (1982) on enhancing elements for activation of eukaryotic promoters. The enhancer may be spliced into the vector at a position 5' or 3' to the antibody polypeptide-encoding sequence, but is generally located at a site 5' from the promoter.

### (vi) Transcription Termination Component

Expression vectors used in eukaryotic host cells will typically also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding an antibody. One useful transcription termination component is the bovine growth hormone polyadenylation region. See WO94/11026 and the expression vector disclosed therein.

### (vii) Selection and Transformation of Host Cells

Suitable host cells for cloning or expressing the DNA in the vectors herein include higher eukaryote cells described herein, including vertebrate host cells. Propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for antibody production and cultured in conventional nutrient media modified as appropriate for inducing promoters, selecting transformants, or amplifying the genes encoding the desired sequences.

### (viii) Culturing the Host Cells

The host cells used to produce an antibody of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430; WO 87/00195; or U.S. Pat. Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### (ix) Purification of Antibody

When using recombinant techniques, the antibody can be produced intracellularly, or directly secreted into the medium. If the antibody is produced intracellularly, as a first step, the particulate debris, either host cells or lysed fragments, are generally removed, for example, by centrifugation or ultrafiltration. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being a generally acceptable purification technique. The suitability of affinity reagents such as protein A as an affinity ligand depends on the species and isotype of any immunoglobulin Fc domain that is present in the antibody. Protein A can be used to purify antibodies that are based on human γ1, γ2, or γ4 heavy chains (Lindmark et al., J. Immunol. Meth. 62:1-13 (1983)). Protein G is recommended for all mouse isotypes and for human γ3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a CH3 domain, the Bakerbond ABX™ resin (J. T. Baker, Phillipsburg, N.J.) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to further purification steps, as necessary, for example by low pH hydrophobic interaction chromatography using an elution buffer at a pH between about 2.5-4.5, generally performed at low salt concentrations (e.g., from about 0-0.25M salt).

It should be noted that, in general, techniques and methodologies for preparing antibodies for use in research, testing and clinical use are well-established in the art, consistent with the above and/or as deemed appropriate by one skilled in the art for the particular antibody of interest.

### Activity Assays

Antibodies of the invention can be characterized for their physical/chemical properties and biological functions by various assays known in the art.

Purified antibodies can be further characterized by a series of assays including, but not limited to, N-terminal sequencing, amino acid analysis, non-denaturing size exclusion high pressure liquid chromatography (HPLC), mass spectrometry, ion exchange chromatography and papain digestion.

Where necessary, antibodies are analyzed for their biological activity. Antibodies of the invention may be tested for their antigen binding activity. The antigen binding assays that are known in the art and can be used herein include without limitation any direct or competitive binding assays using techniques such as western blots, radioimmunoassays, ELISA (enzyme linked immunosorbent assay), "sandwich" immunoassays, immunoprecipitation assays, fluorescent immunoassays, chemiluminescent immunoassays, nanoparticle immunoassays, aptamer immunoassays, and protein A immunoassays.

### Antibody Fragments

The present invention encompasses antibody fragments. In certain circumstances there are advantages of using antibody fragments, rather than whole antibodies. The smaller size of the fragments allows for rapid clearance, and may lead to improved access to solid tumors.

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from E. coli, thus allowing the facile production of large amounts of these fragments. Antibody fragments can be isolated from the antibody phage libraries discussed above. Alternatively, Fab'-SH fragments can be directly recovered from E. coli and chemically coupled to form F(ab')2 fragments (Carter et al., Bio/Technology 10: 163-167 (1992)). According to another approach, F(ab')2 fragments can be isolated directly from recombinant host cell culture. Fab and F(ab')2 fragment with increased in vivo half-life comprising salvage receptor binding epitope residues are described in U.S. Pat. No. 5,869,046. Other techniques for the production of antibody fragments will be apparent to the skilled practitioner. In other embodiments, the antibody of choice is a single chain Fv fragment (scFv). See WO 93/16185; U.S. Pat. Nos. 5,571,894; and 5,587,458. Fv and sFv are the only species with intact combining sites that are devoid of constant regions; thus, they are suitable for reduced nonspecific binding during in vivo use. sFv fusion proteins may be constructed to yield fusion of an effector protein at either the amino or the carboxy terminus of an sFv. See Antibody Engineering, ed. Borrebaeck, supra. The antibody fragment may also be a "linear antibody", e.g., as described in U.S. Pat. No. 5,641,870 for example. Such linear antibody fragments may be monospecific or bispecific.

### Humanized Antibodies

The invention encompasses humanized antibodies. Various methods for humanizing non-human antibodies are known in the art. For example, a humanized antibody can have one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al. (1986) Nature 321:522-525; Riechmann et al. (1988) Nature 332:323-327; Verhoeyen et al. (1988) Science 239:1534-1536), by substituting hypervariable region sequences for the corresponding sequences of a human antibody. Accordingly, such "humanized" antibodies are chimeric antibodies (U.S. Pat. No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies can be important to reduce antigenicity. According to the so-called "best-fit" method, the sequence of the variable domain of a rodent antibody is screened against the entire library of known human variable-domain sequences. The human sequence which is closest to that of the rodent is then accepted as the human framework for the humanized antibody (Sims et al. (1993) J. Immunol. 151:2296; Chothia et al. (1987) J. Mol. Biol. 196:901. Another method uses a particular framework derived from the consensus sequence of all human antibodies of a particular subgroup of light or heavy chains. The same framework may be used for several different humanized antibodies (Carter et al. (1992) Proc. Natl. Acad. Sci. USA, 89:4285; Presta et al. (1993) J. Immunol., 151:2623.

It is further generally desirable that antibodies be humanized with retention of high affinity for the antigen and other favorable biological properties. To achieve this goal, according to one method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

### Human Antibodies (not part of the present invention)

Human anti-SSEA-4 antibodies can be constructed by combining Fv clone variable domain sequence(s) selected from human-derived phage display libraries with known human constant domain sequences(s) as described above. Alternatively, human monoclonal anti-SSEA-4 antibodies can be made by the hybridoma method. Human myeloma and mouse-human heteromyeloma cell lines for the production of human monoclonal antibodies have been described, for example, by Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).

It is now possible to produce transgenic animals (e.g. mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci. USA, 90: 2551 (1993); Jakobovits et al., Nature, 362: 255 (1993); Bruggermann et al., Year in Immunol., 7: 33 (1993).

Gene shuffling can also be used to derive human antibodies from non-human, e.g. rodent, antibodies, where the human antibody has similar affinities and specificities to the starting non-human antibody. According to this method, which is also called "epitope imprinting", either the heavy or light chain variable region of a non-human antibody fragment obtained by phage display techniques as described above is replaced with a repertoire of human V domain genes, creating a population of non-human chain/human chain scFv or Fab chimeras. Selection with antigen results in isolation of a non-human chain/human chain chimeric scFv or Fab wherein the human chain restores the antigen binding site destroyed upon removal of the corresponding non-human chain in the primary phage display clone, i.e. the epitope governs (imprints) the choice of the human chain partner. When the process is repeated in order to replace the remaining non-human chain, a human antibody is obtained (see PCT WO 93/06213 published Apr. 1, 1993). Unlike traditional humanization of non-human antibodies by CDR grafting, this technique provides completely human antibodies, which have no FR or CDR residues of non-human origin.

### Bispecific Antibodies (not part of the present invention)

Bispecific antibodies are monoclonal antibodies that have binding specificities for at least two different antigens. Bispecific antibodies are human or humanized antibodies. One of the binding specificities is for SSEA-4 including a specific lysine linkage and the other is for any other antigen. Bispecific antibodies may bind to two different SSEA-4 having two different lysine linkages. Bispecific antibodies can be prepared as full length antibodies or antibody fragments (e.g. F(ab')2 bispecific antibodies).

Methods for making bispecific antibodies are known in the art. Traditionally, the recombinant production of bispecific antibodies is based on the co-expression of two immunoglobulin heavy chain-light chain pairs, where the two heavy chains have different specificities (Milstein and Cuello, Nature, 305: 537 (1983)). Because of the random assortment of immunoglobulin heavy and light chains, these hybridomas (quadromas) produce a potential mixture of 10 different antibody molecules, of which only one has the correct bispecific structure. The purification of the correct molecule, which is usually done by affinity chromatography steps, is rather cumbersome, and the product yields are low. Similar procedures are disclosed in WO 93/08829 published May 13, 1993, and in Traunecker et al., EMBO J., 10: 3655 (1991).

Antibody variable domains with the desired binding specificities (antibody-antigen combining sites) may be fused to immunoglobulin constant domain sequences. The fusion, for example, is with an immunoglobulin heavy chain constant domain, comprising at least part of the hinge, CH2, and CH3 regions. The first heavy-chain constant region (CH1), containing the site necessary for light chain binding, may be present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

The bispecific antibodies may becomposed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach, the interface between a pair of antibody molecules can be engineered to maximize the percentage of heterodimers which are recovered from recombinant cell culture. The interface comprises at least a part of the CH3 domain of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Pat. No. 4,676,980), and for treatment of HIV infection (WO 91/00360, WO 92/00373, and EP 03089). Heteroconjugate antibodies may be made using any convenient cross-linking methods. Suitable cross-linking agents are well known in the art, and are disclosed in U.S. Pat. No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F(ab')2 fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from E. coli, which can be chemically coupled to form bispecific antibodies. Shalaby et al., J. Exp. Med., 175: 217-225 (1992) describe the production of a fully humanized bispecific antibody F(ab')2 molecule. Each Fab fragment was separately secreted from E. coli and subjected to directed chemical coupling in vitro to form the bispecific antibody. The bispecific antibody thus formed was able to bind to cells overexpressing the HER2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al. J. Immunol. 147: 60 (1991).

### Multivalent Antibodies (not part of the present invention)

A multivalent antibody may be internalized (and/or catabolized) faster than a bivalent antibody by a cell expressing an antigen to which the antibodies bind. The antibodies can be multivalent antibodies (which are other than of the IgM class) with three or more antigen binding sites (e.g. tetravalent antibodies), which can be readily produced by recombinant expression of nucleic acid encoding the polypeptide chains of the antibody. The multivalent antibody can comprise a dimerization domain and three or more antigen binding sites. The dimerization domain comprises (or consists of), for example, an Fc region or a hinge region. In this scenario, the antibody will comprise an Fc region and three or more antigen binding sites amino-terminal to the Fc region. A multivalent antibody may comprise (or consist of), for example, three to about eight, or four antigen binding sites. The multivalent antibody comprises at least one polypeptide chain (for example, two polypeptide chains), wherein the polypeptide chain(s) comprise two or more variable domains. For instance, the polypeptide chain(s) may comprise VD1-(X1)n-VD2-(X2)n-Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein may further comprise at least two (for example, four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain. Antibody Variants

Amino acid sequence modification(s) of the antibodies are described herein. For example, it may be desirable to improve the binding affinity and/or other biological properties of the antibody. Amino acid sequence variants of the antibody are prepared by introducing appropriate nucleotide changes into the antibody nucleic acid, or by peptide synthesis. Such modifications include, for example, deletions from, and/or insertions into and/or substitutions of, residues within the amino acid sequences of the antibody. Any combination of deletion, insertion, and substitution can be made to arrive at the final construct, provided that the final construct possesses the desired characteristics. The amino acid alterations may be introduced in the subject antibody amino acid sequence at the time that sequence is made.

A useful method for identification of certain residues or regions of the antibody that are preferred locations for mutagenesis is called "alanine scanning mutagenesis" as described by Cunningham and Wells (1989) Science, 244:1081-1085. Here, a residue or group of target residues are identified (e.g., charged residues such as Arg, Asp, His, Lys, and Glu) and replaced by a neutral or negatively charged amino acid (e.g., alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at the target codon or region and the expressed immunoglobulins are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to a cytotoxic polypeptide. Other insertional variants of the antibody molecule include the fusion to the N- or C-terminus of the antibody to an enzyme (e.g. for ADEPT) or a polypeptide which increases the serum half-life of the antibody.

Another type of variant is an amino acid substitution variant. These variants have at least one amino acid residue in the antibody molecule replaced by a different residue. The sites of greatest interest for substitutional mutagenesis include the hypervariable regions, but FR alterations are also contemplated. Conservative substitutions are shown in Table A under the heading of "preferred substitutions". If such substitutions result in a change in biological activity, then more substantial changes, denominated "exemplary substitutions" in Table A, or as further described below in reference to amino acid classes, may be introduced and the products screened.

**TABLE A**

| Original Exemplary Preferred |
|---|
| Residue Substitutions |
| Ala (A) Val; Leu; Ile Val |
| Arg (R) Lys; Gln; Asn Lys |
| Asn (N) Gln; His; Asp, Lys; Arg Gln |
| Asp (D) Glu; Asn Glu |
| Cys (C) Ser; Ala Ser |
| Gln (Q) Asn; Glu Asn |
| Glu (E) Asp; Gln Asp |
| Gly (G) Ala Ala |
| His (H) Asn; Gln; Lys; Arg Arg |
| Ile (I) Leu; Val; Met; Ala; Leu |
| Phe; Norleucine |
| Leu (L) Norleucine; Ile; Val; Ile |
| Met; Ala; Phe |
| Lys (K) Arg; Gln; Asn Arg |
| Met (M) Leu; Phe; Ile Leu |
| Phe (F) Trp; Leu; Val; Ile; Ala; Tyr Tyr |
| Pro (P) Ala Ala |
| Ser (S) Thr Thr |
| Thr (T) Val; Ser Ser |
| Trp (W) Tyr; Phe Tyr |
| Tyr (Y) Trp; Phe; Thr; Ser Phe |
| Val (V) Ile; Leu; Met; Phe; Leu |
| Ala; Norleucine |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Amino acids may be grouped according to similarities in the properties of their side chains (in A. L. Lehninger, in Biochemistry, second ed., pp. 73-75, Worth Publishers, New York (1975)):
- (1) non-polar: Ala (A), Val (V), Leu (L), Ile (I), Pro (P), Phe (F), Trp (W), Met (M)
- (2) uncharged polar: Gly (G), Ser (S), Thr (T), Cys (C), Tyr (Y), Asn (N), Gln (O)
- (3) acidic: Asp (D), Glu (E)
- (4) basic: Lys (K), Arg (R), His (H)

Alternatively, naturally occurring residues may be divided into groups based on common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class. Such substituted residues also may be introduced into the conservative substitution sites or, into the remaining (non-conserved) sites.

One type of substitutional variant involves substituting one or more hypervariable region residues of a parent antibody (e.g. a humanized or human antibody). Generally, the resulting variant(s) selected for further development will have modified (e.g., improved) biological properties relative to the parent antibody from which they are generated. A convenient way for generating such substitutional variants involves affinity maturation using phage display. Briefly, several hypervariable region sites (e.g. 6-7 sites) are mutated to generate all possible amino acid substitutions at each site. The antibodies thus generated are displayed from filamentous phage particles as fusions to at least part of a phage coat protein (e.g., the gene III product of M13) packaged within each particle. The phage-displayed variants are then screened for their biological activity (e.g. binding affinity) as herein disclosed. In order to identify candidate hypervariable region sites for modification, scanning mutagenesis (e.g., alanine scanning) can be performed to identify hypervariable region residues contributing significantly to antigen binding. Alternatively, or additionally, it may be beneficial to analyze a crystal structure of the antigen-antibody complex to identify contact points between the antibody and antigen. Such contact residues and neighboring residues are candidates for substitution according to techniques known in the art, including those elaborated herein. Once such variants are generated, the panel of variants is subjected to screening using techniques known in the art, including those described herein, and antibodies with superior properties in one or more relevant assays may be selected for further development.

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

It may be desirable to introduce one or more amino acid modifications in an Fc region of antibodies of the invention, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions including that of a hinge cysteine.

### Immunoconjugates

In another aspect, the invention provides immunoconjugates, or antibody-drug conjugates (ADC), comprising an antibody conjugated to a cytotoxic agent such as a chemotherapeutic agent, a drug, a growth inhibitory agent, a toxin (e.g., an enzymatically active toxin of bacterial, fungal, plant, or animal origin, or fragments thereof), or a radioactive isotope (i.e., a radioconjugate).

The use of antibody-drug conjugates for the local delivery of cytotoxic or cytostatic agents, i.e. drugs to kill or inhibit tumor cells in the treatment of cancer (Syrigos and Epenetos (1999) Anticancer Research 19:605-614; Niculescu-Duvaz and Springer (1997) Adv. Drg Del. Rev. 26:151-172; U.S. Pat. No. 4,975,278) allows targeted delivery of the drug moiety to tumors, and intracellular accumulation therein, where systemic administration of these unconjugated drug agents may result in unacceptable levels of toxicity to normal cells as well as the tumor cells sought to be eliminated (Baldwin et al., (1986) Lancet pp. (Mar. 15, 1986):603-05; Thorpe, (1985) "Antibody Carriers Of Cytotoxic Agents In Cancer Therapy: A Review," in Monoclonal Antibodies '84: Biological And Clinical Applications, A. Pinchera et al. (ed.s), pp. 475-506). Maximal efficacy with minimal toxicity is sought thereby. Both polyclonal antibodies and monoclonal antibodies have been reported as useful in these strategies (Rowland et al., (1986) Cancer Immunol. Immunother., 21:183-87). Drugs used in these methods include daunomycin, doxorubicin, methotrexate, and vindesine (Rowland et al., (1986) supra). Toxins used in antibody-toxin conjugates include bacterial toxins such as diphtheria toxin, plant toxins such as ricin, small molecule toxins such as geldanamycin (Mandler et al (2000) Jour. of the Nat. Cancer Inst. 92(19):1573-1581; Mandler et al (2000) Bioorganic & Med. Chem. Letters 10:1025-1028; Mandler et al (2002) Bioconjugate Chem. 13:786-791), maytansinoids (EP 1391213; Liu et al., (1996) Proc. Natl. Acad. Sci. USA 93:8618-8623), and calicheamicin (Lode et al (1998) Cancer Res. 58:2928; Hinman et al (1993) Cancer Res. 53:3336-3342). The toxins may effect their cytotoxic and cytostatic effects by mechanisms including tubutin binding, DNA binding, or topoisomerase inhibition. Some cytotoxic drugs tend to be inactive or less active when conjugated to large antibodies or protein receptor ligands.

### Antibody Derivatives

Antibodies of the invention can be further modified to contain additional nonproteinaceous moieties that are known in the art and readily available. In one embodiment, the moieties suitable for derivatization of the antibody are water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, the polymers can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### Pharmaceutical Formulations

Therapeutic formulations comprising an antibody of the invention are prepared for storage by mixing the antibody having the desired degree of purity with optional physiologically acceptable carriers, excipients or stabilizers (Remington's Pharmaceutical Sciences 16th edition, Osol, A. Ed. (1980)), in the form of aqueous solutions, lyophilized or other dried formulations. Acceptable carriers, excipients, or stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™ or polyethylene glycol (PEG).

The formulation herein may also contain more than one active compound as necessary for the particular indication being treated, including, but not limited to those with complementary activities that do not adversely affect each other. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for in vivo administration must be sterile. This is readily accomplished by filtration through sterile filtration membranes.

Sustained-release preparations may be prepared. Suitable examples of sustained-release preparations include semipermeable matrices of solid hydrophobic polymers containing the immunoglobulin of the invention, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated immunoglobulins remain in the body for a long time, they may denature or aggregate as a result of exposure to moisture at 37° C., resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### Uses

Antibodies of the invention are used for the treatment of cancer.

The antibody of the invention is specific for SSEA-4.

In certain embodiments, an immunoconjugate comprising an antibody of the invention conjugated with a cytotoxic agent is administered to the patient. In some embodiments, the immunoconjugate and/or antigen to which it is bound is/are internalized by cells expressing one or more proteins on their cell surface which are associated with SSEA-4, resulting in increased therapeutic efficacy of the immunoconjugate in killing the target cell with which it is associated. In one embodiment, the cytotoxic agent targets or interferes with nucleic acid in the target cell. Examples of such cytotoxic agents include any of the chemotherapeutic agents noted herein (such as a maytansinoid or a calicheamicin), a radioactive isotope, or a ribonuclease or a DNA endonuclease.

Antibodies of the invention can be used either alone or in combination with other compositions in a therapy. For instance, an antibody of the invention may be co-administered with another antibody, and/or adjuvant/therapeutic agents (e.g., steroids). For instance, an antibody of the invention may be combined with an anti-inflammatory and/or antiseptic in a treatment scheme, e.g. in treating any of the diseases described herein, including cancer, muscular disorders, ubiquitin-pathway-related genetic disorders, immune/inflammatory disorders, neurological disorders, and other ubiquitin pathway-related disorders. Such combined therapies noted above include combined administration (where the two or more agents are included in the same or separate formulations), and separate administration, in which case, administration of the antibody of the invention can occur prior to, and/or following, administration of the adjunct therapy or therapies.

An antibody of the invention (and adjunct therapeutic agent) can be administered by any suitable means, including parenteral, subcutaneous, intraperitoneal, intrapulmonary, and intranasal, and, if desired for local treatment, intralesional administration. Parenteral infusions include intramuscular, intravenous, intraarterial, intraperitoneal, or subcutaneous administration. In addition, the antibody is suitably administered by pulse infusion, particularly with declining doses of the antibody. Dosing can be by any suitable route, e.g. by injections, such as intravenous or subcutaneous injections, depending in part on whether the administration is brief or chronic.

The location of the binding target of an antibody of the invention may be taken into consideration in preparation and administration of the antibody. When the binding target is an intracellular molecule, certain embodiments of the invention provide for the antibody or antigen-binding fragment thereof to be introduced into the cell where the binding target is located. In one embodiment, an antibody of the invention can be expressed intracellularly as an intrabody. The term "intrabody," as used herein, refers to an antibody or antigen-binding portion thereof that is expressed intracellularly and that is capable of selectively binding to a target molecule, as described in Marasco, Gene Therapy 4: 11-15 (1997); Kontermann, Methods 34: 163-170 (2004); U.S. Pat. Nos. 6,004,940 and 6,329,173; U.S. Patent Application Publication No. 2003/0104402, and PCT Publication No. WO2003/077945. Intracellular expression of an intrabody is effected by introducing a nucleic acid encoding the desired antibody or antigen-binding portion thereof (lacking the wild-type leader sequence and secretory signals normally associated with the gene encoding that antibody or antigen-binding fragment) into a target cell. Any standard method of introducing nucleic acids into a cell may be used, including, but not limited to, microinjection, ballistic injection, electroporation, calcium phosphate precipitation, liposomes, and transfection with retroviral, adenoviral, adeno-associated viral and vaccinia vectors carrying the nucleic acid of interest. One or more nucleic acids encoding all or a portion of an anti-SSEA-4 antibody of the invention can be delivered to a target cell, such that one or more intrabodies are expressed which are capable of intracellular binding to a SSEA-4 and modulation of one or more SSEA-4-mediated cellular pathways.

Antibodies can possess certain characteristics that enhance delivery of antibodies into cells, or can be modified to possess such characteristics. Techniques for achieving this are known in the art. For example, cationization of an antibody is known to facilitate its uptake into cells (see, e.g., U.S. Pat. No. 6,703,019). Lipofections or liposomes can also be used to deliver the antibody into cells. Where antibody fragments are used, the smallest inhibitory fragment that specifically binds to the binding domain of the target protein is generally advantageous. For example, based upon the variable-region sequences of an antibody, peptide molecules can be designed that retain the ability to bind the target protein sequence. Such peptides can be synthesized chemically and/or produced by recombinant DNA technology. See, e.g., Marasco et al., Proc. Natl. Acad. Sci. USA, 90: 7889-7893 (1993).

Entry of modulator polypeptides into target cells can be enhanced by methods known in the art. For example, certain sequences, such as those derived from HIV Tat or the Antennapedia homeodomain protein are able to direct efficient uptake of heterologous proteins across cell membranes. See, e.g., Chen et al., Proc. Natl. Acad. Sci. USA (1999), 96:4325-4329.

When the binding target is located in the brain, certain embodiments of the invention provide for the antibody or antigen-binding fragment thereof to traverse the blood-brain barrier. Certain neurodegenerative diseases are associated with an increase in permeability of the blood-brain barrier, such that the antibody or antigen-binding fragment can be readily introduced to the brain. When the blood-brain barrier remains intact, several art-known approaches exist for transporting molecules across it, including, but not limited to, physical methods, lipid-based methods, and receptor and channel-based methods.

Physical methods of transporting the antibody or antigen-binding fragment across the blood-brain barrier include, but are not limited to, circumventing the blood-brain barrier entirely, or by creating openings in the blood-brain barrier. Circumvention methods include, but are not limited to, direct injection into the brain (see, e.g., Papanastassiou et al., Gene Therapy 9: 398-406 (2002)), interstitial infusion/convection-enhanced delivery (see, e.g., Bobo et al., Proc. Natl. Acad. Sci. USA 91: 2076-2080 (1994)), and implanting a delivery device in the brain (see, e.g., Gill et al., Nature Med. 9: 589-595 (2003); and Gliadel Wafers™, Guildford Pharmaceutical). Methods of creating openings in the barrier include, but are not limited to, ultrasound (see, e.g., U.S. Patent Publication No. 2002/0038086), osmotic pressure (e.g., by administration of hypertonic mannitol (Neuwelt, E. A., Implication of the Blood-Brain Barrier and its Manipulation, Vols 1 & 2, Plenum Press, N.Y. (1989))), permeabilization by, e.g., bradykinin or permeabilizer A-7 (see, e.g., U.S. Pat. Nos. 5,112,596, 5,268,164, 5,506,206, and 5,686,416), and transfection of neurons that straddle the blood-brain barrier with vectors containing genes encoding the antibody or antigen-binding fragment (see, e.g., U.S. Patent Publication No. 2003/0083299).

Lipid-based methods of transporting the antibody or antigen-binding fragment across the blood-brain barrier include, but are not limited to, encapsulating the antibody or antigen-binding fragment in liposomes that are coupled to antibody binding fragments that bind to receptors on the vascular endothelium of the blood-brain barrier (see, e.g., U.S. Patent Application Publication No. 20020025313), and coating the antibody or antigen-binding fragment in low-density lipoprotein particles (see, e.g., U.S. Patent Application Publication No. 20040204354) or apolipoprotein E (see, e.g., U.S. Patent Application Publication No. 20040131692).

Receptor and channel-based methods of transporting the antibody or antigen-binding fragment across the blood-brain barrier include, but are not limited to, using glucocorticoid blockers to increase permeability of the blood-brain barrier (see, e.g., U.S. Patent Application Publication Nos. 2002/0065259, 2003/0162695, and 2005/0124533); activating potassium channels (see, e.g., U.S. Patent Application Publication No. 2005/0089473), inhibiting ABC drug transporters (see, e.g., U.S. Patent Application Publication No. 2003/0073713); coating antibodies with a transferrin and modulating activity of the one or more transferrin receptors (see, e.g., U.S. Patent Application Publication No. 2003/0129186), and cationizing the antibodies (see, e.g., U.S. Pat. No. 5,004,697).

The antibody composition of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibodies of the invention present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody of the invention (when used alone or in combination with other agents such as chemotherapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 µg/kg to 15 mg/kg (e.g. 0.1 mg/kg-10 mg/kg) of antibody can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the antibody). An initial higher loading dose, followed by one or more lower doses may be administered. An exemplary dosing regimen comprises administering an initial loading dose of about 4 mg/kg, followed by a weekly maintenance dose of about 2 mg/kg of the antibody. However, other dosage regimens may be useful. The progress of this therapy is easily monitored by conventional techniques and assays.

### Articles of Manufacture (not part of the present invention)

An article of manufacture containing materials useful for the treatment, prevention and/or diagnosis of the disorders described above is described. The article of manufacture comprises a container and a label or package insert on or associated with the container. Suitable containers include, for example, bottles, vials, syringes, etc. The containers may be formed from a variety of materials such as glass or plastic. The container holds a composition which is by itself or when combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody of the invention. The label or package insert indicates that the composition is used for treating the condition of choice. Moreover, the article of manufacture may comprise (a) a first container with a composition contained therein, wherein the composition comprises an antibody of the invention; and (b) a second container with a composition contained therein, wherein the composition comprises a further cytotoxic or otherwise therapeutic agent. The article of manufacture may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

In certain embodiments, the subject being treated is a mammal. In certain embodiments, the subject is a human. In certain embodiments, the subject is a domesticated animal, such as a dog, cat, cow, pig, horse, sheep, or goat. In certain embodiments, the subject is a companion animal such as a dog or cat. In certain embodiments, the subject is a livestock animal such as a cow, pig, horse, sheep, or goat. In certain embodiments, the subject is a zoo animal. In another embodiment, the subject is a research animal such as a rodent, dog, or non-human primate. In certain embodiments, the subject is a non-human transgenic animal such as a transgenic mouse or transgenic pig.

### Pharmaceutical Compositions and Formulations

After preparation of the antibodies as described herein, "pre-lyophilized formulation" can be produced. The antibody for preparing the formulation is preferably essentially pure and desirably essentially homogeneous (i.e. free from contaminating proteins etc). "Essentially pure" protein means a composition comprising at least about 90% by weight of the protein, based on total weight of the composition, preferably at least about 95% by weight. "Essentially homogeneous" protein means a composition comprising at least about 99% by weight of protein, based on total weight of the composition. The protein is an antibody.

The amount of antibody in the pre-lyophilized formulation is determined taking into account the desired dose volumes, mode(s) of administration etc. Where the protein of choice is an intact antibody (a full-length antibody), from about 2 mg/mL to about 50 mg/mL, preferably from about 5 mg/mL to about 40 mg/mL and most preferably from about 20-30 mg/mL is an exemplary starting protein concentration. The protein is generally present in solution. For example, the protein may be present in a pH-buffered solution at a pH from about 4-8, and preferably from about 5-7. Exemplary buffers include histidine, phosphate, Tris, citrate, succinate and other organic acids. The buffer concentration can be from about 1 mM to about 20 mM, or from about 3 mM to about 15 mM, depending, for example, on the buffer and the desired isotonicity of the formulation (e.g. of the reconstituted formulation). The preferred buffer is histidine in that, as demonstrated below, this can have lyoprotective properties. Succinate was shown to be another useful buffer.

The lyoprotectant is added to the pre-lyophilized formulation. In preferred embodiments, the lyoprotectant is a non-reducing sugar such as sucrose or trehalose. The amount of lyoprotectant in the pre-lyophilized formulation is generally such that, upon reconstitution, the resulting formulation will be isotonic. However, hypertonic reconstituted formulations may also be suitable. In addition, the amount of lyoprotectant must not be too low such that an unacceptable amount of degradation/aggregation of the protein occurs upon lyophilization. Where the lyoprotectant is a sugar (such as sucrose or trehalose) and the protein is an antibody, exemplary lyoprotectant concentrations in the pre-lyophilized formulation are from about 10 mM to about 400 mM, and preferably from about 30 mM to about 300 mM, and most preferably from about 50 mM to about 100 mM.

The ratio of protein to lyoprotectant is selected for each protein and lyoprotectant combination. In the case of an antibody as the protein of choice and a sugar (e.g., sucrose or trehalose) as the lyoprotectant for generating an isotonic reconstituted formulation with a high protein concentration, the molar ratio of lyoprotectant to antibody may be from about 100 to about 1500 moles lyoprotectant to 1 mole antibody, and preferably from about 200 to about 1000 moles of lyoprotectant to 1 mole antibody, for example from about 200 to about 600 moles of lyoprotectant to 1 mole antibody.

In preferred embodiments of the invention, it has been found to be desirable to add a surfactant to the pre-lyophilized formulation. Alternatively, or in addition, the surfactant may be added to the lyophilized formulation and/or the reconstituted formulation. Exemplary surfactants include nonionic surfactants such as polysorbates (e.g. polysorbates 20 or 80); poloxamers (e.g. poloxamer 188); Triton; sodium dodecyl sulfate (SDS); sodium laurel sulfate; sodium octyl glycoside; lauryl-, myristyl-, linoleyl-, or stearyl-sulfobetaine; lauryl-, myristyl-, linoleyl- or stearyl-sarcosine; linoleyl-, myristyl-, or cetyl-betaine; lauroamidopropyl-, cocamidopropyl-, linoleamidopropyl-, myristamidopropyl-, palnidopropyl-, or isostearamidopropyl-betaine (e.g lauroamidopropyl); myristamidopropyl-, palmidopropyl-, or isostearamidopropyl-dimethylamine; sodium methyl cocoyl-, or disodium methyl oleyl-taurate; and the MONAQUATTM series (Mona Industries, Inc., Paterson, N.J.), polyethyl glycol, polypropyl glycol, and copolymers of ethylene and propylene glycol (e.g. Pluronics, PF68 etc). The amount of surfactant added is such that it reduces aggregation of the reconstituted protein and minimizes the formation of particulates after reconstitution. For example, the surfactant may be present in the pre-lyophilized formulation in an amount from about 0.001-0.5%, and preferably from about 0.005-0.05%.

In certain embodiments of the invention, a mixture of the lyoprotectant (such as sucrose or trehalose) and a bulking agent (e.g. mannitol or glycine) is used in the preparation of the prelyophilization formulation. The bulking agent may allow for the production of a uniform lyophilized cake without excessive pockets therein etc.

Other pharmaceutically acceptable carriers, excipients or stabilizers such as those described in Remington's Pharmaceutical Sciences 16th edition, Osol, A. Ed. (1980) may be included in the pre-lyophilized formulation (and/or the lyophilized formulation and/or the reconstituted formulation) provided that they do not adversely affect the desired characteristics of the formulation. Acceptable carriers, excipients or stabilizers are nontoxic to recipients at the dosages and concentrations employed and include; additional buffering agents; preservatives; co-solvents; antioxidants including ascorbic acid and methionine; chelating agents such as EDTA; metal complexes (e.g. Zn-protein complexes); biodegradable polymers such as polyesters; and/or salt-forming counterions such as sodium.

The pharmaceutical compositions and formulations described herein are preferably stable. A "stable" formulation/composition is one in which the antibody therein essentially retains its physical and chemical stability and integrity upon storage. Various analytical techniques for measuring protein stability are available in the art and are reviewed in Peptide and Protein Drug Delivery, 247-301, Vincent Lee Ed., Marcel Dekker, Inc., New York, N.Y., Pubs. (1991) and Jones, A. Adv. Drug Delivery Rev. 10: 29-90 (1993). Stability can be measured at a selected temperature for a selected time period.

The formulations to be used for in vivo administration must be sterile. This is readily accomplished by filtration through sterile filtration membranes, prior to, or following, lyophilization and reconstitution. Alternatively, sterility of the entire mixture may be accomplished by autoclaving the ingredients, except for protein, at about 120° C. for about 30 minutes, for example.

After the protein, lyoprotectant and other optional components are mixed together, the formulation is lyophilized. Many different freeze-dryers are available for this purpose such as Hull50® (Hull, USA) or GT20® (Leybold-Heraeus, Germany) freeze-dryers. Freeze-drying is accomplished by freezing the formulation and subsequently subliming ice from the frozen content at a temperature suitable for primary drying. Under this condition, the product temperature is below the eutectic point or the collapse temperature of the formulation. Typically, the shelf temperature for the primary drying will range from about -30 to 25° C. (provided the product remains frozen during primary drying) at a suitable pressure, ranging typically from about 50 to 250 mTorr. The formulation, size and type of the container holding the sample (e.g., glass vial) and the volume of liquid will mainly dictate the time required for drying, which can range from a few hours to several days (e.g. 40-60hrs). A secondary drying stage may be carried out at about 0-40° C., depending primarily on the type and size of container and the type of protein employed. However, it was found herein that a secondary drying step may not be necessary. For example, the shelf temperature throughout the entire water removal phase of lyophilization may be from about 15-30° C. (e.g., about 20° C.). The time and pressure required for secondary drying will be that which produces a suitable lyophilized cake, dependent, e.g., on the temperature and other parameters. The secondary drying time is dictated by the desired residual moisture level in the product and typically takes at least about 5 hours (e.g. 10-15 hours). The pressure may be the same as that employed during the primary drying step. Freeze-drying conditions can be varied depending on the formulation and vial size.

In some instances, it may be desirable to lyophilize the protein formulation in the container in which reconstitution of the protein is to be carried out in order to avoid a transfer step. The container in this instance may, for example, be a 3, 5, 10, 20, 50 or 100 cc vial. As a general proposition, lyophilization will result in a lyophilized formulation in which the moisture content thereof is less than about 5%, and preferably less than about 3%.

At the desired stage, typically when it is time to administer the protein to the patient, the lyophilized formulation may be reconstituted with a diluent such that the protein concentration in the reconstituted formulation is at least 50 mg/mL, for example from about 50 mg/mL to about 400 mg/mL, more preferably from about 80 mg/mL to about 300 mg/mL, and most preferably from about 90 mg/mL to about 150 mg/mL. Such high protein concentrations in the reconstituted formulation are considered to be particularly useful where subcutaneous delivery of the reconstituted formulation is intended. However, for other routes of administration, such as intravenous administration, lower concentrations of the protein in the reconstituted formulation may be desired (for example from about 5-50 mg/mL, or from about 10-40 mg/mL protein in the reconstituted formulation). In certain embodiments, the protein concentration in the reconstituted formulation is significantly higher than that in the pre-lyophilized formulation. For example, the protein concentration in the reconstituted formulation may be about 2-40 times, preferably 3-10 times and most preferably 3-6 times (e.g. at least three fold or at least four fold) that of the pre-lyophilized formulation.

Reconstitution generally takes place at a temperature of about 25° C. to ensure complete hydration, although other temperatures may be employed as desired. The time required for reconstitution will depend, e.g., on the type of diluent, amount of excipient(s) and protein. Exemplary diluents include sterile water, bacteriostatic water for injection (BWFI), a pH buffered solution (e.g. phosphate-buffered saline), sterile saline solution, Ringer's solution or dextrose solution. The diluent optionally contains a preservative. Exemplary preservatives have been described above, with aromatic alcohols such as benzyl or phenol alcohol being the preferred preservatives. The amount of preservative employed is determined by assessing different preservative concentrations for compatibility with the protein and preservative efficacy testing. For example, if the preservative is an aromatic alcohol (such as benzyl alcohol), it can be present in an amount from about 0.1-2.0% and preferably from about 0.5-1.5%, but most preferably about 1.0-1.2%. Preferably, the reconstituted formulation has less than 6000 particles per vial which are >10 µm size.

### Therapeutic Applications

Described herein is an isolated humanized monoclonal antibody for use for treating cancer in a subject in need thereof.

The subject (e.g., a human patient) in need of the treatment is diagnosed with, suspected of having, or at risk for cancer. Examples of the cancer include, but are not limited to, brain cancer, lung cancer, breast cancer, oral cancer, esophagus cancer, stomach cancer, liver cancer, bile duct cancer, pancreas cancer, colon cancer, kidney cancer, cervix cancer, ovary cancer and prostate cancer. In some embodiments, the cancer is brain cancer, lung cancer, breast cancer, ovarian cancer, prostate cancer, colon cancer, or pancreas cancer. In some preferred embodiments, the cancer is brain cancer or glioblastoma multiforme (GBM) cancer.

The antibody is an anti-SSEA-4 antibody.

The treatment results in reduction of tumor size, elimination of malignant cells, prevention of metastasis, prevention of relapse, reduction or killing of disseminated cancer, prolongation of survival and/or prolongation of time to tumor cancer progression.

In some embodiments, the treatment further comprises administering an additional therapy to said subject prior to, during or subsequent to said administering of the antibodies. In some embodiments, the additional therapy is treatment with a chemotherapeutic agent. In some embodiments, the additional therapy is radiation therapy.

The invention is particularly advantageous in treating and preventing early stage tumors, thereby preventing progression to the more advanced stages resulting in a reduction in the morbidity and mortality associated with advanced cancer. The invention is also advantageous in preventing the recurrence of a tumor or the regrowth of a tumor, for example, a dormant tumor that persists after removal of the primary tumor, or in reducing or preventing the occurrence of a tumor.

The invention as disclosed herein is useful for the treatment or prevention of a cancer, for example where a cancer is characterized by increased SSEA-4 expression. In some embodiments the cancer comprises a cancer stem cell. In some embodiments, the cancer is a pre-cancer, and/or a malignant cancer and/or a therapy resistant cancer. In some embodiments, the cancer is a brain cancer.

According to the invention, the cancer may be a solid tumor, e.g., such as, breast cancer, colorectal cancer, rectal cancer, lung cancer, renal cell cancer, a glioma (e.g., anaplastic astrocytoma, anaplastic oligoastrocytoma, anaplastic oligodendroglioma, glioblastoma multiforme (GBM)), kidney cancer, prostate cancer, liver cancer, pancreatic cancer, soft-tissue sarcoma, carcinoid carcinoma, head and neck cancer, melanoma, and ovarian cancer. In one embodiment, the cancer is a brain cancer or GBM. An effective amount of the pharmaceutical composition/formulation described above, containing at least one antibody described herein, can be administered to a subject (e.g., a human) in need of the treatment via a suitable route, such as intravenous administration, e.g., as a bolus or by continuous infusion over a period of time, by intramuscular, intraperitoneal, intracerebrospinal, subcutaneous, intraarticular, intrasynovial, intrathecal, oral, inhalation or topical routes. Commercially available nebulizers for liquid formulations, including jet nebulizers and ultrasonic nebulizers are useful for administration. Liquid formulations can be directly nebulized and lyophilized powder can be nebulized after reconstitution. Alternatively, the antibodies can be aerosolized using a fluorocarbon formulation and a metered dose inhaler, or inhaled as a lyophilized and milled powder.

The subject to be treated as described herein can be a mammal, more preferably a human. Mammals include, but are not limited to, farm animals, sport animals, pets, primates, horses, dogs, cats, mice and rats. A human subject who needs the treatment may be a human patient having, at risk for, or suspected of having cancer, which include, but not limited to, brain cancer, lung cancer, breast cancer, oral cancer, esophagus cancer, stomach cancer, liver cancer, bile duct cancer, pancreas cancer, colon cancer, kidney cancer, cervix cancer, ovary cancer and prostate cancer. A subject having cancer can be identified by routine medical examination.

"An effective amount" as used herein refers to the amount of each active agent required to confer therapeutic effect on the subject, either alone or in combination with one or more other active agents. Effective amounts vary, as recognized by those skilled in the art, depending on the particular condition being treated, the severity of the condition, the individual patient parameters including age, physical condition, size, gender and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons or for virtually any other reasons.

Empirical considerations, such as the half-life, generally will contribute to the determination of the dosage. For example, antibodies that are compatible with the human immune system, such as humanized antibodies or fully human antibodies, may be used to prolong half-life of the antibody and to prevent the antibody being attacked by the host's immune system. Frequency of administration may be determined and adjusted over the course of therapy, and is generally, but not necessarily, based on treatment and/or suppression and/or amelioration and/or delay of cancer. Alternatively, sustained continuous release formulations of the antibodies described herein may be appropriate. Various formulations and devices for achieving sustained release are known in the art.

In one example, dosages for an antibody as described herein may be determined empirically in individuals who have been given one or more administration(s) of the antibody. Individuals are given incremental dosages of the antibody. To assess efficacy of the antibody, an indicator of the disease (e.g., cancer) can be followed according to routine practice.

Generally, for administration of any of the antibodies described herein, an initial candidate dosage can be about 2 mg/kg. For the purpose of the present disclosure, a typical daily dosage might range from about any of 0.1 µg/kg to 3 µg/kg to 30 µg/kg to 300 µg/kg to 3 mg/kg, to 30 mg/kg to 100 mg/kg or more, depending on the factors mentioned above. For repeated administrations over several days or longer, depending on the condition, the treatment is sustained until a desired suppression of symptoms occurs or until sufficient therapeutic levels are achieved to alleviate cancer, or a symptom thereof. An exemplary dosing regimen comprises administering an initial dose of about 2 mg/kg, followed by a weekly maintenance dose of about 1 mg/kg of the antibody, or followed by a maintenance dose of about 1 mg/kg every other week. However, other dosage regimens may be useful, depending on the pattern of pharmacokinetic decay that the practitioner wishes to achieve. For example, dosing from one-four times a week is contemplated. In some embodiments, dosing ranging from about 3 µg/mg to about 2 mg/kg (such as about 3 µg/mg, about 10 µg/mg, about 30 µg/mg, about 100 µg/mg, about 300 µg/mg, about 1 mg/kg, and about 2 mg/kg) may be used. In some embodiments, dosing frequency is once every week, every 2 weeks, every 4 weeks, every 5 weeks, every 6 weeks, every 7 weeks, every 8 weeks, every 9 weeks, or every 10 weeks; or once every month, every 2 months, or every 3 months, or longer. The progress of this therapy is easily monitored by conventional techniques and assays. The dosing regimen (including the antibody used) can vary over time.

For the purpose of the present disclosure, the appropriate dosage of an antibody described herein will depend on the specific antibody (or compositions thereof) employed, the type and severity of the cancer, whether the antibody is administered for preventive or therapeutic purposes, previous therapy, the patient's clinical history and response to the antibody, and the discretion of the attending physician. The administration of the antibodies described herein may be essentially continuous over a preselected period of time or may be in a series of spaced dose, e.g., either before, during, or after developing cancer.

As used herein, the term "treating" refers to the application or administration of a composition including one or more active agents to a subject, who has cancer, a symptom of cancer, or a predisposition toward cancer, with the purpose to cure, heal, alleviate, relieve, alter, remedy, ameliorate, improve, or affect cancer, the symptom of cancer, or the predisposition toward cancer.

Alleviating cancer includes delaying the development or progression of cancer, or reducing cancer severity. Alleviating cancer does not necessarily require curative results. As used therein, "delaying" the development of cancer means to defer, hinder, slow, retard, stabilize, and/or postpone progression of cancer. This delay can be of varying lengths of time, depending on the history of cancer and/or individuals being treated. A method that "delays" or alleviates the development of cancer, or delays the onset of cancer, is a method that reduces probability (the risk) of developing one or more symptoms of cancer in a given time frame and/or reduces extent of the symptoms in a given time frame, when compared to not using the method. Such comparisons are typically based on clinical studies, using a number of subjects sufficient to give a statistically significant result.

"Development" or "progression" of cancer means initial manifestations and/or ensuing progression of cancer. Development of cancer can be detectable and assessed using standard clinical techniques as well known in the art. However, development also refers to progression that may be undetectable. For purpose of this disclosure, development or progression refers to the biological course of the symptoms. "Development" includes occurrence, recurrence, and onset. As used herein "onset" or "occurrence" of cancer includes initial onset and/or recurrence.

Conventional methods, known to those of ordinary skill in the art of medicine, can be used to administer the pharmaceutical composition to the subject, depending upon the type of disease to be treated or the site of the disease. This composition can also be administered via other conventional routes, e.g., administered orally, parenterally, by inhalation spray, topically, rectally, nasally, buccally, vaginally or via an implanted reservoir. The term "parenteral" as used herein includes subcutaneous, intracutaneous, intravenous, intramuscular, intraarticular, intraarterial, intrasynovial, intrasternal, intrathecal, intralesional, and intracranial injection or infusion techniques. In addition, it can be administered to the subject via injectable depot routes of administration such as using 1-, 3-, or 6-month depot injectable or biodegradable materials and methods.

Injectable compositions may contain various carriers such as vegetable oils, dimethylactamide, dimethyformamide, ethyl lactate, ethyl carbonate, isopropyl myristate, ethanol, and polyols (glycerol, propylene glycol, liquid polyethylene glycol, and the like). For intravenous injection, water soluble antibodies can be administered by the drip method, whereby a pharmaceutical formulation containing the antibody and a physiologically acceptable excipients is infused. Physiologically acceptable excipients may include, for example, 5% dextrose, 0.9% saline, Ringer's solution or other suitable excipients. Intramuscular preparations, e.g., a sterile formulation of a suitable soluble salt form of the antibody, can be dissolved and administered in a pharmaceutical excipient such as Water-for-Injection, 0.9% saline, or 5% glucose solution.

Without further elaboration, it is believed that one skilled in the art can, based on the above description, utilize the present invention to its fullest extent. Examples 1-14 hereunder are background examples. The present invention as defined in the claims is exemplified in Examples 15 and 16.

### EXAMPLE 1: Flow Cytometric Analysis of Glycan Epitopes on GBM Cell Lines

We analyzed the expression levels of various glycan epitopes by flow cytometry in four human GBM cell lines: G5T, LN-18, U-138 and U-251. The glycan epitopes examined include O-linked glycans (Tn, sTn, TF), Lewis antigens (Le^{x}, Le^{y} and sLe^{x}), complex gangliosides [GM2, GM1, GD1a, GD3, GD2, GTlb and A2B5 (c-series gangliosides)], and globo-series GSLs (SSEA-3, SSEA-4 and Globo H; Fig. 1A). Our results showed that most of these four GBM cell lines expressed high levels of Tn, TF, Le^{x}, and Le^{y}, a low level of sLe^{x}, and no sTn (Table 7). In addition, these four GBM cell lines were positive for all the gangliosides we examined. Regarding the expression levels of globo-series GSLs, U-251 showed a weak staining for an anti-SSEA-4 antibody, MC813-70, and G5T, U-138 and LN-18 displayed a high staining intensity for MC813-70 (Fig. 1B). Positive staining for an anti-SSEA-3 antibody, MC631, was only observed on G5T among these four cell lines, and none showed positive staining for an anti-Globo H antibody, VK9 (Table 7). We further looked into the expression patterns of globo-series GSLs in more GBM cell lines, and found that of the 17 GBM cell lines, nine showed strong MC813-70 staining signal (SSEA-4^{hi}); three were weakly stained (SSEA-4^{lo}), and five were not stained by MC813-70 (Fig. 1*A*). Nine out of 17 cell lines were positive for MC631 staining and six were positive for VK9 staining. SVG p12, an immortalized human fetal glia cell, showed a very weak MC813-70 staining signal, but no MC631 or VK-9 staining signal (Fig. 1). These results indicated that most of the GBM cell lines examined were positively stained by anti-SSEA-4 antibody using MC813-70 as an example.

### EXAMPLE 2: Verification of SSEA-4 expression in GBM Cancer Cells

To exclude the possibility that anti-SSEA-4 antibody may bind to the extended core 1 O-glycan on glycoproteins in GBM cells, we treated DBTRG cells with methanol to remove lipids before staining with an anti-SSEA-4 antibody, MC813-70. Upon methanol treatment, the immunoreactivity of MC813-70 disappeared, as analyzed by flow cytometry and immunofluorescence microscopy, suggesting that the immunoreactivity of MC813-70 was toward glycolipids, not glycoproteins. To confirm the existence of SSEA-4 epitope on GBM cell surface, we further performed the MC631 staining on a2,3-sialidase-treated DBTRG cells, and the result showed that when treating with a2,3-sialidase, the cells became MC813-70 negative and MC631 positive, supporting that the GBM cells did express SSEA-4.

To further verify the expression of SSEA-4, we next purified the gangliosides (the glycolipids with sialic acids) using anion-exchange chromatography, developed the purified gangliosides on HPTLC plates, and visualized by orcinol-H₂SO₄ stain or immunoblotting. The purified gangliosides from three different GBM cell lines exhibited similar patterns, with abundant GM3, GM2, Neu5Ac-(n)Lc4/Gg4Cer and Neu5Ac2-(n)Lc4/Gg4Cer. Consistent with the results of flow cytometric analysis, MC813-70 recognized two gangliosides (due to a different chain length of fatty acids) on TLC from DBTRG and D54MG, but not GBM 8901 cells (Fig. 3A). The positions of the immuno-reactive double bands in GBM gangliosides were the same as in the gangliosides purified from 2102Ep cells (Fig. 3A, lane 4), embryonal carcinoma cells known to express a high level of SSEA-4 glycolipid (Kannagi R, et al. (1983) Stage-specific embryonic antigens (SSEA-3 and -4) are epitopes of a unique globo-series ganglioside isolated from human teratocarcinoma cells. EMBO J 2(12):2355-2361). A double-band developed at a shorter distance than MC813-70 positive glycolipid was detected by MC813-70 in YAC-1 cells (Fig. 3A, lane 5), in which GMlb is a major ganglioside (Zarei M, Muthing J, Peter-Katalinic J, & Bindila L (2010) Separation and identification of GMlb pathway Neu5Ac- and Neu5Gc gangliosides by on-line nanoHPLC-QToF MS and tandem MS: toward glycolipidomics screening of animal cell lines. Glycobiology 20(1): 118-126), supporting that MC813-70 harbors a weak cross-reactivity toward GMlb. Immunoblotting with MC631 revealed that it could also recognize MC813-70 positive glycolipid with a lower affinity than MC813-70 did. To examine the number of sialic acids on MC813-70 positive glycolipids, we eluted gangliosides in different salt conditions and performed immunoblotting with MC813-70. The result supported that MC813-70 reactive gangliosides were monosialylated as the two bands appeared at the fraction eluted in low salt condition. We next used sialidases to elucidate the linkage of the sialic acid on this MC813-70-reactive monosialoganglioside. The gangliosides developed on TLC plate were treated with a2,3-sialidase or the sialidase that cleaves all linkages of sialic acids, and blotted with MC813-70 and MC631. The results showed that the immunoreactivity of MC813-70 disappeared after sialidase treatment, while MC631 could detect strong signals at the positions resembled MC813-70 reactive doublets, indicating the presence of an α2,3-linked sialic acid.

We also analyzed the gangliosides from DBTRG cells by MALDI-MS profiling (Fig. 3B). The spectra were dominated by several major peaks that occurred in signal clusters due to the expected heterogeneity of the ceramide (Cer) portions. Based on the *m*/*z* values of major molecular ions, as fitted to permethylation of hexose (Hex), *N*-*acetyl*hexosamine (HexNAc), or NeuAc residues, in combination with sphingosine and fatty acyl chains, the respective gangliosides profiles were assigned. Consistent with the HPTLC results, the MS profiling revealed that the major species of gangliosides in DBTRG cells were GM3, GM2, Neu5Ac-(n)Lc4/Gg4Cer, and Neu5Ac2-(n)Lc4/Gg4Cer. The signal with Neu5Ac-Hex4-HexNAc-Cer (*m*/*z* = 2025.2) that represented SSEA-4, although with low intensity, was also detected, reflecting the existence of SSEA-4 in DBTRG cells. These data support that the MC813-70 reactive ganglioside was SSEA-4, and despite of being a minor constituent of total gangliosides, SSEA-4 was expressed in GBM cells.

### EXAMPLE 3: Expression of SSEA-4 in GBM Tissues

SSEA-4 is a widely used marker for stem cells, but the information about the expression of SSEA-4 in GBM tissues as well as normal brain tissues is not known. To understand if SSEA-4 is overexpressed in clinical GBM specimens, in addition to GBM cell lines, we analyzed the expression of SSEA-4 among astrocytomas from grade I to IV and normal brain tissues by immunohistochemistry on human tissue microarrays (Fig. 4). We found that 38 out of 55 GBM tissue specimens (69%) were positive for MC813-70 staining, and around half of GBM specimens were intensely stained (≥2+). As shown in the positive specimens, SSEA-4 was situated on the plasma membrane of GBM cells. Furthermore, around 55% of low-grade astrocytoma specimens were weakly stained (scored as 1+) by MC813-70, and the scores of SSEA-4 intensity was positively correlated with the grades of astrocytomas. On the contrary, most normal brain tissues were SSEA-4 negative. The result showed SSEA-4 is highly expressed in tumors, in particular, in GBM tumors.

### EXAMPLE 4: Anti-SSEA-4 Mediates CDC against GBM Cell Lines

To test if targeting SSEA-4 would trigger complement-dependent cytotoxicity (CDC) in GBM cells, GBM cell lines were treated with MC813-70 and rabbit complement, and the degree of CDC was evaluated by detecting the level of released lactate dehydrogenase caused by cell death. Fig. 5 showed that in the presence of complement, mAb MC813-70 remarkably reduced the number of viable GBM cells. We observed a significant CDC in SSEA-4hi GBM cell lines: 71.7% cytotoxicity of DBTRG, 46.6% of LN-229, 67% of G5T, and 65.4% of LN-18 cells. MC813-70-mediated CDC did not kill two GBM cell lines, Hs683 and U87, which expressed low or no SSEA-4. Therefore, the level of MC813-70 mediated CDC positively correlated with the expression level of SSEA-4 in each GBM cell line.

### EXAMPLE 5: Anti-SSEA-4 Suppresses Brain Tumor Growth In Vivo

To check if MC813-70 was able to suppress GBM tumor growth in vivo, MC813-70 was administered to the nude mice injected with DBTRG cells subcutaneously, when the tumors grew to palpable bumps (15-30 mm3at day 11 post-injection). MC813-70 (200 µg) was given intraperitoneally to each mouse every four days for a total of three times, with an irrelevant mouse IgG3 (isotype control) injected in parallel for comparison. The experiment revealed that the administration of MC813-70 could inhibit DBTRG tumor growth (Fig. 6). The growth of DBTRG tumors were completely suppressed in two of three mice treated with MC813-70, and the third mice developed tumor after the cease of antibody treatment. Comparing to the mice receiving MC813-70 treatment, DBTRG tumor grew aggressively (with the tumor volume of 184 mm3 in average at day 31) in the control group injected with mouse IgG3. These data demonstrated that MC813-70 was able to inhibit the growth of SSEA-4-expressing GBM tumors, possibly through CDC and antibody-dependent cell-mediated cytotoxicity (ADCC) in vivo.

### EXAMPLE 6: Expression of Globo H, SSEA3 and SSEA-4 on Various Cancer Cell Lines

SSEA-4 has been reported to be expressed on renal carcinoma (Saito S, et al. (1997) Expression of globo-series gangliosides in human renal cell carcinoma. Jpn J Cancer Res 88(7):652-659), basaloid lung cancer (Gottschling S, et al. (2013) Stage-specific embryonic antigen-4 is expressed in basaloid lung cancer and associated with poor prognosis. Eur Respir J 41(3):656-663), epithelial ovarian carcinoma (Ye F, Li Y, Hu Y, Zhou C, & Chen H (2010) Stage-specific embryonic antigen 4 expression in epithelial ovarian carcinoma. Int J Gynecol Cancer 20(6):958-964), breast cancer (Huang YL, et al. (2013) Carbohydrate-based vaccines with a glycolipid adjuvant for breast cancer. Proc Natl Acad Sci U S A 110(7):2517-2522), and oral cancer (Noto Z, et al. (2013) CD44 and SSEA-4 positive cells in an oral cancer cell line HSC-4 possess cancer stem-like cell characteristics. Oral Oncol). Here, we analyzed the expression levels of SSEA-3, Globo H and SSEA-4 on various cancer cell lines by flow cytometry. As shown in Table 7, we have examined a total of 134 cancer cell lines (17 brain cancer cell lines, 20 lung cancer cell lines, 23 breast cancer cell lines, 13 oral cancer cell lines, 2 esophageal cancer cell lines, 6 gastric cancer cell lines, 10 liver cancer cell lines, 5 bile duct cancer cell lines, 8 pancreatic cancer cell lines, 7 colon cancer cell lines, 6 renal cancer cell lines, 4 cervical cancer cell lines, 9 ovarian cancer cell lines and 4 prostate cancer cell lines).

**Table 7. Expression of Globo H, SSEA-3 and SSEA-4 on various cancer cell lines.**

| Brain Cancer Cell Line | SSEA-3 | SSEA-4 | Globo H |
|---|---|---|---|
| A172 | - | - | - |
| D54MG | + | + | + |
| DBTRG | - | + | - |
| G5T | + | + | - |
| G9T | + | + | + |
| GBM 8401 | - | - | - |
| GBM 8901 | - | - | - |
| Hs683 | - | + | - |
| LN-18 | + | + | + |
| LN-229 | + | + | + |
| SF126 | - | + | + |
| SNB75 | + | + | - |
| T95G | - | - | - |
| U-138 MG | + | + | - |
| U-251 MG | + | + | - |
| U-373 MG | + | + | + |
| U-87 MG | - | - | - |

| Lung cancer cell line | SSEA-3 | SSEA-4 | Globo H |
|---|---|---|---|
| A549 | - | + | + |
| Calu-3 | - | + | - |
| CL1 | - | + | - |
| CL1-0 | - | + | + |
| CL1-5 | - | + | + |
| CL2 | - | - | + |
| CL3 | - | - | - |
| H1299 | - | - | - |
| H1355 | - | + | + |
| H157 | + | + | + |
| H441 | - | + | + |
| H460 | - | - | - |
| H480 | + | + | + |
| H520 | - | - | + |
| H661 | + | + | + |
| H928 | + | + | + |
| NuLi-1 | + | + | + |
| PC-13 | - | - | + |
| PC-14 | - | - | - |
| PC-9 | - | + | - |

| Breast cancer cell line | SSEA-3 | SSEA-4 | Globo H |
|---|---|---|---|
| Au565 | - | - | - |
| BT-20 | - | - | - |
| BT-474 | - | - | - |
| BT-483 | - | - | - |
| BT-549 | - | + | - |
| DU4475 | - | + | + |
| HBL-100 | - | + | + |
| HBL-435 | - | - | - |
| HCC1395 | + | + | + |
| HCC1599 | - | + | + |
| HCC1806 | + | + | + |
| HCC1937 | - | + | - |
| HCC38 | - | + | + |
| Hs578T | + | + | + |
| MCF-7 | + | + | + |
| MDA-MB-157 | + | + | + |
| MDA-MB-231 | + | + | + |
| MDA-MB-361 | - | + | + |
| MDA-MB-453 | - | + | + |
| MDA-MB-468 | - | + | - |
| SK-BR-3 | - | - | + |
| T47D | + | + | + |
| ZR75 | - | + | + |

| Oral cancer cell line | SSEA-3 | SSEA-4 | Globo H |
|---|---|---|---|
| Ca922 | - | + | + |
| Cal27 | - | + | + |
| HSC3 | - | + | + |
| OC3 | - | - | + |
| OECM1 | - | - | + |
| SAS | - | - | + |
| SCC25 | - | - | + |
| SCC4 | + | + | + |
| Tu-183 | - | - | + |
| Tw1.5 | - | + | + |
| Tw2.6 | - | + | + |
| UMSCC-1 | + | + | + |
| YD-15 | + | + | + |

| Esophageal cancer cell line | SSEA-3 | SSEA-4 | Globo H |
|---|---|---|---|
| CE81T | - | - | + |
| KYSE70 | - | + | + |

| Gastric cancer cell line | SSEA-3 | SSEA-4 | Globo H |
|---|---|---|---|
| AGS | - | - | + |
| AZ521 | + | + | + |
| KATO III | + | + | + |
| NCI-N87 | - | - | + |
| SCM-1 | + | + | + |
| SNU-1 | - | + | + |

| Liver cancer cell line | SSEA-3 | SSEA-4 | Globo H |
|---|---|---|---|
| 59T | + | + | + |
| Changliver | - | - | + |
| HA22T | - | - | + |
| Hep3b | + | + | + |
| HepG2 | - | + | + |
| Huh-7 | - | + | + |
| J5 | - | - | + |
| Mahlavu | - | - | + |
| NTU-BL | + | + | + |
| SK-HEP-1 | + | + | + |

| Bile duct cancer cell line | SSEA-3 | SSEA-4 | Globo H |
|---|---|---|---|
| HuccT1 | + | + | + |
| SNU-1079 | - | - | - |
| SNU-1196 | - | - | + |
| SNU-245 | - | + | + |
| SNU-308 | - | - | - |

| Pancreatic cancer cell line | SSEA-3 | SSEA-4 | Globo H |
|---|---|---|---|
| AsPC1 | - | + | - |
| BxPC3 | - | + | + |
| HPAC | - | + | + |
| KP-4 | + | + | + |
| MIA PaCa-2 | + | + | + |
| Panc0203 | - | + | + |
| PANC1 | - | + | - |
| PL45 | + | + | + |

| Colon cancer cell line | SSEA-3 | SSEA-4 | Globo H |
|---|---|---|---|
| CX-1 | - | + | + |
| DLD-1 | - | - | + |
| H3347 | - | + | + |
| HCT1116 | - | + | - |
| HT-29 | - | - | + |
| SW480 | - | + | + |
| SW620 | - | + | + |

| Renal cancer cell line | SSEA-3 | SSEA-4 | Globo H |
|---|---|---|---|
| 769-P | + | + | + |
| A498 | - | + | - |
| A704 | - | - | + |
| ACHN | + | + | + |
| Caki-1 | + | + | + |
| Caki-2 | + | + | + |

| Cervical cancer cell line | SSEA-3 | SSEA-4 | Globo H |
|---|---|---|---|
| Hela | + | + | - |
| Hela 229 | + | + | - |
| Hela S3 | - | - | - |
| ME-180 | - | + | + |

| Ovarian cancer cell line | SSEA-3 | SSEA-4 | Globo H |
|---|---|---|---|
| C33A | - | + | - |
| CAOV3 | - | + | - |
| ES-2 | - | + | + |
| NUGCC | + | + | + |
| OVCAR-3 | - | + | + |
| SiHa | - | - | - |
| SKOV3 | - | + | - |
| TOV-112D | - | + | + |
| TOV-21G | + | + | + |

| Prostate cancer cell lines | SSEA-3 | SSEA-4 | Globo H |
|---|---|---|---|
| 22Rr1 | - | + | + |
| Du145 | - | + | - |
| hTERT-HPNE | + | + | - |
| PC-3 | - | + | - |

**Table 8.Summary of expression of globo-series GSLs on cancer cell lines.**

| Tumor origin | SSEA-4+ | SSEA-3+ | Globo H+ | SSEA4+ or SSEA3+ or Globo H+ | SSEA-4+ SSEA-3+ | SSEA-4+ Globo H+ | SSEA-4+ SSEA-3+ Globo H+ |
|---|---|---|---|---|---|---|---|
| Brain | 12/17 | 9/17 | 6/17 | 12/17 | 9/17 | 6/17 | 5/17 |
| Lung | 13/20 | 5/20 | 13/20 | 16/20 | 5/20 | 10/20 | 5/20 |
| Breast | 17/23 | 6/23 | 14/23 | 18/23 | 6/23 | 13/23 | 6/23 |
| Mouth | 8/13 | 2/13 | 11/13 | 12/13 | 2/13 | 7/13 | 2/13 |
| Esophagus | 1/2 | 0/2 | 2/2 | 2/2 | 0/2 | 1/2 | 0/2 |
| Stomach | 4/6 | 3/6 | 6/6 | 6/6 | 3/6 | 4/6 | 3/6 |
| Liver | 6/10 | 4/10 | 9/10 | 9/10 | 4/10 | 6/10 | 4/10 |
| Bile duct | 2/5 | 1/5 | 3/5 | 3/5 | 1/5 | 2/5 | 1/5 |
| Pancreas | 8/8 | 3/8 | 6/8 | 8/8 | 3/8 | 6/8 | 3/8 |
| Colon | 5/7 | 0/7 | 6/7 | 7/7 | 0/7 | 4/7 | 0/7 |
| Kidney | 5/6 | 0/6 | 5/6 | 6/6 | 0/6 | 4/6 | 0/6 |
| Cervix | 3/4 | 2/4 | 1/4 | 3/4 | 2/4 | 1/4 | 0/4 |
| Ovary | 8/9 | 2/9 | 5/9 | 8/9 | 2/9 | 5/9 | 2/9 |
| Prostate | 4/4 | 1/4 | 1/4 | 4/4 | 1/4 | 1/4 | 0/4 |

Expression of globo-series GSLs was determined by flow cytometry. Those cell lines in which over 15% of total cells are positive in flow cytometry are labelled positive.

We found that SSEA-4 was expressed in every type of cancer cell line (96 of 134 cancer cell lines). Globo H was also expressed in many of cancer cell lines (88 of 134), preferentially in lung, breast, pancreas, colon, stomach, mouth, liver, kidney cancer cell lines. We also observed that many of cancer cell lines (70 of 134) expressed SSEA-4 and Globo H simultaneously. On the other hand, the expression of SSEA-3 always followed a high expression of SSEA-4, indicating that SSEA-4 and Globo H were the major globo-series GSLs on the cancer cells.

To validate the identity of SSEA-4, we purified the gangliosides from nine MC813-70-positive cell lines as well as TF1a, a MC813-70-negative leukemia cell line, and performed immunostaining on HPTLC plates. As expected, SSEA-4 was detected in these nine cancer cell lines but not TF1a, and the intensity was well correlated with the geometric mean fluorescence intensity as examined by flow cytometry. These results revealed that SSEA-4 could be expressed in a variety of cancer cell lines and may serve as a potential target for multiple types of cancers.

### EXAMPLE 7: Expression of Glycan-Related Molecules on Cancer Cells and Cancer Stem-Like Cells

For cancer stem-like cells, 8 glioblastoma cell lines were collected from ATCC and were maintained in the ultra-low attachment dish (Corning) with NSM consisting of neurobasal media (Invitrogen), B27 supplement (invitrogen), and human recombinant bFGF and EGF (20 ng/ml each; Upstate). The media were refreshed every 3 days and all cells were cultured in 5% CO2 and humidified atmosphere at 37°C.

The expression profiles of various markers on LN18, U138, U251, DBTRG and G5T cell lines and their spheres were examined. A total of 22 markers were examined including 17 glycans (GM3, GM2, GM1, GD1a, GD3, GD2, GT1b, A2B5, LeX, sLeX, LeY, SSEA-3, SSEA-4, Globo H, TF, Tn and sTn), and 5 glycoproteins (CD44, CD24, CD45, CD90, CD133). Anti-Globo H monoclonal antibody was generated in house. Other antibodies specific to the markers were purchased from vendors listed below. CD 133/1 and CD 133/2 were antibodies against different epitopes of CD133 glycoprotein.

| Antibody | Vendor |
|---|---|
| GM3 | seikagaku |
| GM2 | Merck Calbiochem |
| GM1 | Merck Calbiochem |
| GDla | Millipore |
| GDla | Millipore |
| GD3 | BD |
| GD2 | BD |
| GT1b | Millipore |
| A2B5 | Millipore |
| LeX | BD |
| sLeX | BD |
| LeY | Abcam |
| SSEA-3 | eBioscience |
| SSEA-4 | eBioscience |
| Globo H | Purified in Wong's Lab |
| TF | Thermo Scientific |
| Tn | DakoCytomation |
| sTn | Abnova |
| CD44 | eBioscience |
| CD24 | eBioscience |
| CD90 | BD |
| CD133/1 | Miltenyi Biotec |
| CD133/2 | Miltenyi Biotec |

Cells (1×10⁵) were resuspended in 50µL FACS buffer (1% BSA/PBS solution) containing various concentration antibodies and incubated on ice for 30 min. After being washed twice with FACS buffer, if necessary, cells were incubated with Alexa488-labeled goat anti-mouse or FITC-anti-rabbit antibody (1:100; Jackson ImmunoResearch) for 30 min on ice before analysis on a FACSCalibur system (BD Biosciences).

Among the gangliosides expression patterns, we found that GD2 highly predominated and consistently expressed in 5 GBM neurosphere cells. However, in the neolactoseries pathway, LeX, a well-known marker for pluripotent stem cells, did not show any significant difference between these groups. Besides, GM2, GM1, GD1a, GT1b, A2B5, Tf, Tn, SSEA3, SSEA4, CD24, CD44 and CD90 were highly expressed in 5 GBM cells. Interestingly, in the globoseries pathway, SSEA4 abundantly expressed in parental cells instead of the neurosphere cells, indicating that SSEA4 may serve as the therapeutic targets of GBM cells.

**Table 9: Expression of Glycans and Glycoproteins on Cancer Cells and Cancer Stem-Like Cells**

| Antibody | LN18 | | U138 | | U251 | | DBTRG | | G5T | |
|---|---|---|---|---|---|---|---|---|---|---|
| | parental | sphere | parental | sphere | parental | sphere | parental | sphere | parental | sphere |
| GM3 | ++ | + | ++ | + | | | N.D. | N.D. | | |
| GM2 | +++ | +++ | ++++ | ++++ | ++++ | +++ | N.D. | N.D. | ++ | ++ |
| GM1 | +++ | +++ | ++++ | ++++ | ++++ | +++ | N.D. | N.D. | ++ | ++ |
| GD1a | ++++ | ++++ | ++++ | ++++ | ++++ | ++++ | N.D. | N.D. | + | +++ |
| GD3 | | + | N.D. | N.D. | N.D. | N.D. | | ++ | | + |
| GD2 | + | +++ | + | ++ | | +++ | - | +++ | + | +++ |
| GTlb | ++++ | ++++ | ++++ | ++++ | ++ | ++ | N.D. | N.D. | + | + |
| A2B5 | ++ | ++ | ++ | ++ | + | + | N.D. | N.D. | + | + |
| LeX | | | | + | +++ | ++ | N.D. | N.D. | +++ | +++ |
| sLeX | | | | | ++ | | N.D. | N.D. | | |
| LeY | ++ | + | | + | ++ | +++ | N.D. | N.D. | | ++ |
| SSEA3 | ++ | + | ++ | | | | | | ++++ | +++ |
| SSEA4 | ++++ | + | ++++ | + | +++ | | ++++ | ++ | ++++ | ++++ |
| Globo H | + | | | | | | N.D. | N.D. | | |
| TF | +++ | + | ++ | ++ | + | +++ | N.D. | N.D. | | + |
| Tn | +++ | ++ | + | ++ | ++ | + | N.D. | N.D. | + | + |
| sTn | | | + | ++ | | | N.D. | N.D. | | |
| CD44 | ++++ | ++++ | ++++ | ++++ | ++++ | ++++ | N.D. | N.D. | ++++ | ++++ |
| CD24 | ++ | + | + | | + | | +++ | + | ++++ | + |
| CD90 | ++++ | ++++ | ++++ | ++++ | ++++ | ++++ | N.D. | N.D. | ++++ | ++++ |
| CD133/1 | | | | | - | - | - | - | | |
| CD133/2 | | | | | N.D. | N.D. | N.D. | N.D. | | |

### EXAMPLE 8: Enrichment of GBM stem cells by GD2 plus SSEA-4 and CD133

In order to understand whether the combination of GD2 plus SSEA4 and CD133 could more specifically determine the population of GBM stem cells, we compare the self-renewal ability and tumorigenicity of GD2⁺SSEA4⁺CD133⁺ population to the other populations.

Cells from GBM tumor were dissociated into single cell suspensions and labeled with anti-GD2 antibody (BD), anti-SSEA4 (Biolegend), anti-CD133 (Miltenyi Biotec). These labeled cells were then physically sorted using the FACS Aria II flow cytometer (BD). For detection of self-renewal ability, GBM tumor cells were dissociated, stained and plated in 96-well plates. The numbers of neurospheres whose diameter is over 100 um were calculated. For tumorigenicity, various number of cells, including 10,000-10 cells, were subcutaneously or intracranially inject into the immunodeficient mice. It is observed that the onset of tumors will be significantly faster in the GD2⁺SSEA4⁺CD133⁺ population compared to the other populations.

### EXAMPLE 9: Generation of Anti-Globo H Monoclonal Antibodies

Hybridoma methodology was employed for the development of mAbs specific to Globo H. Female BALB/c mice, aged 6-8 weeks old, were immunized three times subcutaneously with the Globo H vaccine. Three immunizations were given at 2-wk intervals. Each vaccination contained 2µg of Globo H. All of the sera were obtained by centrifugation at 4,000 × g for 10 min. The serologic responses were analyzed by glycan microarray. A final boost was given intraperitoneally with 2 µg of Globo H, and 3 days later, the spleen cells from immunized mice were used for generating hybridomas.

Hybridoma cells secreting antibodies with the desired antigen-binding activities were screened as follows. Microtiter plates were coated by incubating with 4 µg/mL of neutravidin in carbonate buffer, 0.1M, pH 9.6, overnight at 4 C. The wells were blocked with 1% BSA in PBS, pH=7.3 for 1 hour and incubated with 4 µg/mL GloboH-biotin for 1hour. The antisera were at various dilutions for 1 hour at 37°C. After washing, the ligand-bound antibodies were detected by HRP-conjugated goat anti-mouse IgG, Fcγ fragment specific (Jackson ImmunoResearch) at 1: 10,000 and incubated for 1 hour at 37°C, followed by incubation with TMB substrate. The OD was determined at 450 nm. Positive clones were selected for further characterization. Two exemplary clones, mAb 273 and 651, were identified in this study as specifically binding to Globo H. For mouse monoclonal isotyping, the IsoQuick Strips and Kits was used (sigma, 19535). Add hybridoma medium to the reaction vial. Insert the strip into the sample making sure the strips are upright. The sample will travel up the strip. Allow the strip to develop for 5 minutes before making final interpretations.

The V_{H} and V_{L} gene segments of the mAbs 273 and 651 were amplified by PCR from the hybridoma clone secreting the antibody. The gene segments thus obtained were sequenced to determine the V_{H} and V_{L} sequences of mAb 273 and 651, which are shown in Tables 1 and 2.

### EXAMPLE 10: Generation of Anti-SSEA-4 Monoclonal Antibodies

Hybridoma methodology was employed for the development of mAbs specific to SSEA-4. Female BALB/c mice, aged 6-8 weeks old, were immunized three times subcutaneously with the SSEA-4 vaccine. Three immunizations were given at 2-wk intervals. Each vaccination contained 2µg of SSEA-4. All of the sera were obtained by centrifugation at 4,000 × g for 10 min. The serologic responses were analyzed by glycan microarray. A final boost was given intraperitoneally with 2 µg of SSEA-4, and 3 days later, the spleen cells from immunized mice were used for generating hybridomas.

Hybridoma cells secreting antibodies with the desired antigen-binding activities were screened as follows. Microtiter plates were coated by incubating with 4 µg/mL of neutravidin in carbonate buffer, 0.1M, pH 9.6, overnight at 4°C. The wells were blocked with 1% BSA in PBS, pH=7.3 for 1 hour and incubated with 4 µg/mL SSEA-4-biotin for 1hour. The antisera were at various dilutions for 1 hour at 37°C. After washing, the ligand-bound antibodies were detected by HRP-conjugated goat anti-mouse IgG or IgM antibody (Jackson ImmunoResearch) at 1: 10,000 and incubated for 1 hour at 37°C, followed by incubation with TMB substrate. The OD was determined at 450 nm. Positive clones were selected for further characterization. Three exemplary clones 45, 46 and 48, were identified in this study as specifically binding to SSEA-4. For mouse monoclonal isotyping, the IsoQuick Strips and Kits was used (sigma, 19535). Add hybridoma medium to the reaction vial. Insert the strip into the sample making sure the strips are upright. The sample will travel up the strip. Allow the strip to develop for 5 minutes before making final interpretations.

The V_{H} and V_{L} gene segments of the mAbs 45, 46 and 48 were amplified by PCR from the hybridoma clone secreting the antibody. The gene segments thus obtained were sequenced to determine the V_{H} and V_{L} sequences of mAbs 45, 46 and 48, which are shown in Tables 3-5.

### EXAMPLE 11: Generations of Chimeric Antibodies

The V_{H} and V_{L} gene segments of the mAb 273 and 651 were amplified by PCR from the hybridoma clone secreting the antibody. The gene segments thus obtained were sequenced to determine the V_{H} and V_{L} sequences of mAb 273 and 651, which are shown in Tables 1 and 2. The heavy chain and light chain variable region were cloned to human IgG1 antibody expression vector show as Fig.9. VH was using enzyme site *BsiWI* and *ApaI,* and VL was using enzyme site *BsPEI* and *NheI.* Vectors were transiently transfected into either 293F or CHO-S cells. Recombinant chimeric Ab was purified and further study for binding assay and complement-dependent tumor cell lysis assay.

The V_{H} and V_{L} gene segments of the mAb 46 and 48 were amplified by PCR from the hybridoma clone secreting the antibody. The gene segments thus obtained were sequenced to determine the V_{H} and V_{L} sequences of mAb 46 and 48, which are shown in Tables 5 and 4. The heavy chain and light chain variable region were cloned to human IgG1 antibody expression vector show as Fig.9. VH was using enzyme site *BsiWI* and *ApaI,* and VL was using enzyme site *BsPEI* and *NheI.* Vectors were transiently transfected into either 293F or CHO-S cells. Recombinant chimeric Ab was purified and further study for binding assay and complement-dependent tumor cell lysis assay.

### EXAMPLE 12: Binding Analysis of AntibodiesBy Glycan Array

The mAbs 273 and 651, and anti-SSEA-4 (mAbs 45, 46 and 48) were subjected to glycan binding studies as described below. 152 chemically synthesized glycans were synthesized and spotted on the glycan microarray. Glycan array were incubated with antibodies at various dilutions for 1 hour at 37°C. Then the slides were washed three times each with 0.05% Tween 20/PBS buffer (PBST). Next, Cy5-conjugated goat anti-mouse IgM or IgG antibody was added to the slide. Finally, the slides were washed three times with PBST. The microarray slides were dried before being scanned at 635 nm with a microarray fluorescence chip reader (4000B; Genepix). Data were analyzed by the software GenePix Pro-6.0 (Axon Instruments). The results obtained from this study are shown in Figure 2.

The mAb 273 is capable of binding the Globo H hexasaccharide epitope of Fucα1→ 2Galβ1→ 3GalNAcβ1→ 3Galα1→ 4Galβ1→ 4Glcβ1 (#53 on glycan array), and a segment of Galβ1→ 3GalNAcβ1→ 3Galα1→ 4Galβ1→ 4Glcβ1 (#57 on glycan array). The mAb 651 recognizes the glycan epitopes of Fucα1→ 2Galβ1→ 3GalNAcβ1→ 3Galα1→ 4Galβ1→ 4Glcβ1 (#53 on glycan array), Galβ1→ 3GalNAcβ1→ 3Galα1→ 4Galβ1→ 4Glcβ1(#57 on glycan array) andNeu5Acα2→ 3Galβ1→ 3GalNAcβ1→ 3Galα1→ 4Galβ1→ 4Glcβ1 (#12 on glycan array).

The mAb 45 is capable of binding the SSEA-4 hexasaccharide epitope of Neu5Acα2→ 3Galβ1→ 3GalNAcβ1→ 3Galα1→ 4Galβ1→ 4Glcβ1 (#12 on glycan array), and an analogue Neu5Gcα2→ 3Galβ1→ 3GalNAcβ1→ 3Galα1→ 4Galβ1→ 4Glcβ1 (#49 on glycan array). The mAb 46 binds toNeu5Acα2→ 3Galβ1→ 3GalNAcβ1→ 3Galα1→ 4Galβ1→ 4Glcβ1 (#12 on glycan array), Neu5Gcα2→ 3Galβ1→ 3GalNAcβ1→ 3Galα1→ 4Galβ1→ 4Glcβ1 (#49 on glycan array), Neu5Acα2→ 3Galβ1→ 3GalNAcβ1→ 3Galα1 (#11 on glycan array) and Neu5Acα2→ 3Galβ1→ 3GalNAcβ1→ 3Galβ1 (#10 on glycan array). The mAb 48 is capable of binding Neu5Acα2→ 3Galβ1→ 3GalNAcβ1→ 3Galα1→ 4Galβ1→ 4Glcβ1 (#12 on glycan array), and Neu5Gcα2→ 3Galβ1→ 3GalNAcβ1→ 3Galα1→ 4Galβ1→ 4Glcβ1 (#49 on glycan array).

The glycan microarray was also used to investigate the binding specificity of MC813-70. As shown in Fig. 2(H), we found that among the 152 chemically synthesized glycans on the glycan microarray, MC813-70 recognizes Neu5Acα2→ 3Galβ1→ 3GalNAcβ1→ 3Galα1→ 4Galβ1→ 4Glcβ1 (#12 on glycan array) and Neu5Gcα2→ 3Galβ1→ 3GalNAcβ1→ 3Galα1→ 4Galβ1→ 4Glcβ1 (#49 on glycan array). MC813-70 does not bind GMlb (#104 on glycan array) or GDla (#106 on glycan array).

We also used the glycan array to determine the dissociation constants of MC45, MC48 and MC813-70 with SSEA-4 hexasaccharide on surface, and the Kd values for MC45, 48 and 813 are shown below. These results showed that these mAbs are highly specific for SSEA4.

| | **Kd (nM) ± SD(nM)** |
|---|---|
| **MC45** | 0.37 ± 0.08 |
| **MC48** | 0.46 ± 0.1 |
| **MC813-70** | 4.21 ± 0.26 |

### EXAMPLE 13: Binding Analysis of Antibodiesto Cancer CellsBy Flow Cytometry

Binding of mAb 273 and anti-SSEA-4 (mAbs 45, 46 and 48) to cancer cell lines were examined. Cells (1 × 10⁵) were resuspended in 100µL FACS buffer (1% BSA/PBS solution) containing various concentration antibody and incubated on ice for 30 min. After being washed twice with FACS buffer, cells were incubated with 649-labeled goat anti-mouse antibody (1:100; Jackson ImmunoResearch) for 30 min on ice before analysis on a FACSCalibur system (BD Biosciences). The results are shown in Figures 7A-D. Breast cancer cells MCF-7 were stained with mAb 273 (Fig. 7A). Pancreatic cancer cells (HPAC and BxPC3) and breast cancer cells MCF-7were stained with mAb 45 (Fig. 7B). Pancreatic cancer cells (HPAC and BxPC3) and breast cancer cells MCF-7 were stained with mAb 46 (Fig. 7C). Pancreatic cancer cells (HPAC and BxPC3) and breast cancer cells MCF-7were stained with mAb 48 (Fig. 7D).

### EXAMPLE 14-1: Antibodies Mediate Complement-Dependent Cytotoxicity (CDC)

The ability of mAb 273 to mediate CDC of Globo H expressing cells was examined. MCF-7 cells in the presence of human serum as a source of complement. Cell death was assessed by the addition of the viability probe 7-AAD. Based on the results of the 7-AAD measurement, percentage-specific lysis was calculated using a FACScan flow cytometer. The antibodies showed about 30% killing activity at 40 µg/mL. As shown in Figure 5(B), mAb273 can mediate successfully Complement-Dependent Cytotoxicity of Globo H expressing cell.

Example 14-2: The ability of exemplary mAbs 46 and 48 to mediate CDC of SSEA-4 expressing cells was examined. Homo sapiens pancreas adenocarcinoma cell (BxPC3) in the presence of rabbit serum as a source of complement. Cell death was assessed by the addition of the viability probe 7-AAD. Based on the results of the 7-AAD measurement, percentage-specific lysis was calculated using a FACScan flow cytometer. The antibodies showed about 20% killing activity at 40µg/mL. As shown in Figure 5(C), mAbs 46 and 48 successfully mediated CDC of SSEA-4 expressing cells.

### Materials and Methods

Reagents. Anti-Le^{x}, anti-sLe^{x}, and anti-GD2 antibodies were purchased from BD Biosciences (Franklin Lakes, NJ). Anti-GDla, anti-GTlb and Alexa Fluor® 488 anti-A2B5 antibodies were purchased from Millipore (Billerica, MA). Anti-GMl and anti-GM2 antibodies were purchased from Calbiochem (Merck, Darmstadt, Germany). Anti-Le^{y} and anti-sTn antibodies were purchased from Abcam (Cambridge, UK). Anti-TF antibody was purchased from Thermo Scientific (Waltham, MA). Anti-Tn antibody was purchased from DakoCytomation (Glostrup, Denmark). Fluorescence-labelled or purified MC813-70 and MC631 were purchased from Biolegend (San Diego, CA). MC813-70 ascites were purchased from Developmental Studies Hybridoma Bank at the University of Iowa. The usages of these antibodies in individual experiments were descried in the following paragraphs.

Cell Culture. U-251, U-138, LN-18, T98, LN-229, U87, U-373, Hs683, D54MG, GBM 8401, GBM 8901, G5T, G9T, SNB75, A172 and SF126 cells were routinely maintained in high glucose DMEM (Life Technologies, Carlsbad, CA) supplemented with 10% FBS (Biological Industries, Israel). DBTRG cells were maintained in RPMI 1640 (Life Technologies) with 10% FBS.

Flow Cytometry. Cells (1 × 10⁵) were stained with 0.5µg Alexa Flour 488-conjugated anti-SSEA-3 mAb (MC-631), anti-SSEA-4 mAb (MC813-70) or allophycocyanin (APC)-conjugated anti-Globo H mAb (VK9, a gift from Philip O. Livingston, Memorial Sloan-Kettering Cancer Center, New York, NY) in 50 µL FACS buffer (PBS solution with 1% FBS) on ice for 30 min. For lectin staining, cells were incubated in lectin binding buffer [1% BSA, 0.5 × Carbo-Free Blocking buffer (Vector Laboratories, Burlingame, CA), 2 mM MgCl₂, 2 mM CaCl₂] containing biotinylated lectin for 30 min on ice. After being washed twice with lectin binding buffer, cells were incubated with streptavidin-APC (1:500 diluted in FACS buffer; Biolegend) on ice for 30 min. After washing twice with 200 µL FACS buffer, cells were resuspended in 200 µL FACS buffer containing 1 µg/ml propidium iodide (PI) and subjected to analysis. Data acquisition was performed on a FACSCanto (BD Biosciences) with FACSDiva software (BD Biosciences), and data analyses were performed using FlowJo software (TreeStar, Ashland, OR). Live cells (PI-negative) were gated for analysis. For methanol washing, cells were washed and fixed with 4% paraformaldehyde in PBS for 15 min at room temperature, followed by incubation in methanol for 10 min before staining with specific antibodies.

Immunofluorescent Staining. Cells were plated on tissue culture plastic chamber slides (Nunc, Roskilde, Denmark) overnight to allow sufficient attachment, fixed with 4% paraformaldehyde for 15 min at room temperature, washed three times with PBS, and then blocked with 3% BSA in PBS. Cells were then incubated overnight with 10 µg/mL of mAb MC813-70 (Biolegend), washed three times with PBS and incubated for 2 h at room temperature with 5 µg/ml FITC-conjugated anti-mouse IgG (eBioscience, San Diego, CA). Nuclei were counterstained with Hoechst 33342 (2 µg/mL, Life Technologies). All images were acquired by an Olympus IX71 microscope.

Immunohistochemistry. For MC813-70 staining on normal brain and GBM specimens, three different tissue microarray slides (Biomax, Rockville, MD), comprising a total of 19 normal brain sections and 55 GBM sections were tested. The slides were dried at 56°C for 1 h, deparaffinized in xylene and rehydrated in graded alcohols, followed by treating with blocking buffer [2% Blocking Reagent (Roche, Basel, Switzerland) in PBS with 0.1% Triton X-100] for 30 min at room temperature. The slides were then incubated at 4°C for overnight with mAb MC813-70 (10 µg/mL in blocking buffer). After gently washing with PBST, the immunoreactivity on sepecimens was detected with Supersensitive™ Polymer-HRP IHC Detection System (BioGenex, Fremont, CA), and the slides were counterstained with hematoxylin and prepped for mounting.

Glycan Array Fabrication. Microarrays were printed (BioDot; Cartesian Technologies, Irvine, CA) by robotic pin (SMP3; TeleChem International Inc., Sunnyvale, CA) with the deposition of -0.6 nL per spot. Amine-containing glycans in printing buffer (300 mM sodium phosphate, pH 8.5, 0.01% Triton X-100) were spotted onto *N*-Hydroxysuccinimide (NHS)-activated glass slides. Each glycan was printed at 100 µM in a replicate of four or 50 µM in a replicate of six for Kd determination. Printed slides were allowed to incubate in 80% humidity for 30 min, followed by desiccation for overnight. Remaining NHS groups were blocked by immersing the slides for 1 h in SuperBlock (PBS) Blocking Buffer (Pierce, Appleton, WI).

Ab Binding Assay. MAb MC813-70 Alexa Fluor 647 (Biolegend) was prepared in 100 µL of PBS-B-T (pH 7.4, with 3% BSA and 0.05% Tween-20) and applied to cover the grid. After incubation in a moist chamber for 30 minutes, the slides were rinsed with PBST and deionized water and blow-dried. The slides were scanned at 635 nm in genepix 4300A (Molecular device, Sunnyvale, CA). Data were analyzed by GenePix Pro-6.0 (Molecular Devices).

Sialidase Treatment. Cells were washed and resuspended in PBS buffer at 1 × 10⁷ cells/mL. Cells were incubated with or without 500 mU a2,3 sialidase (NEB, Ipswich, MA)/10⁶ cells/100 µL for 1 h at 37°C, and washed twice with FACS buffer followed by surface staining and flow cytometry. The efficiency of sialidase treatment was measured by biotinylated *Maackia amurensis* lectin II (MAL II; Vector Laboratories), which recognizes a2,3-linked sialic acids.

Extraction of Glycosphingolipids. Cells (4 × 10⁷) were harvested, washed with PBS and homogenized in water. Per 3 vol. homogenate was added with 8 vol. methanol and 4 vol. chloroform and the sample was incubated in a bath sonicator for 30 min. After centrifugation at 3000 × g for 15 min, the pellet was repeatedly extracted with 4:8:3 chloroform/methanol/water, and the combined supernatant was dried under a stream of nitrogen. The total lipid extract was then dissolved in chloroform/methanol/water (30/60/8, v/v/v), and gangliosides were purified by DEAE-Sephadex A-25 (GE Healthcare, Buckinghamshire, UK) based anion-exchange chromatogrpahy. Unbound flow-through containing neutral glycolipids was collected and dried. After washing with chloroform/methanol/water (30/60/8, v/v/v), gangliosides were eluted with chloroform/methanol/aqueous NaCl (0.02, 0.2 and 0.8 M stepwisely) (30/60/8, v/v/v), followed by desalting with Sep-Pak C18 Cartridges (Waters, Milford, MA). The extracts were dried under nitrogen and the ganglioside residues as well as neutral glycolipid residues were redissolved in 100 µL chloroform/methanol (2/1, v/v).

High-Performance Thin-Layer Chromatograhy. GSLs were separated on glass-packed silica gel 60 precoated high-performance thin-layer chromatography (HPTLC) plates (Merck). Gangliosides were chromatographed in chloroform/methanol/water(120/85/20, v/v/v) and neutral GSLs in chloroform/methanol/water (120/70/17, v/v/v), respectively, each supplemented with 2 mM CaCl₂. For analytic purposes, GSLs were stained with 0.3% orcinol in 3 M H₂SO₄ and then transferred to a preheated heating plate (110 °C) until bluepurple spots appeared. For preparative purposes, gangliosides were stained with 0.02% primulin (Sigma, St. Louis, MO) in acetone/water (4/1, v/v). Spots of gangliosides were marked with a pencil under UV light and scraped from the plate with an adsorbent scraper (Sigma) and the gangliosides were extracted with chloroform/methanol/water (30/60/8, v/v/v) under sonication for 10 min. The silica was removed by centrifugation, re-extracted again, and the combined supernatant were dried and redissolved in methanol.

TLC Immunostaining. GSLs were separated on HPTLC plates as described above. After chromatography, the TLC plate was air-dried, immersed in 2.1% poly(isobutyl-methacrylate) (Sigma) in hexane/chloroform (42:8, v/v) three times and soaked in PBS at 37°C for overnight. The plate was dried, blocked with in PBS for 30 min at room temperature and reacted with MC813-70 or MC-631 (5 µg/mL) for 2 h at room temperature. Gently washed with PBST (0.05% Tween-20) for three times, the plate was incubated with biotinylated secondary antibody (1 µg/mL) for 1 h, followed by incubation with streptavidin-alkaline phosphatase (1:1000; Millipore). After washing with PBST, the TLC plate was developed with NBT/BCIP (Thermo Scientific). The reaction was stopped by washing with distilled water and the plates were air-dried.

MALDI-MS Profiling and MS/MS Analysis. MALDI-MS analysis of permethylated glycans were conducted in an ABI 4700 Proteomics Analyzer (Applied Biosystems, Foster City, CA) using 2,5-dihydroxybenzoic acid (DHB) as the matrix (10 mg/mL). MALDI-MS/MS sequencing with low- and high-energy collision-induced dissociation was operated in a Q/TOF Ultima MALDI (Waters Micromass) and a 4700 Proteomics Analyzer using the DHB matrix as described above.

Complement-Dependent Cytotoxicity (CDC) Assay. The CDC activity of exemplary anti-SSEA4 mAb, such as (MC813-70) mAb, was measured by lactate dehydrogenase (LDH)-release assay using the CytoTox 96® Non-Radioactive Cytotoxicity Assay kit (Promega, Fitchburg, WI). Cells (1 × 10⁴) were plated in each well of 96-well plates and washed with PBS twice after growth for overnight. The cells were then incubated with 1 µg of MC813-70 or mouse IgG3 isotype control in 50 µL phenol red-free DMEM or RPMI with rabbit complement (dilution of 1:5; Life Technologies). After incubation in a 5% CO2 incubator at 37°C for 1 h, the degree of cell lysis was determined by measuring the amount of LDH released into the culture supernatant. Maximum LDH release was determined by lysing the cells with Lysis Solution provided by the kit. Percentage of specific lysis was calculated according to the equation: % lysis = [experimental release - spontaneous release] / [maximum release - spontaneous release] × 100.

In vivo Tumor Growth. BALB/cAnN.Cg-Foxn1nu/CrlNarl mice were purchased from National Laboratory Animal Center (Taiwan) and maintained under specific pathogen-free conditions. The health status of animal was monitored daily. Procedures involving animals and their care were conducted according to Academia Sinica Institutional Animal Care and Utilization Committee in compliance with national and international laws and policies. DBTRG cells (1 × 10⁷/250 µL PBS) were subcutaneously injected to the flank regions of mice (8- to 10-weeks old) to generate the xenograft model. On day 11, 15 and 19, each mouse was peritoneally injected with 200 µg of MC813-70 (purified from the ascites) or mouse IgG3 isotype control Ab. The tumor size was determined by vernier caliper by measuring the length (L) and width (W), and the tumor volume was calculated (in mm³) as 1/2 × LW².

### EXAMPLE 15 Exemplary Phage display biopanning procedures

The phage-displayed human naive scFv library contained 2.5×10¹⁰ clones (Lu et al., 2011) was subtracted with non-specific binding in PEG-conjugated carboxyl Dynabeads (Invitrogen) at room temperature (RT) for 1 hour, and subsequently incubated with SSEA-4-PEG immobilized Dynabeads at 4°C for 1 hour. After washing with PBS or PBS containing 0.01% Tween 20 (PBST0.01), the phages that bound to SSEA-4-PEG-Dynabeads were recovered by infection with E-coli TG1 cells at 37°C for 0.5 hour. Some of the infected cells were serially diluted to determine titer, and the others were rescued by M13KO7 phage and amplified. After determination of rescued phages titer, the next round of biopanning was performed. In the fourth and fifth round of biopanning, the phage clones were randomly selected to culture for ELISA screening.

### ELISA screening of selected phage clones

For detection of antigen recognition, microwell plates (Nunc) were coated with 0.2 µ g/ml of SSEA-4-BSA, Globo H-BSA, SSEA-3-BSA and BSA, respectively. The selected phage clones were diluted 1:2 in PBS containing 3% BSA and added to each well. The plates were incubated at RT for 1 hour, washed with PBST0.1, and incubated with horseradish peroxidase (HRP)-conjugated mouse anti-M13 phage antibody (GE Healthcare). The plates were washed again, and OPD and H2O2 were added. After termination of reaction by 3 N HCl, the absorbance was measured using a 490 nm using microplate reader (Model 680, BioRad). We extracted phagemids from ELISA-positive phage clones to identity scFv coding regions by auto-sequencing.

### Construction and expression of anti-SSEA-4 human IgG

The VH region of selected scFv was cloned with Agel and Nhel site into modified expression vector pcDNA5-FRT-Gamma1 containing a signal peptide and the constant region of human immunoglobulin gamma 1 heavy chain. The VL region of selected scFv was cloned with Agel and EcoRV site into modified expression vector p-Kappa-HuGs containing a signal peptide and constant region of human immunoglobulin kappa light chain. Both plasmids were transfected into FreeStyle293 cells (Invitrogen) and continuously incubated in serum-free medium at 37°C for 1 week to produce human antibody.

### Purification of anti-SSEA-4 human IgG

The culture medium was collected, centrifuged and filtrated with 0.45 µm pore-size membrane. The supernatant then was subjected to protein G column chromatography (GE healthcare) for purification of anti-SSEA-4 human IgG. After dialysis of eluents with PBS, the antibody was examined by SDS-PAGE analysis with coomassie blue staining as usual. The concentration of antibody was assessed by Bradford reagent (Thermo Scientific) and spectrophotometer.

### Humanization of MC48

Two human genes, GenBank accession Q9UL73 and AY577298, were the most similar to MC48 VH and VL, respectively. We humanized three sequences of MC48, including the 1st humanized MC48 (hMC48) VH consisted of modified framework (FR) 1 to FR4 of Q9UL73 gene and the 1st hMC48 VL consisted of four FRs from the accession AY577298, the 2nd hMC48 FRs of VH followed 1YY8 from PDB, while the 2nd hMC48 VL same as 1st sequence, and the 3rd hMC48 VH sequence modified FR1, 2 and 4 of Q9UL73 gene and the 3rd hMC48 VL changed FR2 and FR4 to human AY577298 gene. All of these humanized sequences were conserved CDR1 to CDR3 of VH and VL of MC48.

### Construction of single chain fragments variable (scFv) of humanized MC48 variants

The scFv form of humanized MC48 sequences (VH-GGGGSGGGGSGGGGS-VL (SEQ ID NO: 115)) were gene synthesized (Genomics) and cut by Sfi I and Not I (Fermentas). After gel extraction, the digested products were cloned to pCANTAB-5E phagemid (GE Healthcare).

### Generation of humanized MC48 (hMC48) scFv phage clones.

hMC48 variant phagemids were transformed to TG1 E-coli and recovered in 2 × YT medium (BD Pharmingen) containing 100 µg/ml ampicillin and 2% glucose and rescued by M13KO7 helper phage (NEB) for 1 hour at 37°C. After centrifugation by 1,500 × g for 10 min, these pellets were resuspended in 2 × YT medium containing 100 µg/ml ampicillin and 50 µg/ml kanamycin overnight to generate scFv-phages.

### Binding assay of hMC48 scFv phage clones by ELISA

SSEA-4-BSA was coated on an ELISA plate at the concentration of 0.2 µg/ml. After washing and blocking, the serial diluted phages were incubated at RT for 1.5 hour. After washing, 1:1000 diluted HRP-conjugated anti-M13 antibody (GE Healthcare) was added at RT for 1 hour. Then, liquid substrate 3,3',5,5'-tetramethylbenzidine (TMB) developed and was terminated with 3N HCl. Optical density was measured at 450 nm.

### Results

### Identification of phage-displayed scFv that binds to SSEA-4

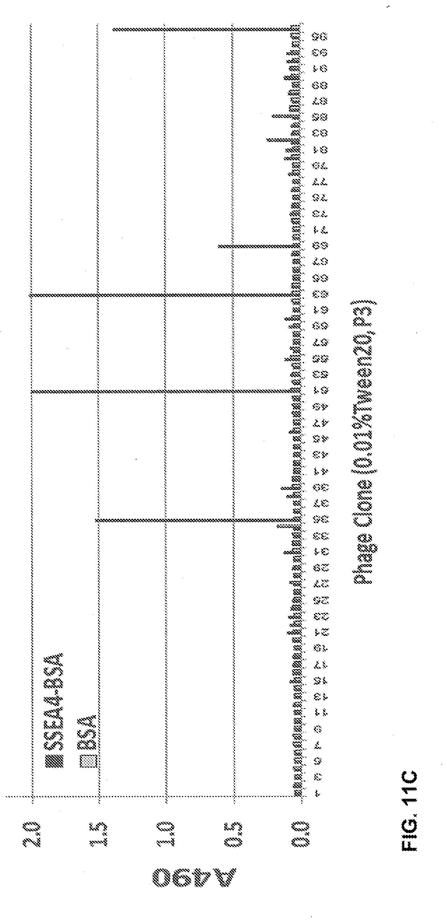

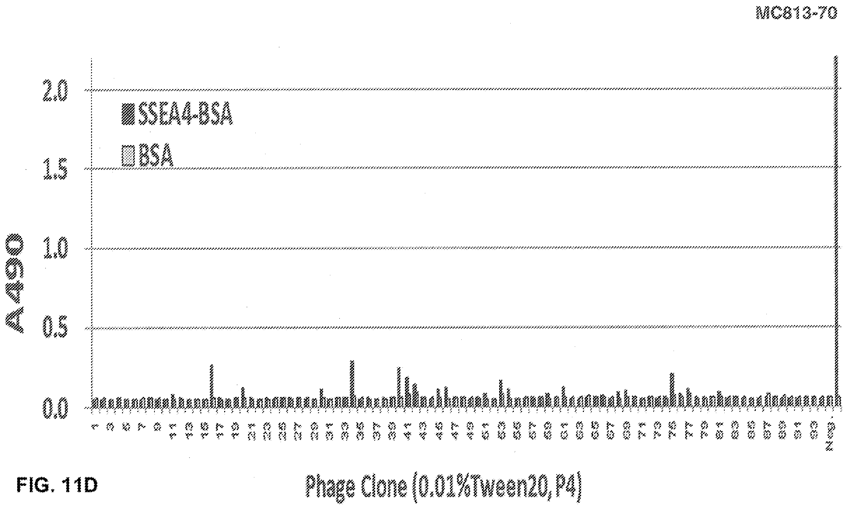

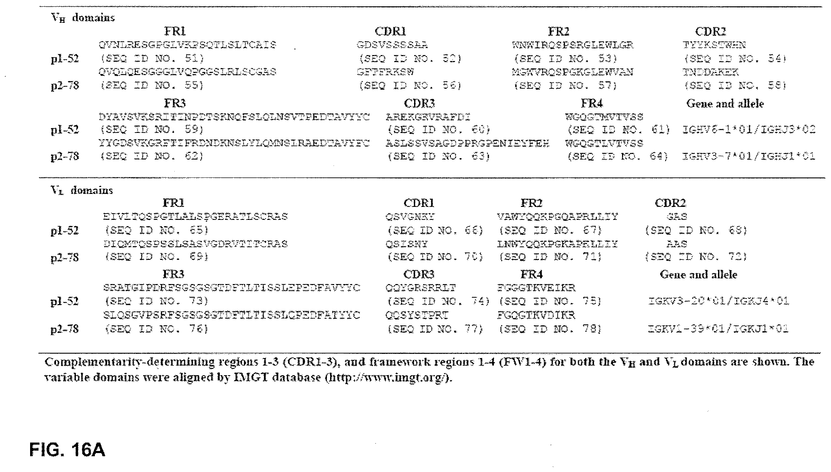

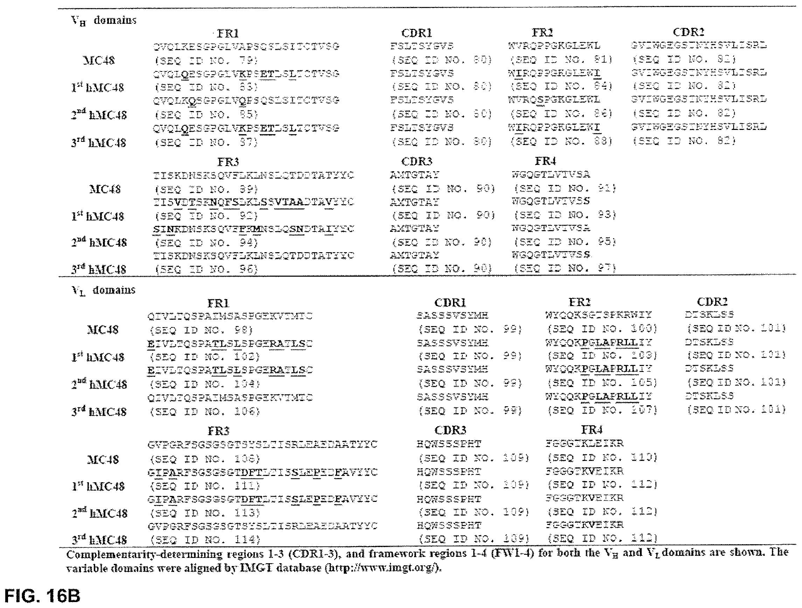

To identify the antibodies that bind to SSEA-4, we used phage-displayed human naive scFv library containing 2.5×10¹⁰ members which was established as our previous report described (Lu et al., 2011). This library was first removed Dynabeads-binding phages and then selected for SSEA-4-binding phages by SSEA-4-PEG-conjugated Dynabeads. We used two buffer systems, PBS and PBS containing 0.01% Tween20 (PBST0.01), during biopanning. After five rounds of affinity selection, the phage recovery of the fifth round had increased about 55-fold and 80-fold than that of the first round in PBS and PBST0.01 system, respectively (Fig. 10). The phage clones were randomly selected and tested for SSEA-4 binding by ELISA (Fig. 11). We found seven clones that specifically bound to SSEA-4-BSA, but not to BSA control protein. By sequencing all 8 individual clones, we identified two unique anti-SSEA-4 phage clones (p1-52 and p2-78) which contain distinct human VH and VL coding regions (Fig 16A).

To examine the specificity and binding affinity of the two phage clones, we performed a comparative ELISA using the same phage titer to Globo-series glycans including SSEA-4-BSA, Globo H-BSA and SSEA-3-BSA (Fig. 12). The p2-78 phage clone showed the strong binding to SSEA-4-BSA and SSEA-3-BSA, and slightly weaker binding to Globo H-BSA. However, we found that the binding activity of p1-52 phage clone to SSEA-4-BSA is very weak. Thus we focused on p2-78 clone for further study.

To establish the fully human antibody (hAb) against SSEA-4, we molecularly engineered the VH and VL coding sequences of p2-78 scFv into human IgG1 backbone, respectively. The anti-SSEA-4 p2-78 hAb was produced using FreeStyle 293 expression system and then purified through the protein G sepharose column. We examined the purity of antibody by SDS-PAGE analysis with coomassie blue staining (Fig. 13A). The result shows the purity level of antibody exceed 95%. Subsequently, we performed ELISA to investigate the binding activity of p2-78 hAb for Globo-series glycans (Fig. 13B). We found that p2-78 hAb bound to SSEA-4 and SSEA-3, but not to Globo H, which demonstrates the human IgG version of p2-78 retains the activity of its parental scFv version to recognize the binding epitope of SSEA-4.

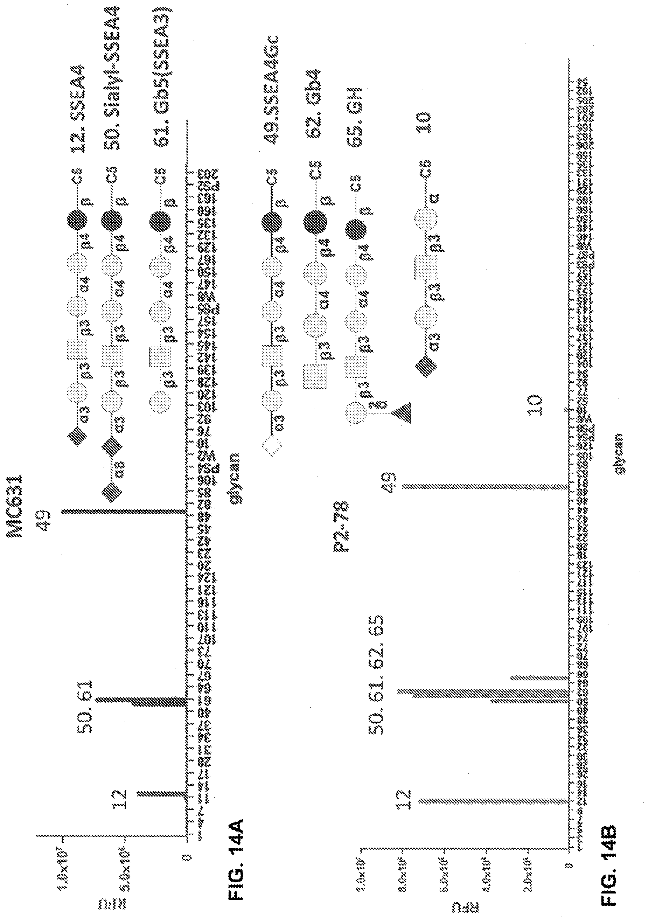

We used glycan array containing 203 different glycans to further confirm the specificity of p2-78 hAb. The results showed that p2-78 hAb recognized SSEA4, Sialyl-SSEA4, SSEA4Gc, and Gb5 (SSEA3) (Fig. 14B). Interestingly, p2-78 hAb also recognized GloboH, similar to the results from ELISA assay (Fig. 12). The commercially available IgM antibody, MC631, was used as a positive control (Fig. 14A).

### Development of humanized MC48 mAbs

Non-humanized Murine mAbs may have certain limitations in clinical settings, including their short serum half-life, inability to trigger human effector functions and the production of human anti-murine antibodies (HAMA) response (LoBuglio et al., 1989). Therefore, mAbs can be humanized by grafting their CDRs onto the VH and VL FRs of human Ig molecules (Roguska et al., 1994).

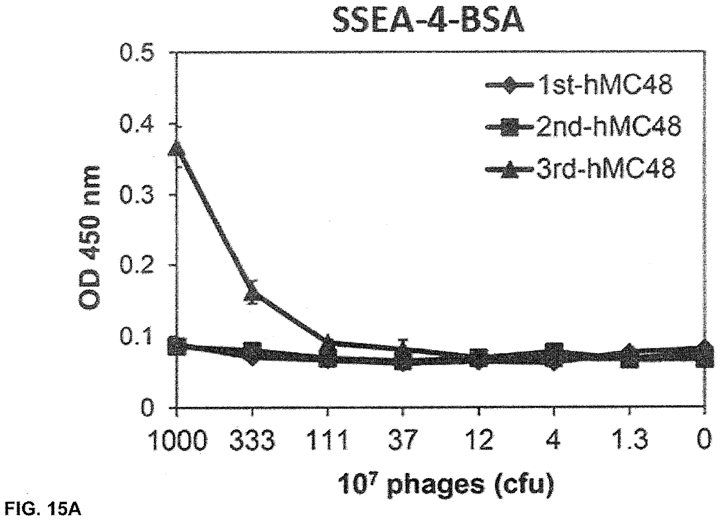

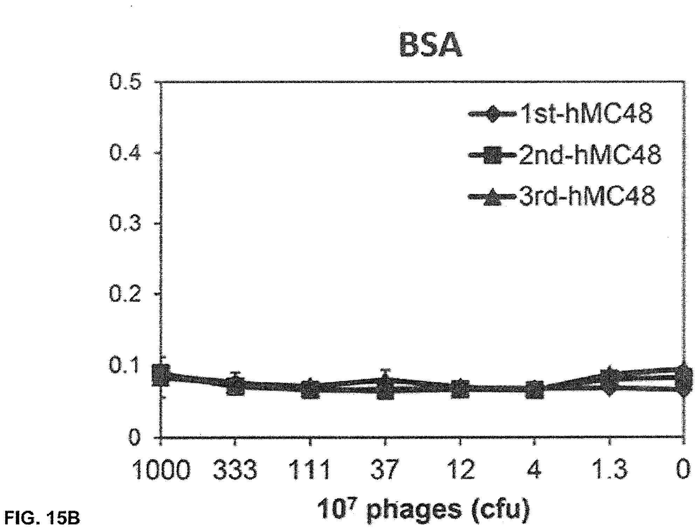

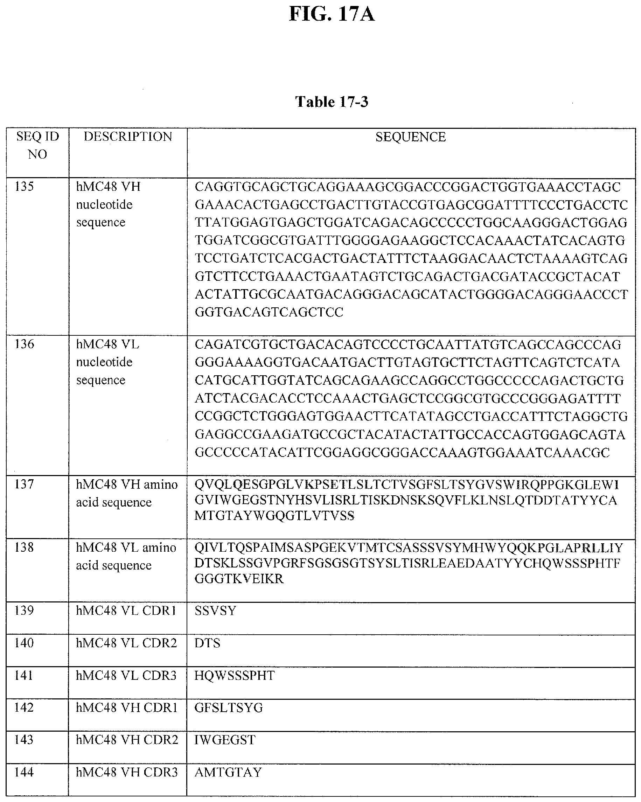

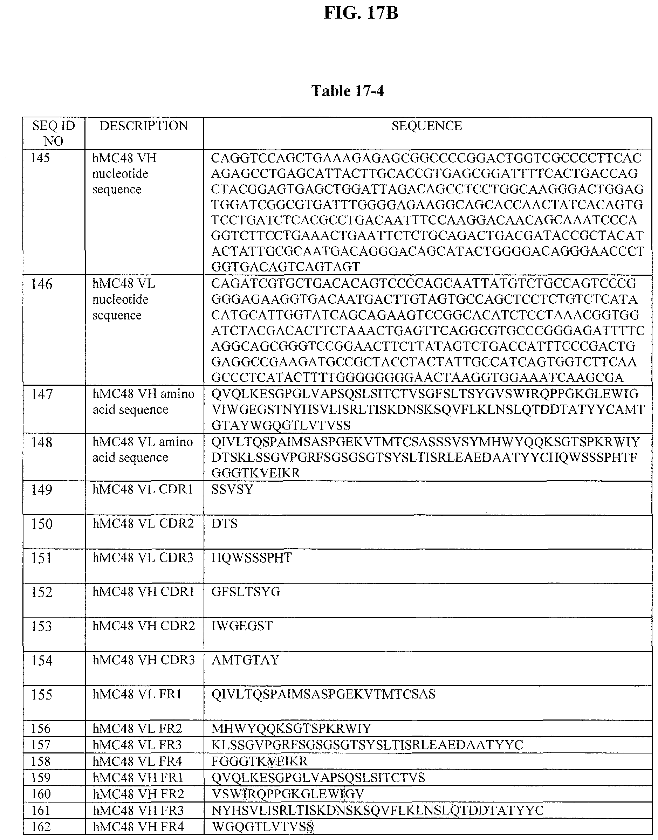

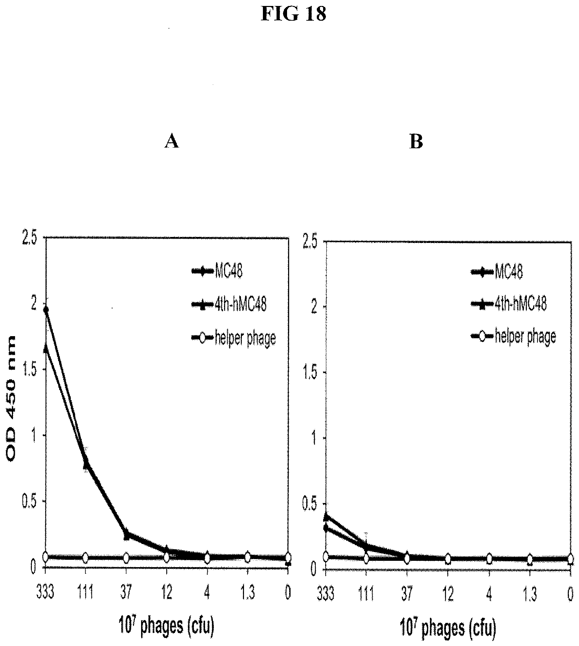

To develop humanized MC48, we sequenced V_{H} and V_{L} variable region of MC48 from a hybridoma cell (Table 4). After alignment of V_{H} and V_{L} variable region of MC48 with the NCBI IgBLAST database, we modified FRs of MC48 and generated 1^{st}, 2^{nd}, 3^{rd} and 4^{th} humanized MC48 sequences (Table 17, Figure 17). We next constructed and generated the phage-displayed scFv formats according to these humanized MC48 sequences. To determine the binding activity of the humanized MC48 phage clones, we carried out solid-based ELISA coating SSEA-4-BSA (Figs. 15 and 18). We found that the humanized MC48 scFv phage could recognize SSEA-4 in a dose-dependent manner. The data indicated that the 4^{th} humanized MC48 scFv phage maintained its binding affinity compared with the murine mAb MC48.

### Example 16

### Complement-Dependent Cytotoxicity (CDC) Assay.

The ability of exemplary humanized MC 48 to mediate CDC of SSEA-4 expressing cells is examined. Homo sapiens breast or pancreatic carcinoma cells were plated in each well of 96-well plates for growth of overnight prior to the assay. The cells were then incubated with serially diluted concentrations of humanized MC 48 or human IgG1 isotype control in RPMI in the presence of rabbit serum as a source of complement (dilution of 1:5; Life Technologies).Cell death is assessed by the addition of the viability probe 7-AAD. Based on the results of the 7-AAD measurement, percentage-specific lysis is calculated using a FACScan flow cytometer.The antibodies show significant killing activity at 10µg/mL compared to isotype control. As shown, humanized MC48-4 successfully mediates CDC of SSEA-4 expressing cells.

## Claims

1. An isolated humanized monoclonal antibody that specifically binds to Neu5Acα2→ 3Galβ1→ 3GalNAcβ1→ 3Galα1→ 4Galβ1→ 4Glcβ1, wherein the antibody comprises a VH having SEQ ID NO: 147 and a VL having SEQ ID NO: 148.

2. The isolated humanized monoclonal antibody of claim 1, wherein the antibody is an IgG.

3. The isolated humanized monoclonal antibody of claim 2, wherein the antibody is an IgG1.

4. Isolated humanized monoclonal antibody of any one of claims 1-3 for use for treating cancer in a subject in need thereof.

5. Isolated humanized monoclonal antibody for its use according to claim 4, wherein the cancer is selected from the group consisting of brain cancer, lung cancer, breast cancer, oral cancer, esophageal cancer, stomach cancer, liver cancer, bile duct cancer, pancreatic cancer, colon cancer, kidney cancer, cervical cancer, ovarian cancer and prostate cancer.

6. A pharmaceutical composition comprising the isolated humanized monoclonal antibody according to claims 1-3.

7. Pharmaceutical composition of claim 6 for use for treating cancer in a subject in need thereof.

8. Pharmaceutical composition for its use according to claim 7, wherein a cell of the cancer expresses SSEA4 antigen on the surface of the cell.

9. Pharmaceutical composition for its use according to claim 7, wherein a cell of the cancer further expresses at least one of GloboH and/or SSEA3 on the surface of the cell.

10. Pharmaceutical composition for its use according to claim 7, wherein a cell of the cancer expresses Globo H, SSEA3 and SSEA4 antigen simultaneously on the surface of the cell.

11. Pharmaceutical composition for its use according to any one of claims 8-10, wherein the cancer is selected from the group consisting of brain cancer, lung cancer, breast cancer, oral cancer, esophageal cancer, stomach cancer, liver cancer, bile duct cancer, pancreatic cancer, colon cancer, kidney cancer, bone cancer (osteosarcoma), skin cancer, cervical cancer, ovarian cancer, and prostate cancer.

12. An isolated humanized monoclonal antibody of claim 1, wherein the antibody is a glycoantibody and wherein the glycoantibody comprises an N-glycan attached to the Asn-297 of the Fc region of said antibody wherein the N-glycan consists of the structure of Sia₂(α2-6)Gal₂GlcNAc₂Man₃GlcNAc₂ and wherein the glycoantibody specifically binds to an SSEA-4 antigen.

13. An antigen binding fragment of the antibody according to claim 1, which fragment specifically binds to Neu5Acα2→ 3Galβ1→ 3GalNAcβ1→ 3Galα1→ 4Galβ1→ 4Glcβ1 and comprises a VH having SEQ ID NO: 147 and a VL having SEQ ID NO: 148.

14. The antigen binding fragment of claim 13, wherein the antigen-binding fragment is an Fab fragment, an F(ab')2 fragment, or a single-chain Fv fragment.

## Patentansprüche

1. Isolierter humanisierter monoklonaler Antikörper, der spezifisch an Neu5Acα2→ 3Galβ1→ 3GaINAcβ1→ 3Galα1→ 4Galβ1→ 4Glcβ1 bindet, wobei der Antikörper eine VH umfasst, die SEQ ID NO: 147 aufweist, und eine VL, die SEQ ID NO: 148 aufweist.

2. Isolierter humanisierter monoklonaler Antikörper, nach Anspruch 1, wobei der Antikörper ein IgG ist.

3. Isolierter humanisierter monoklonaler Antikörper, nach Anspruch 2, wobei der Antikörper ein IgG1 ist.

4. Isolierter humanisierter monoklonaler Antikörper nach einem der Ansprüche 1 bis 3 zur Verwendung zum Behandeln von Krebs bei einem Patienten, der dessen bedarf.

5. Isolierter humanisierter monoklonaler Antikörper zu seiner Verwendung nach Anspruch 4, wobei der Krebs ausgewählt ist aus der Gruppe bestehend aus Hirnkrebs, Lungenkrebs, Brustkrebs, Mundkrebs, Speiseröhrenkrebs, Magenkrebs, Leberkrebs, Gallengangkrebs, Bauchspeicheldrüsenkrebs, Dickdarmkrebs, Nierenkrebs, Gebärmutterhalskrebs, Eierstockkrebs und Prostatakrebs.

6. Pharmazeutische Zusammensetzung, umfassend den isolierten humanisierten monoklonalen Antikörper nach den Ansprüchen 1 bis 3.

7. Pharmazeutische Zusammensetzung nach Anspruch 6 zur Verwendung zum Behandeln von Krebs bei einem Patienten, der dessen bedarf.

8. Pharmazeutische Zusammensetzung zur Verwendung nach Anspruch 7, wobei eine Zelle des Krebses SSEA4-Antigen auf der Oberfläche der Zelle exprimiert.

9. Pharmazeutische Zusammensetzung zur Verwendung nach Anspruch 7, wobei eine Zelle des Krebses ferner mindestens eines von GloboH und/oder SSEA3 auf der Oberfläche der Zelle exprimiert.

10. Pharmazeutische Zusammensetzung zur Verwendung nach Anspruch 7, wobei eine Zelle des Krebses Globo H, SSEA3 und SSEA4-Antigen gleichzeitig auf der Oberfläche der Zelle exprimiert.

11. Pharmazeutische Zusammensetzung zur Verwendung nach einem der Ansprüche 8-10, wobei der Krebs ausgewählt ist aus der Gruppe bestehend aus Hirnkrebs, Lungenkrebs, Brustkrebs, Mundkrebs, Speiseröhrenkrebs, Magenkrebs, Leberkrebs, Gallengangkrebs, Bauchspeicheldrüsenkrebs, Dickdarmkrebs, Nierenkrebs, Knochenkrebs (Osteosarkom), Hautkrebs, Gebärmutterhalskrebs, Eierstockkrebs und Prostatakrebs.

12. Isolierter humanisierter monoklonaler Antikörper nach Anspruch 1, wobei der Antikörper ein Glykoantikörper ist und wobei der Glykoantikörper ein N-Glykan umfasst, das an das Asn-297 der Fc-Region des Antikörpers angelagert ist, wobei das N-Glykan aus der Struktur von Sia₂(α2-6)Gal₂GlcNAc₂Man₃GlcNAc₂ besteht und wobei der Glykoantikörper spezifisch an ein SSEA-4-Antigen bindet.

13. Antigenbindendes Fragment des Antikörpers nach Anspruch 1, wobei das Fragment spezifisch an Neu5Acα2→ 3Galβ1 → 3GalNAcβ1→ 3Galα1→ 4Galβ1→ 4Glcβ1 bindet und eine VH umfasst, die SEQ ID NO: 147 aufweist, und eine VL, die SEQ ID NO: 148 aufweist.

14. Antigenbindendes Fragment nach Anspruch 13, wobei das antigenbindende Fragment ein Fab-Fragment, ein F(ab')2-Fragment oder ein einkettiges Fv-Fragment ist.

## Revendications

1. Anticorps monoclonal humanisé isolé qui se lie spécifiquement à Neu5Acα2→ 3Galβ1→ 3GalNAcβ1 → 3Galα1 → 4Galβ1 → 4Glcβ1, dans lequel l'anticorps comprend une VH ayant SEQ ID N° : 147 et une VL ayant SEQ ID N° : 148.

2. Anticorps monoclonal humanisé isolé selon la revendication 1, dans lequel l'anticorps est une IgG.

3. Anticorps monoclonal humanisé isolé selon la revendication 2, dans lequel l'anticorps est une IgG1.

4. Anticorps monoclonal humanisé isolé selon l'une quelconque des revendications 1 à 3 pour une utilisation en vue de traiter le cancer chez un sujet qui en éprouve le besoin.

5. Anticorps monoclonal humanisé isolé pour son utilisation selon la revendication 4, dans lequel le cancer est choisi dans le groupe constitué par le cancer du cerveau, le cancer du poumon, le cancer du sein, le cancer buccal, le cancer de l'œsophage, le cancer de l'estomac, le cancer du foie, le cancer des voies biliaires, le cancer du pancréas, le cancer du côlon, le cancer du rein, le cancer du col de l'utérus, le cancer de l'ovaire et le cancer de la prostate.

6. Composition pharmaceutique comprenant l'anticorps monoclonal humanisé isolé selon les revendications 1 à 3.

7. Composition pharmaceutique selon la revendication 6 pour une utilisation en vue de traiter le cancer chez un sujet qui en éprouve le besoin.

8. Composition pharmaceutique pour son utilisation selon la revendication 7, dans laquelle une cellule du cancer exprime l'antigène SSEA4 sur la surface de la cellule.

9. Composition pharmaceutique pour son utilisation selon la revendication 7, dans laquelle une cellule du cancer exprime en outre au moins l'un parmi GloboH et/ou SSEA3 sur la surface de la cellule.

10. Composition pharmaceutique pour son utilisation selon la revendication 7, dans laquelle une cellule du cancer exprime les antigènes Globo H, SSEA3 et SSEA4 simultanément sur la surface de la cellule.

11. Composition pharmaceutique pour son utilisation selon l'une quelconque des revendications 8 à 10, dans laquelle le cancer est choisi dans le groupe constitué par le cancer du cerveau, le cancer du poumon, le cancer du sein, le cancer buccal, le cancer de l'œsophage, le cancer de l'estomac, le cancer du foie, le cancer des voies biliaires, le cancer du pancréas, le cancer du côlon, le cancer du rein, le cancer des os (ostéosarcome), le cancer de la peau, le cancer du col de l'utérus, le cancer de l'ovaire, et le cancer de la prostate.

12. Anticorps monoclonal humanisé isolé selon la revendication 1, dans lequel l'anticorps est un glycoanticorps et dans lequel le glycoanticorps comprend un N-glycane attaché à l'Asn-297 de la région Fc dudit anticorps dans lequel le N-glycane est constitué de la structure de Sia₂(α2-6)Gal₂GlcNAc₂Man₃GlcNAc₂ et dans lequel le glycoanticorps se lie spécifiquement à un antigène SSEA-4.

13. Fragment de liaison à un antigène de l'anticorps selon la revendication 1, le fragment se liant spécifiquement à Neu5Acα2→ 3Galβ1→ 3GalNAcβ1→ 3Galα1→ 4Galβ1→ 4Glcβ1 et comprenant une VH ayant SEQ ID N° : 147 et une VL ayant SEQ ID N° : 148.

14. Fragment de liaison à un antigène selon la revendication 13, dans lequel le fragment de liaison à un antigène est un fragment Fab, un fragment F(ab')2, ou un fragment Fv monochaîne.
